# EUROPEAN PATENT APPLICATION

(11) **EP 2 366 696 A1**
(43) Date of publication of application: **21.09.2011**
(21) Application number: 10181554.6
(22) Date of filing: 15.12.2005
(51) Int. Cl.: C07D 265/24, C07C 255/50, C07C 271/58, C07D 239/91, C07D 239/94

(54) **Process for producing 4-aminoquinazolines**

(30) Priority: 17.12.2004 US 637278 P
(62) Divisional of application: 05854269.7
(71) Applicant: Vertex Pharmaceuticals Incorporated, Cambridge, MA 02130 (US)
(72) Inventor: Silva, Richard, A., Needham, MA MA 02492 (US); Jones, Andrew, Needham, MA MA 02492 (US); Blythe, Todd, Georgetown, MA MA 01833 (US)
(74) Representative: Cohausz & Florack

(57) **Abstract**

The present invention relates to methods for preparing compounds of formula (I): or suitable salts thereof useful as inhibitors of voltage-gated sodium channels and calcium channels. The invention also relates to methods for preparing intermediates related thereto.

## Description

### CROSS REFERENCE TO RELATED APPLICATIONS

The present application claims the benefit, under 35 U.S.C. § 119, of United States Provisional Application number: 60/637,278 filed December 17, 2004, entitled "Processes for Producing 4-Aminoquinazolines" and the entire contents of this application are hereby incorporated by reference.

### TECHNICAL FIELD OF THE INVENTION

The present invention relates to methods of preparing compounds useful as inhibitors of ion channels, and intermediates thereto.

### BACKGROUND OF THE INVENTION

The present invention provides processes for producing 4-amino-quinazolines and analogs thereof. These compounds are useful as inhibitors of voltage-gated sodium channels and calcium channels.

### SUMMARY OF THE INVENTION

As described herein, the present invention provides methods for preparing compounds useful as inhibitors of voltage-gated sodium channels and calcium channels. Such compounds include compounds of formula I: 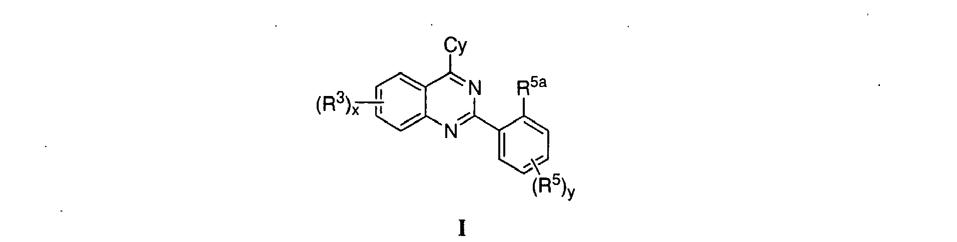 or suitable salts thereof;
wherein Cy, R³, x, R5a, R⁵ and y are as defined in any of the embodiments herein.

The present invention also provides compounds useful as intermediates in the processes of the present invention.

### DETAILED DESCRIPTION OF THE INVENTION

The compounds of the present invention include compounds of formula I: 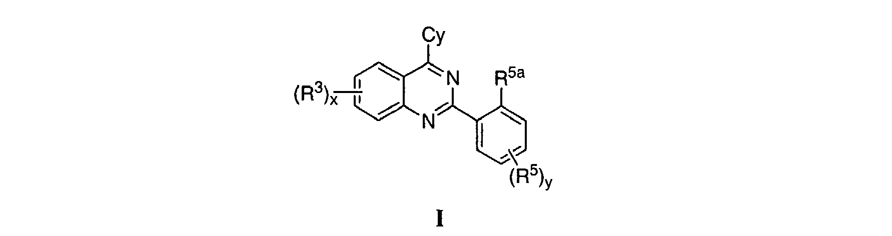 or suitable salts thereof;
wherein:
Cy is a ring selected from: 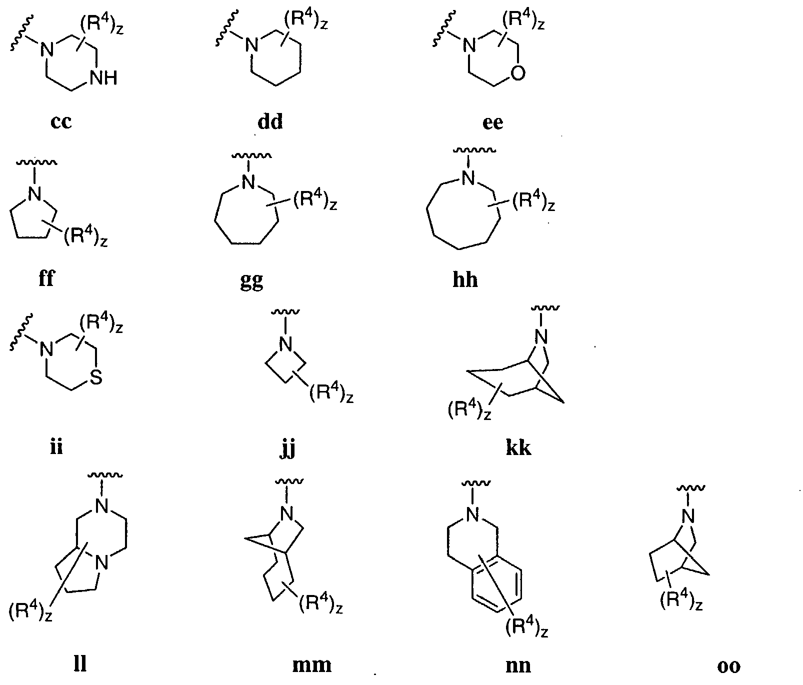 wherein Cy is optionally substituted at one or more substitutable carbon, nitrogen, or sulfur atoms with z independent occurrences of -R⁴;
each z is independently 0-5;
each R⁴ is independently halogen, CN, NO_{z}, -N(R')_{2,} -CH₂N(R')2_{,} -OR', -CH_{z}OR', -SR', -CH₂SR', -COOR', -NRCOR', -CON(R')₂, -OCON(R')₂, COR', -NHCOOR', -SO2R', -S0₂N(R')_{z}, or an optionally substituted group selected from Cₜ.C₆aliphatic, aryl, heteroaryl, cycloaliphatic, heterocycloaliphatic, aryICₜ-C₆alkyl, heteroarylC_{I}-C₆alkyl, cycloaliphaticCl-C6alkyl, or heterocycloaliphaticC¡-C₆alkyl;
x is 0-4;
each R³ is independently halogen, CN, NO₂, -N(R')₂, -CH₂N(R')2_{,} -OR', -CH₂0R', -SR', -CH₂SR', -COOR', -NRCOR', -CON(R')₂, -OCON(R')₂, COR', -NHCOOR', -S0₂R', -S0₂N(R')2_{,} or an optionally substituted group selected from Cₗ-C₆aliphatic, aryl, heteroaryl, cycloaliphatic, heterocycloaliphatic, arylC₁-C₆alkyl, heteroarylC_{I}-C₆alkyl, cycloaliphaticCₗ-C₆alkyl, or heterocycloaliphaticCₗ-C₆alkyl.
y is 0-5;
each R⁵ is independently halogen, CN, N0₂, -N(R')2_{,} -CH₂N(R')2_{,} -OR', -CH₂0R', -SR', -CH₂SR', -NRCOR', -CON(R')_{2,} -S(O)₂N(R')₂, -OCOR', -COR', -C0₂R', -OCON(R')2_{,} -NR'SO2R', -OP(O)(OR')2_{,} -P(O)(OR')2_{,} -OP(O)₂0R' , -P(O)₂OR', -PO(R')2_{,} -OPO(R')2_{,} or an optionally substituted group selected from C₁-C₆aliphatic, aryl, heteroaryl, cycloaliphatic, heterocycloaliphatic, aryIC¡-C₆alkyl, heteroarylCₗ-C₆alkyl, cycloaliphaticCₗ-C₆alkyl, or heterocycloaliphaticC¡-C₆alkyl;
_{R}^{5a} is _{Cl}, _{Br}, _{F}, _{CF3}, _{Me}, _{E}t, C_{N}, -COOH, -NH₂, -N(CH₃)₂, -N(Et)₂, -N(iPr)₂, -O(CH₂)₂0CH₃, -CONH₂, -COOCH₃, -OH, -OCH₃, -OCH₂CH₃, -CH₂0H, -NHCOCH₃, -S0₂NH₂, -so₂NHC(CH₃)₂, -OCOC(CH₃)₃, -OCOCH₂C(CH₃)₃, -O(CH₂)₂N(CH₃)₂,4-CH₃-piperazin-1-yl, OCOCH(CH₃)₂, OCO(cyclopentyl), -COCH₃, optionally substituted phenoxy, or optionally substituted benzyloxy; and
each occurrence of R' is independently hydrogen or an optionally substituted C¡-₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

Compounds of this invention include those described generally above, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75^{th} Ed. Additionally, general principles of organic chemistry are described in "Organic Chemistry", Thomas Sorrell, University Science Books, Sausalito: 1999, and "March's Advanced Organic Chemistry", 5 th Ed., Ed.: Smith, M.B. and March, J., John Wiley & Sons, New York: 2001, the entire contents of which are hereby incorporated by reference.

As described herein, compounds of the invention may optionally be substituted with one or more substituents, such as are illustrated generally above, or as exemplified by particular classes, subclasses, and species of the invention. It will be appreciated that the phrase "optionally substituted" is used interchangeably with the phrase "substituted or unsubstituted." In general, the term "substituted", whether preceded by the term "optionally" or not, refers to the replacement of hydrogen radicals in a given structure with the radical of a specified substituent. Unless otherwise indicated, an optionally substituted group may have a substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable", as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40°C or less, in the absence of moisture or other chemically reactive conditions, for at least a week.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocycle" "cycloaliphatic" or "cycloalkyl"), that has a single point of attachment to the rest of the molecule. Unless otherwise specified, aliphatic groups contain 1-20 aliphatic carbon atoms. In some embodiments, aliphatic groups contain 1-10 aliphatic carbon atoms. In other embodiments, aliphatic groups contain 1-8 aliphatic carbon atoms. In still other embodiments, aliphatic groups contain 1-6 aliphatic carbon atoms, and in yet other embodiments aliphatic groups contain 1-4 aliphatic carbon atoms. In some embodiments, "cycloaliphatic" (or "carbocycle" or "cycloalkyl") refers to a monocyclic C₃-Cₛ hydrocarbon or bicyclic C₈-C₁₂ hydrocarbon that is completely saturated or that contains one or more units of unsaturation, but which is not aromatic, that has a single point of attachment to the rest of the molecule wherein any individual ring in said bicyclic ring system has 3-7 members. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl.

The term "heteroaliphatic", as used herein, means aliphatic groups wherein one or two carbon atoms are independently replaced by one or more of oxygen, sulfur, nitrogen, phosphorus, or silicon. Heteroaliphatic groups may be substituted or unsubstituted, branched or unbranched, cyclic or acyclic, and include "heterocycle", "heterocyclyl", "heterocycloaliphatic", or "heterocyclic" groups.

The term "heterocycle", "heterocyclyl", "heterocycloaliphatic", or "heterocyclic" as used herein means non-aromatic, monocyclic, bicyclic, or tricyclic ring systems in which one or more ring members are an independently selected heteroatom. In some embodiments, the "heterocycle", "heterocyclyl", "heterocycloaliphatic", or "heterocyclic" group has three to fourteen ring members in which one or more ring members is a heteroatom independently selected from oxygen, sulfur, nitrogen, or phosphorus, and each ring in the system contains 3 to 7 ring members.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2H-pyrrolyl), NH (as in pyrrolidinyl) or NR' (as in N-substituted pyrrolidinyl)).

The term "unsaturated", as used herein, means that a moiety has one or more units of unsaturation.

The term "alkoxy", or "thioalkyl", as used herein, refers to an alkyl group, as previously defined, attached to the principal carbon chain through an oxygen ("alkoxy") or sulfur ("thioalkyl") atom.

The terms "haloalkyl", "haloalkenyl" and "haloalkoxy" means alkyl, alkenyl or alkoxy, as the case may be, substituted with one or more halogen atoms. The term "halogen" means F, Cl, Br, or I.

The term "aryl" used alone or as part of a larger moiety as in "aralkyl", "aralkoxy", or "aryloxyalkyl", refers to monocyclic, bicyclic, and tricyclic ring systems having a total of five to fourteen ring members, wherein at least one ring in the system is aromatic and wherein each ring in the system contains 3 to 7 ring members. The term "aryl" may be used interchangeably with the term "aryl ring". The term "aryl" also refers to heteroaryl ring systems as defined hereinbelow.

The term "heteroaryl", used alone or as part of a larger moiety as in "heteroaralkyl" or "heteroarylalkoxy", refers to monocyclic, bicyclic, and tricyclic ring systems having a total of five to fourteen ring members, wherein at least one ring in the system is aromatic, at least one ring in the system contains one or more heteroatoms, and wherein each ring in the system contains 3 to 7 ring members. The term "heteroaryl" may be used interchangeably with the term "heteroaryl ring" or the term "heteroaromatic".

An aryl (including aralkyl, aralkoxy, aryloxyalkyl and the like) or heteroaryl (including heteroaralkyl and heteroarylalkoxy and the like) group may contain one or more substituents and thus may be "optionally substituted". Unless otherwise defined above and herein, suitable substituents on the unsaturated carbon atom of an aryl or heteroaryl group are generally selected from halogen; -R°; -OR°; -SR°; phenyl (Ph) optionally substituted with R°; -O(Ph) optionally substituted with R°; -(CH₂)₁₋₂(Ph), optionally substituted with R°; -CH=CH(Ph), optionally substituted with R°; -N0₂; -CN; -N(R°)₂; -NR°C(O)R°; - NR°C(S)R°; -NR°C(O)N(R°)₂; -NR°C(S)N(R°)₂; -NR°CO₂R°; -NR°NR°C(O)R°; -NR'NR-C(O)N(R-)₂; -NR°NR°CO₂R°; -C(O)C(O)R°; -C(O)CH₂C(O)R°; -CO₂R°; - C(O)R°; -C(S)R°; -C(O)N(R°)₂; -C(S)N(R°)₂; -OC(O)N(R°)₂; -OC(O)R°; -C(O)N(OR°) R°; -C(NOR') R°; -S(O)₂R°; -S(O)₃R°; -S0₂N(R°)₂; -S(O)R°; -NR°SO₂N(R°)₂; -NR'S0₂R'; -N(OR°)R°; -C(=NH)-N(R°)₂₁ -P(O)₂R°; -PO(R°)_{2;} -OPO(R°)₂; -(CH₂)₀₋₂NHC(O)R°; phenyl (Ph) optionally substituted with R°; -O(Ph) optionally substituted with R°; -(CH₂)₁-₂(Ph), optionally substituted with R°; or -CH=CH(Ph), optionally substituted with R°; wherein each independent occurrence of R° is selected from hydrogen, optionally substituted C_{I}-₆ aliphatic, an unsubstituted 5-6 membered heteroaryl or heterocyclic ring, phenyl, -O(Ph), or -CH₂(Ph), or, notwithstanding the definition above, two independent occurrences of R°, on the same substituent or different substituents, taken together with the atom(s) to which each R° group is bound, to form an optionally substituted 3-12 membered saturated, partially unsaturated, or fully unsaturated monocyclic or bicyclic ring having 0-4 heteroatoms independently selected from nitrogen, oxygen, or sulfur.

Optional substituents on the aliphatic group of R° are selected from NH₂, NH(C₁. ₄aliphatic), N(C₁₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄ aliphatic), or haloC₁₋₄aliphatic, wherein each of the foregoing C₁₋₄aliphatic groups of R° is unsubstituted.

An aliphatic or heteroaliphatic group, or a non-aromatic heterocyclic ring may contain one or more substituents and thus may be "optionally substituted". Unless otherwise defined above and herein, suitable substituents on the saturated carbon of an aliphatic or heteroaliphatic group, or of a non-aromatic heterocyclic ring are selected from those listed above for the unsaturated carbon of an aryl or heteroaryl group and additionally include the following: =O, =S, =NNHR^{*}, =NN(R^{*})₂, =NNHC(O)R^{*}, =NNHCO₂(alkyI), =NNHS0₂(alkyl), or =NR^{*}, where each R^{*} is independently selected from hydrogen or an optionally substituted C₁₋₆ aliphatic group.

Unless otherwise defined above and herein, optional substituents on the nitrogen of a non-aromatic heterocyclic ring are generally selected from -R⁺, -N(R⁺)₂, -C(O)R⁺, -CO₂R⁺, -C(O)C(O)R⁺, -C(O)CH₂C(O)R⁺, -S0₂R⁺, -S0₂N(R⁺)₂, -C(=S)N(R⁺')₂, -C(=NH)-N(R⁺)₂, or -NR⁺SO₂R⁺; wherein R⁺ is hydrogen, an optionally substituted Cₜ₋6_{¡}aliphatic, optionally substituted phenyl, optionally substituted -O(Ph), optionally substituted -CH₂(Ph), optionally substituted -(CH₂)1₋2(Ph); optionally substituted -CH=CH(Ph); or an unsubstituted 5-6 membered heteroaryl or heterocyclic ring having one to four heteroatoms independently selected from oxygen, nitrogen, or sulfur, or, notwithstanding the definition above, two independent occurrences of R⁺, on the same substituent or different substituents, taken together with the atom(s) to which each R⁺ group is bound, form an optionally substituted 3-12 membered saturated, partially unsaturated, or fully unsaturated monocyclic or bicyclic ring having 0-4 heteroatoms independently selected from nitrogen, oxygen, or sulfur.

Optional substituents on the aliphatic group or the phenyl ring of R⁺ are selected from -NH₂, -NH(C₁-₄ aliphatic), -N(C₁-₄ aliphatic)₂, halogen, C¡-4 aliphatic, -OH, -O(C₁₋₄ aliphatic), -NO₂, -CN, -CO₂H, -CO₂(C₁₋₄ aliphatic), -O(halo C₁₋₄ aliphatic), or halo(C₁₋₄ aliphatic), wherein each of the foregoing C₁₋₄aliphatic groups of R⁺ is unsubstituted.

The term "alkylidene chain" refers to a straight or branched carbon chain that may be fully saturated or have one or more units of unsaturation and has two points of attachment to the rest of the molecule.

As detailed above, in some embodiments, two independent occurrences of R° (or R⁺, R, R' or any other variable similarly defined herein), are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

Exemplary rings that are formed when two independent occurrences of R° (or R⁺, R, R' or any other variable similarly defined herein), are taken together with the atom(s) to which each variable is bound include, but are not limited to the following: a) two independent occurrences of R° (or R⁺, R, R' or any other variable similarly defined herein) that are bound to the same atom and are taken together with that atom to form a ring, for example, N(R°)₂, where both occurrences of R° are taken together with the nitrogen atom to form a piperidin-1-yl, piperazin-1-yl, or morpholin-4-yl group; and b) two independent occurrences of R° (or R⁺, R, R' or any other variable similarly defined herein) that are bound to different atoms and are taken together with both of those atoms to form a ring, for example OR° 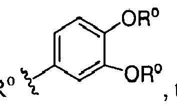 where a phenyl group is substituted with two occurrences of OR 0 va ORO these two where a phenyl group is substituted with two occurrences of OR° ' / ORo these two occurrences of R° are taken together with the oxygen atoms to which they are bound to form 0) a fused 6-membered oxygen containing ring: 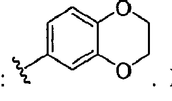 It will be appreciated that a variety of other rings can be formed when two independent occurrences of R° (or R⁺, R, R' or any other variable similarly defined herein) are taken together with the atom(s) to which each variable is bound and that the examples detailed above are not intended to be limiting.

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enriched carbon are within the scope of this invention. Such compounds are useful, for example, as analytical tools or probes in biological assays.

In certain embodiments, the methods described herein are useful for preparing compounds of formula Ia: 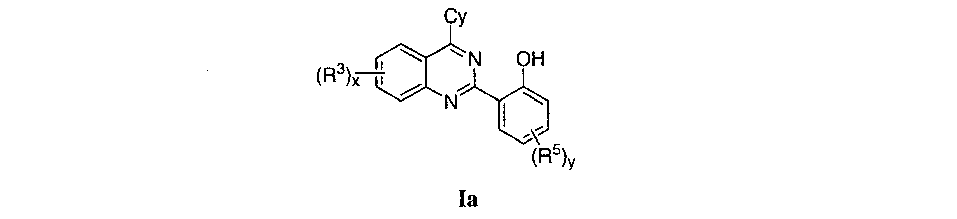 or suitable salts thereof;
wherein:
Cy is a ring selected from: 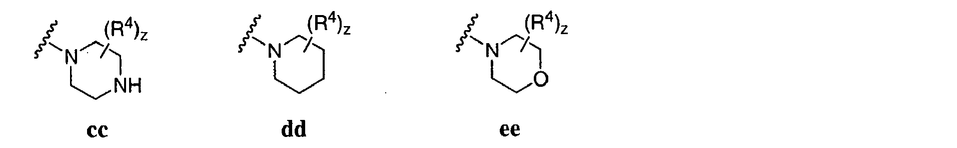 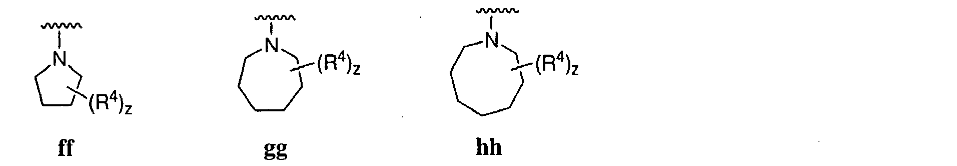 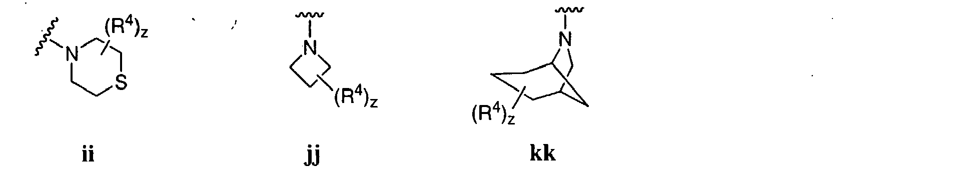 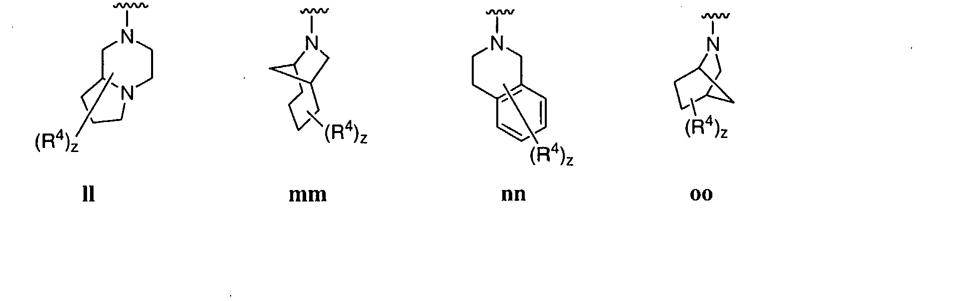 wherein Cy is optionally substituted at one or more substitutable carbon, nitrogen, or sulfur atoms with z independent occurrences of -R⁴;
each z is independently 0-5;
each R⁴ is independently halogen, CN, N0₂, -N(R')_{z}, -CH₂N(R')₂, -OR', -CH₂OR', -SR', -CH₂SR', -COOR', -NRCOR', -CON(R')₂, -OCON(R')2_{,} COR', -NHCOOR', -SO₂R', -S02N(R')z, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cyaoaliphaticcᵢ-C₆alkyl, or heterocycloaliphaticCₜ-C₆alkyl.
x is 0-4;
each R³ is independently halogen, CN, N0₂, -N(R')₂, -CH2N(R')z, -OR', -CH₂OR', -SR', -CH₂SR', -COOR', -NRCOR', -CON(R')₂, -OCON(R')₂, COR', -NHCOOR', -SO2R', -SO₂N(R')₂, or an optionally substituted group selected from C¡.C₆aliphatic, aryl, heteroaryl, cycloaliphatic, heterocycloaliphatic, arylCᵢ-C₆alkyl, heteroarylCₗ-C₆alkyl, cycloaliphaticC¡-C₆alkyl, or heterocycloaliphaticC1-C₆alkyl.
y is 0-5;
each R^{S} is independently halogen, CN, N0₂, -N(R')2_{,} -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R'; -OP(O)(OR')2_{,} -P(O)(OR')z, -OP(0)₂0R', -P(O)zOR', -PO(R')2_{,} -OPO(R')2_{,} or an optionally substituted group selected from Cₗ-C₆aliphatic, aryl, heteroaryl, cycloaliphatic, heterocycloaliphatic, arylC_{I}-C₆alkyl, heteroary]C,-C₆alkyl, cycloaliphaticcᵢ-C₆alkyl, or heterocycloaliphaticcᵢ-C₆alkyl; and
each occurrence of R' is independently hydrogen or an optionally substituted C₁₋₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to
form an optionally substituted 3-12 membered saturated, partially unsaturated, or fully unsaturated monocyclic or bicyclic ring having 0-4 heteroatoms independently selected from nitrogen, oxygen, or sulfur.

In other embodiments, the methods described herein are useful for preparing compounds of formula Ia: 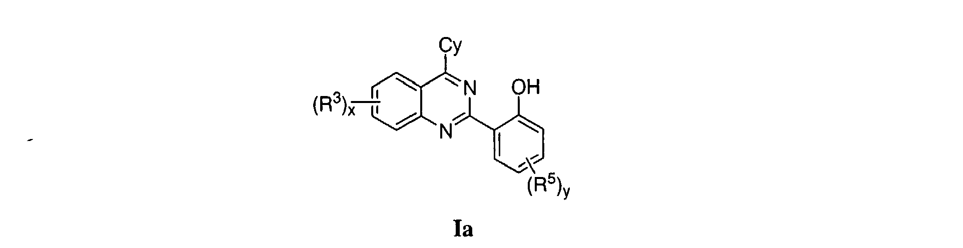 or suitable salts thereof; wherein:
Cy is azetidin-1-yl (j,j), pyrrolidin-1-yl (ff), piperidinl-yl (dd), or piperazin-1-yl (cc), wherein
Cy is optionally substituted with 0-4 occurences of R⁴;
each R⁴ is independently Cl, Br, F, CF₃, CH₃, -CH₂CH₃, CN, -COOH, -N(CH₃)₂, -N(Et)₂, -N(iPr)₂, -O(CH₂)₂0CH₃, -CONH₂, -COOCH₃, -OH, -CH₂OH, -NHCOCH₃, -S0₂NH₂, _{-S02(CH2})_{3CH3}, _{-S02CH}(_{CH3})₂, -S0_{2N}(CH₃)₂, -SO_{2C}H₂CH₃, -C(O)OCH₂CH(CH₃)₂, -C(O)NHCH₂CH(CH₃)₂, -C(O)CH(OH)CH₂CH(CH₃)₂, -C(O)CH(OH)CH₂C(CH₃)₃, -NHCOOCH₃, -C(O)C(CH₃)₃, -COO(CH₂)₂CH₃, -C(O)NHCH(CH₃)₂, -C(O)CH₂CH₃, or an optionally substituted group selected from -piperidinyl, piperizinyl, morpholino, Cₜ. ₄alkoxy, phenyl, phenyloxy, benzyl, benzyloxy, -CH_{2C}yclohexyl, pyridyl, -CH₂pyridyl, or -CH₂thiazolyl;
x is 1 or 2;
each occurrence of R³ is independently Cl, Br, F, CF₃, -OCF₃, Me, Et, CN, -COOH, -NH₂, -N(CH₃)₂, -N(Et)₂, -N(iPr)₂, -O(CH₂)₂0CH₃, -CONH₂, -COOCH₃, -OH, -OCH₃, - OCH₂cH₃, -CH₂OH, -NHCOCH₃, -NHCOCH(CH₃)₂, -S0₂NH₂, -CONH(cyclopropyl), - CONHCH₃, -CONHCH₂CH₃, or an optionally substituted group selected from - piperidinyl, piperizinyl, morpholino, phenyl, phenyloxy, benzyl, or benzyloxy;
y is 0-4; and
each R^{S} is independently Cl, Br, F, CF₃, Me, Et, CN, -COOH, -NH₂, -N(CH₃)₂, -N(Et)₂, - N(iPr)₂, -O(CH₂)₂0CH₃, -CONH₂, -COOCH₃, -OH, -OCH₃, -OCH_{z}CH₃, -CH₂OH, - NHCOCH₃, -S0₂NH₂, -s0₂NHC(CH₃)₂, -OCOC(CH₃)₃, -OCOCH₂C(CH₃)₃, - 0(CH_{z})_{z}N(CH₃)_{z}, 4-CH₃-piperazin-l-yt, OCOCH(CH₃)₂, OCO(cyclopentyl), -COCH₃, optionally substituted phenoxy, or optionally substituted benzyloxy.

In still other embodiments, the methods described herein are useful for preparing compounds of formula Ia wherein x is 1 and R³ is at the 7-position of the quinazoline ring and is -Cl, -CH₃, -CH₂CH₃, -F, -CF₃, -OCF₃, -CONHCH₃, -CONHCH₂CH₃, - CONH(cyclopropyl), -OCH₃, -NH₂, -OCH₂CH₃, or -CN. In yet other embodiments, x is 1 and R³ is at the 7-position of the quinazoline ring and is -Cl, -CH₃, -CH₂CH₃, -F, -CF₃, - OCF₃, -OCH₃, or -OCH₂CH₃. In certain other embodiments, x is 1 and R³ is at the 7-position of the quinazoline ring and is methyl.

According to another embodiment, Cy is piperazin-1-yl (cc), y is 0, x is 1 and R³ is at the 7-position of the quinazoline ring and is methyl.

According to yet another embodiment, the methods described herein are useful for preparing compounds of formula **Ia**: 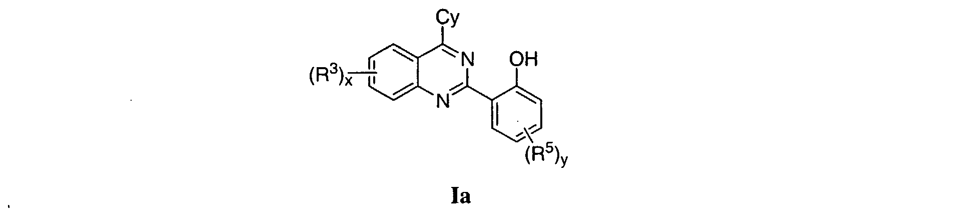 or suitable salts thereof; wherein:
Cy is an optionally substituted ring selected from azetidin-1-yl (jj), pyrrolidin-1-yl (ff),
piperidinl-yl (**dd**), or piperazin-1-yl (cc), wherein Cy is optionally substituted with 0-4 occurences of R⁴;
each R⁴ is independently Cl, Br, F, CF₃, CH₃, -CH₂CH₃, CN, -COOH, -N(CH₃)₂, -N(Et)₂, -N(iPr)₂, -O(CH₂)₂0CH₃, -CONH₂, -COOCH₃, -OH, -CH₂OH, -NHCOCH₃, -S0₂NH₂, -S0₂(CH₂)₃CH₃, -S0₂CH(CH₃)₂, -SO₂N(CH₃)₂, -S0₂CH₂CH₃, -C(O)OCH₂CH(CH₃)₂, _{-C}(_{O})_{NHCH2CH}(_{CH3})₂, -C(_{O})_{CH}(OH)CH_{2C}H(CH₃)₂, -C(O)CH(OH)CH₂C(CH₃)₃, -NHCOOCH₃, -C(O)C(CH₃)₃, -coo(CH₂)₂CH₃, -C(O)NHCH(CH₃)₂, -C(O)CH₂CH₃, or an optionally substituted group selected from -piperidinyl, piperizinyl, morpholino, Cₜ. ₄alkoxy, phenyl, phenyloxy, benzyl, benzyloxy, -CH2_{C}yclohexyl, pyridyl, -CH_{2P}yridyl, or -CH₂thiazolyl;
x is 1;
each R³ is independently Cl, Br, F, CF₃, -OCF₃, Me, Et, CN, -COOH, -OH, or -OCH₃; y is 0 or 1; and
each R⁵ is independently Cl, Br, F, CF₃, Me, -OH, -OCH₃, -OCH₂CH₃, -CH₂OH, - NHCOCH₃, -SO₂NH₂, -S0₂NHC(CH₃)₂.

In certain embodiments, the methods described herein are useful for preparing compounds of formula **Ia**: 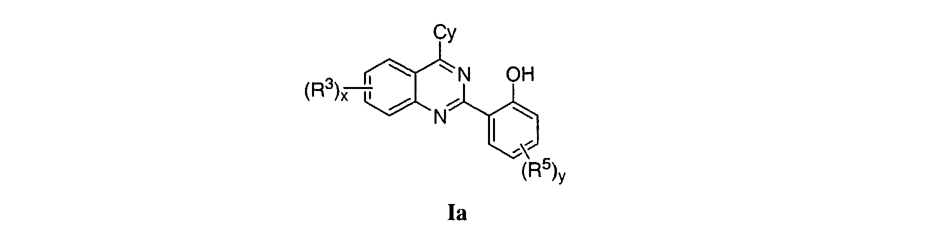 or suitable salts thereof;
wherein:
Cy is unsubstituted piperazin-1-yl, x is 1 and y is 0.

In certain other embodiments, the methods described herein are useful for preparing compounds of formula **Ia**: 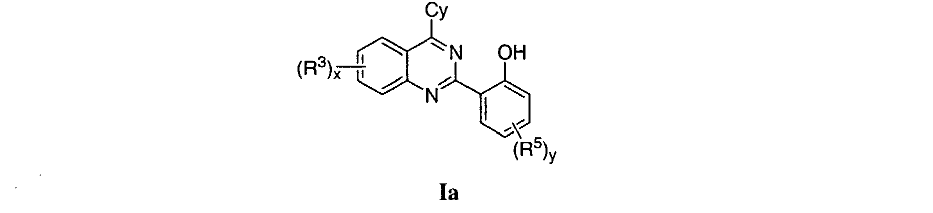 or suitable salts thereof; wherein:
Cy is piperazin-1-yl optionally substituted on the nitrogen with R⁴, x is 1 and y is 0.

Compounds of formula Ia are prepared generally as depicted in Scheme I, below. Scheme I

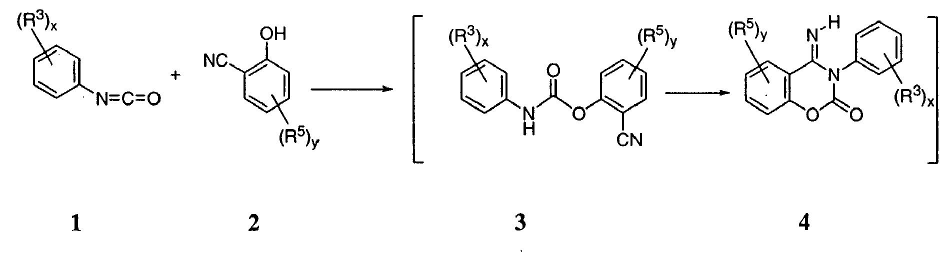

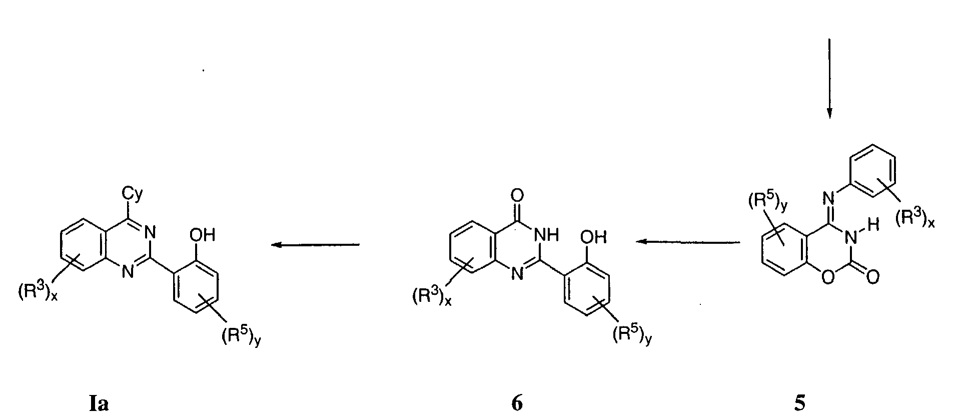

Scheme I above depicts a general method for preparing compounds of formula **Ia**. As is readily apparent, such compounds of formula Ia correspond to compounds of formula **I** wherein R ⁵a is -OH. One of ordinary skill in the art would recognize that a variety of compounds of formula **I**, wherein R ⁵a is other than -OH are prepared from intermediate **6** or a suitable salt thereof using methods known in the art. For example, the -OH group of intermediate **6** may be converted to a suitable leaving group. As used herein, a suitable leaving group is a chemical moiety that is readily displaced by a desired incoming chemical moiety. Suitable leaving groups are well known in the art, e.g., see, "Advanced Organic Chemistry," Jerry March, 4th Ed., pp. 351-357, John Wiley and Sons, N.Y. (1992) and "Comprehensive Organic Transformations," Larock, Richard C., 2°d Ed., John Wiley & Sons, 1999, the contents both of which are incorporated herein by reference.

Such leaving groups include, but are not limited to, halogen, alkoxy, sulphonyloxy, optionally substituted alkylsulphonyl, optionally substituted alkenylsulfonyl, optionally substituted arylsulfonyl, and diazonium moieties.

The suitable leaving group may then be displaced by a variety of moieties to form compounds of formula **I**. Thus, it will be appreciated that after the hydroxyl group of intermediate **6** is converted to a suitable leaving group, a variety of functional groups may be incorporated to form a compound of formula **I** having a variety of R^{5a} groups. For example, said leaving group by be displaced by halogen, a haloalkyl moiety, an alkyl moiety, CN, a carboxylate moiety, NH₃, NH(CH₃)_{z}, N(Et)₂, NH(iPr)₂, HO(CH₂)₂OCH₃, HCONH₂, HCOOCH₃, HOCH₃, HOCH₂CH₃, HCH₂OH, NH₂COCH₃, HS0₂NH₂, HSO₂NHC(CH₃)₂, HOCOC(CH₃)₃, HOCOCH₂C(CH₃)₃, HO(CH₂)₂N(CH₃)₂, 4-CH₃-piperazin-l-yl, HOCOCH(CH₃)₂, HOCO(cyclopentyl), HCOCH₃, optionally substituted phenoxy, or optionally substituted benzyloxy to form a compound of formula **I**. One of ordinary skill in the art would also recognize that these groups may be activated in order to affect said displacement.

According to another embodiment of the present invention, the methods described herein are useful for preparing compound **Iaa-1** or a suitable salt thereof: 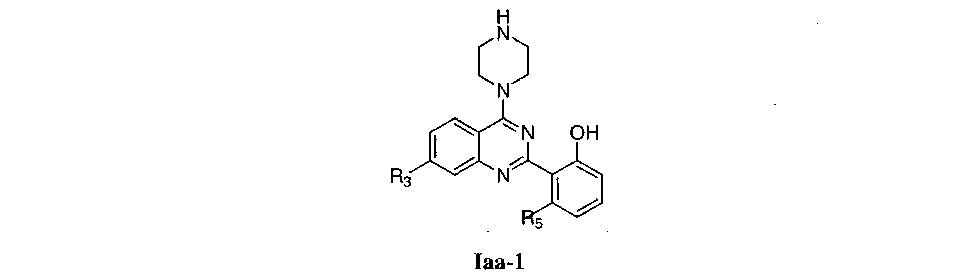 wherein R³ is methyl or hydrogen and R^{S} is fluorine or hydrogen.

According to another embodiment of the present invention, the methods described herein are useful for preparing a compound of formula V from a compound of formula **Iaa-1**: 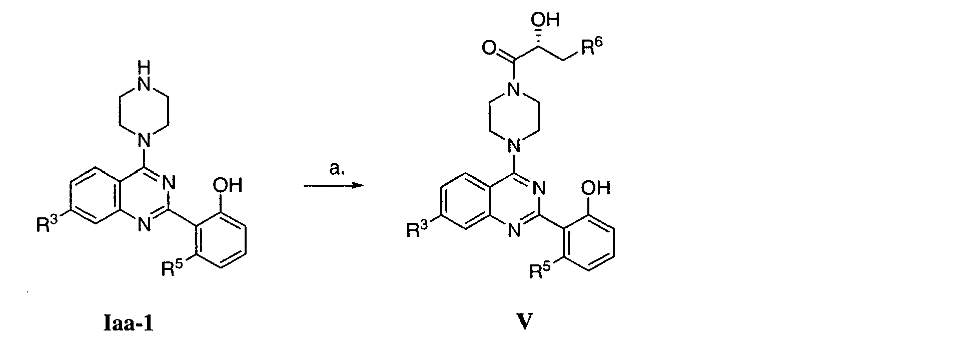 comprising the additional step of:
(a) reacting a compound of formula Iaa-1 with with a suitable acid under suitable amide coupling conditions; wherein R⁶ is isopropyl or t-butyl, R³ is methyl or hydrogen, and R⁵ is fluorine or hydrogen.

In one embodiment of compounds of formula V, R⁶ is isopropyl, R³ is methyl, and R⁵ is hydrogen. In another embodiment of formula V, R⁶ is t-butyl, R³ is methyl, and R⁵ is hydrogen. In yet another embodiment of formula V, R⁶ is isopropyl, R³ is hydrogen, and R⁵ - is hydrogen. In yet another embodiment of formula V, R⁶ is t-butyl, R³ is methyl, and R⁵ is fluorine. Or, in formula V, R⁶ is t-butyl, R³ is hydrogen, and R^{S} is fluorine.

In one embodiment, suitable amide coupling conditions include a variety of commonly used organic solvents (such as methylene chloride, THE, ethyl acetate, acetonitrile, DMF, etc.), commercially available amide coupling reagents known to those skilled in the art (such as EDC, BOP, BOP-Cl, DCC, HOBt, etc.), inorganic (such as K₂C0₃, Na₂C0₃, C_{S2}C0₃) or organic bases (Et₃N, Hunigs base, N-methylmorpholine, imidazole, 4-DMAP,etc.) and a suitable reaction temperature (from 0°C to greater than 100°C) and a suitable atmosphere (such as air, nitrogen , argon, etc.). In one embodiment for preparing compounds of formula V, the organic solvent in DMF, the the coupling agents are EDC and HOBt, the organic base is 4-methylmorpholine, the atmosphere is nitrogen adn the temperature is room temperature.

In another embodiment, the method further comprises the step of forming a salt of the compound of formula **V**. In one embodiment the salt is a methanesulfonic acid salt.

One of ordinary skill in the art would recognize that compounds of formula V may be prepared using methods known in the art. For instance, for preparing compound **V**, wherein R⁶ is isopropyl or t-butyl, the commercially available or synthesized acid intermediate coupling partner is used along with the suitable amide coupling reagents either with or without added organic or inorganinc base and in a variety of commonly used organic solvents. In one embodiment, wherein R⁶ is isopropyl, one of skill in the art would be able to make the coupling partner isocaproic acid from leucine by known organic chemistry techniques. Finally, one of skill in the art would recognize that the free base of compounds of formula V may be converted to a suitable salt for further purification. In one embodiment, the methanesulfonic acid salt is useful for purifying compounds of formula **V**.

In another embodiment, the compound of formula **Ia** is produced as a salt of a sulfonic acid or a dicarboxylic acid. The specific sulfonic acid or dicarboxylic acid useful for producing the salt of compound of formula **Ia** may be selected from acids known in the art. See, e.g., "Practical Process, Research, & Development," Anderson, Neal G., Academic Press, 2000, the contents of which are incorporated herein by reference.

According to one embodiment, the compound of formula **Ia** is produced as a salt of a sulfonic acid. Exemplary sulfonic acids include methylsulfonic acid, p-toluenesulfonic acid, etc. According to one embodiment, the compound of formula **Ia** is produced as a methylsulfonic acid salt. According to another embodiment, the compound of formula **Ia** is produced as a salt of a dicarboxylic acid. In one embodiment, the dicarboxylic acid is selected from oxalic acid, malonic acid, succinic acid, maleic, or fumaric acid. Or, the dicarboxyclic acid is oxalic acid.

In certain embodiments, the present invention provides a method for preparing a compound of formula **Ia**: 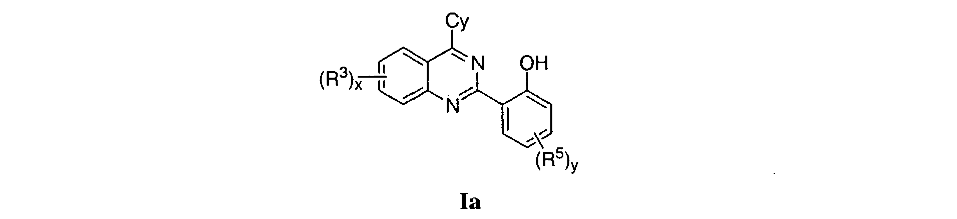 or a suitable salt thereof;
wherein:
Cy is a ring selected from: 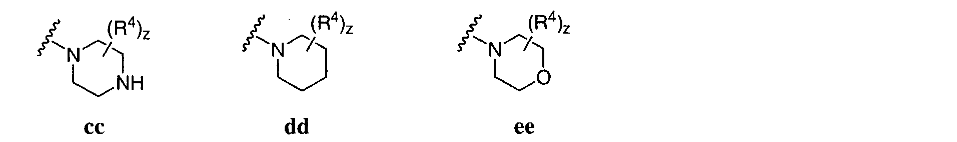 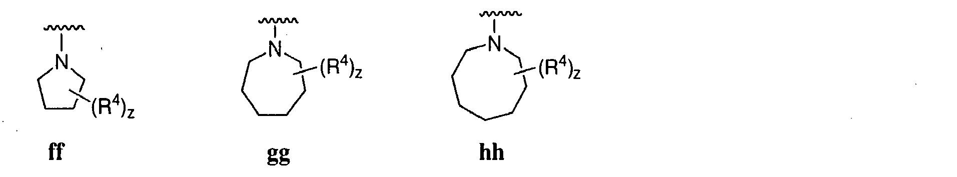 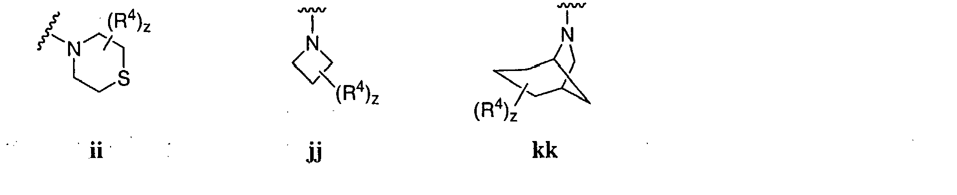 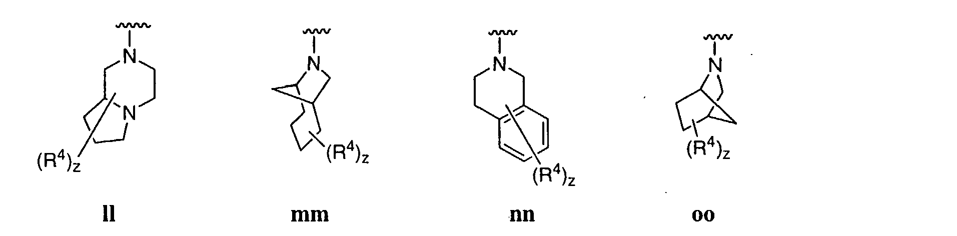 and Cy is optionally substituted at one or more substitutable carbon, nitrogen, or sulfur atoms with z independent occurrences of -R⁴;
each z is independently 0-5;
each R⁴ is independently halogen, CN, N0₂, -N(R')_{2,} -CH₂N(R')2_{,} -OR', -CH₂OR', -SR',
-CH_{z}SR', -COOR', -NRCOR', -CON(R')₂, -OCON(R')₂, COR', -NHCOOR', - SO₂R', -SO2N(R')₂, or an optionally substituted group selected from C₁-C₆aliphatic, aryl, heteroaryl, cycloaliphatic, heterocycloaliphatic, arylC₁-C₆alkyl, heteroarylcₗ-C₆alkyl, cycloaliphaticCt-C6alkyl, or heterocycloaliphaticC₁-C₆alkyl;
x is 0-4;
each R³ is independently halogen, CN, NO₂, -N(R')₂, -CH₂N(R')_{2,} -OR', -CH₂OR', -SR',
-CH₂SR', -COOR', -NRCOR', -CON(R')₂, -OCON(R')₂, COR', -NHCOOR', -
SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylCₗ-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')2_{,} -OR', -CH_{z}OR', -
SR', -CH₂SR', -NRCOR', -CON(R')_{2,} -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticc,-C₆alkyl; and
each occurrence of R' is independently hydrogen or an optionally substituted C₁₋₆
aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms-independently selected from nitrogen, oxygen, or sulfur; or: two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
comprising the steps of:
(a) providing a compound of formula **II**: 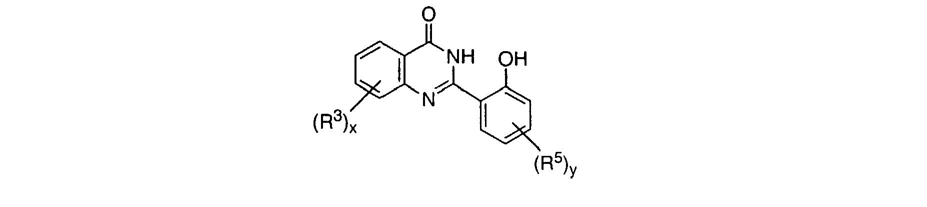 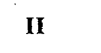 or a suitable salt thereof;
   wherein:
   x is 0-4;
   each R³ is independently halogen, CN, N0₂, -N(R')₂, -CH₂N(R')2_{,} -OR', -CH₂OR', -SR', -CH₂SR', -COOR', -NRCOR', -CON(R')2_{,} -OCON(R')₂, COR', -NHCOOR', -SO₂R', -SO₂N(R')2_{,} or an optionally substituted group selected from C₁-C₆aliphatic, aryl, heteroaryl, cycloaliphatic, heterocycloaliphatic, arylC₁-C₆alkyl, heteroaryl)-C₆alkyl, cycloaliphaticC₁-C₆alkyl, or heterocycloaliphaticC₁-C₆alkyl;
   yis0-5;
   each R⁵ is independently halogen, CN, N0₂, -N(R')₂, -CH₂N(R')2_{,} -OR', -CH₂OR', -SR', -CH₂SR', -NRCOR', -CON(R')₂, -S(O)₂N(R')₂, -OCOR', -COR', -CO₂R', - OCON(R')2_{,} -NR'SO₂R', -OP(O)(OR')₂, -P(O)(OR')2_{,} -OP(O)₂OR', -P(O)₂OR' , - PO(R')2_{,} -OPO(R')2_{,} or an optionally substituted group selected from C₁-C₆aliphatic, aryl, heteroaryl, cycloaliphatic, heterocycloaliphatic, arylC₁-C₆alkyl, heteroaryIC)-C₆alkyl, cyaoaliphaticC₁-C₆alkyl, or heterocycloaliphaticC₁-C₆alkyl; and
   each occurrence of R' is independently hydrogen or an optionally substituted C₁₋₆
   aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen; oxygen, or sulfur; or: ¹.
   two occurrences of R' are taken together with the atom(s) to which they are bound to form an optionally substituted 3-12 membered saturated, partially unsaturated, or fully unsaturated monocyclic or bicyclic ring having 0-4
   heteroatoms independently selected from nitrogen, oxygen, or sulfur;
   and
(b) converting said compound of formula **II** or a suitable salt thereof to a compound of formula **Ia**.

In certain embodiments, the method of preparing a compound of formula **Ia** or a suitable salt thereof from a compound of formula **II** or a suitable salt thereof further comprises the steps of:
(a) protecting the hydroxyl group of compound **II** with a suitable hydroxyl protecting group to form a compound of formula **IIa**: 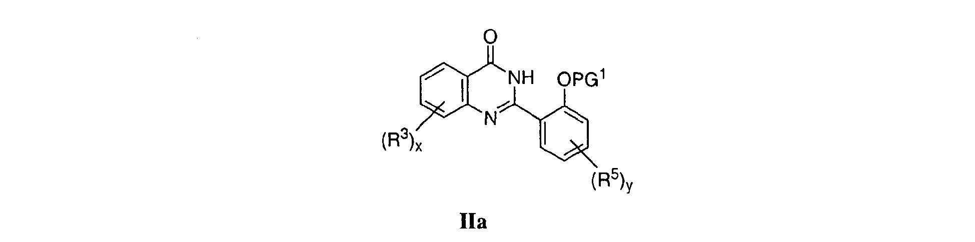 or a suitable salt thereof;
   wherein:
   PG' is a suitable hydroxyl protecting group;
   x is 0-4;
   each R³ is independently halogen, CN, NO₂, -N(R')2_{,} -CH₂N(R')2_{,} -OR', -CH_{Z}OR', -SR', -CH₂SR', -COOR', -NRCOR', -CON(R')₂, -OCON(R')2_{,} COR', -NHCOOR', -SO₂R', -SO₂N(R')2_{,} or an optionally substituted group selected from C₁₋C₆aliphatic, aryl, heteroaryl, cycloaliphatic, heterocycloaliphatic, arylC₁-C₆alkyl, heteroarylcₗ-C₆alkyl, cycloaliphaticC₁-C₆alkyl, or heterocycloaliphaticC₁-C₆alkyl;
   y is 0-5;
   each R⁵ is independently halogen, CN, NO₂, -N(R')2_{,} -CH₂N(R')2_{,} -OR', -CH₂OR', -SR', -CH₂SR', -NRCOR', -CON(R')2_{,} -S(O)₂N(R')₂, -OCOR', -COR', -CO₂R', - OCON(R')2_{,} -NR'SO₂R', -OP(O)(OR')₂, -P(O)(OR')₂, -OP(O)₂OR', -P(O)₂OR' , - PO(R')2_{,} -OPO(R')2_{,} or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
   each occurrence of R' is independently hydrogen or an optionally substituted C₁-₆
   aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound
   to form an optionally substituted 3-12 membered saturated, partially unsaturated, or fully unsaturated monocyclic or bicyclic ring having 0-4 heteroatoms independently selected from nitrogen, oxygen, or sulfur;
(b) converting the the ketone moiety of the compound of formula **IIa** or a suitable salt thereof, to a suitable leaving group to form a compound of formula **IIb**: 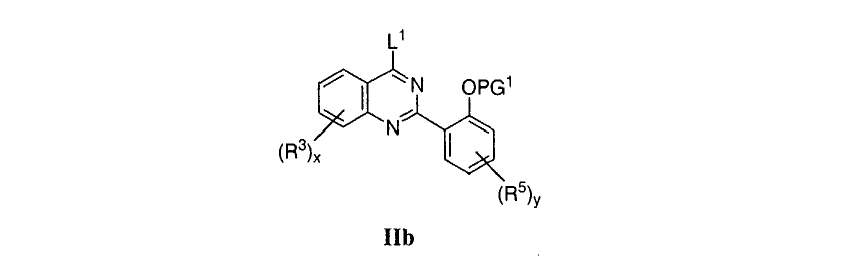 or a suitable salt thereof;
   wherein:
   PG' is a suitable hydroxyl protecting group;
   L^{l} is a suitable leaving group;
   x is 0-4;
   each R³ is independently halogen, CN, NO₂, -N(R')2_{,} -CH₂N(R')2_{,} -OR', -CH₂OR', -SR', -CH₂SR', -COOR', -NRCOR', -CON(R')₂, -OCON(R')₂, COR', -NHCOOR', -SO₂R', -SO₂N(R')2_{,} or an optionally substituted group selected from C₁-C₆aliphatic, aryl, heteroaryl, cycloaliphatic, heterocycloaliphatic, arylC₁-C₆alkyl, heteroarylc₁-C₆alkyl, cycloaliphaticC₁-C₆alkyl, or heterocycloaliphaticC₁-C₆a]kyl;
   y is 0-5;
   each R⁵ is independently halogen, CN, NO₂, -N(R')2_{,} -CH₂N(R')2_{,} -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')2_{,} -OP(O)₂OR', -P(O)₂OR' , - PO(R')2_{,} -OPO(R')2_{,} or an optionally substituted group selected from C₁-C₆aliphatic, aryl, heteroaryl, cycloaliphatic, heterocycloaliphatic, arylC₁-C₆a]kyl, heteroarylcₗ-C₆alkyl, cycloaliphaticC₁-C₆alkyl, or heterocycloaliphaticC₁-C₆alkyl; and
   each occurrence of R' is independently hydrogen or an optionally substituted C₁₋₆
   aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
(c) displacing said suitable leaving group with a suitable Cy moiety to form a compound of formula IIc: 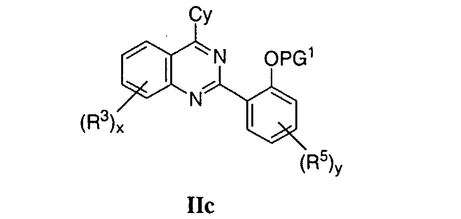 or a suitable salt thereof;
   wherein:
   PG' is a suitable hydroxyl protecting group;
   Cy is a ring selected from: 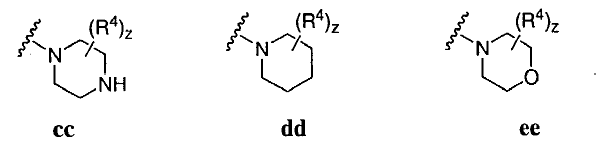 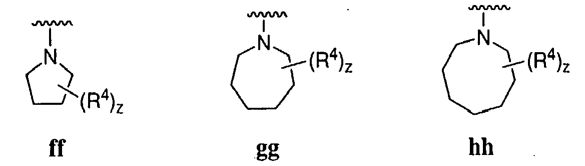 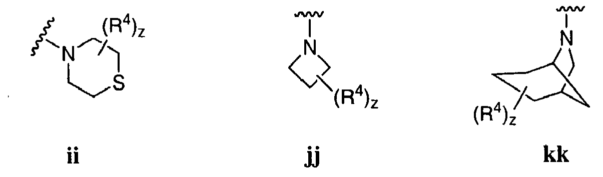 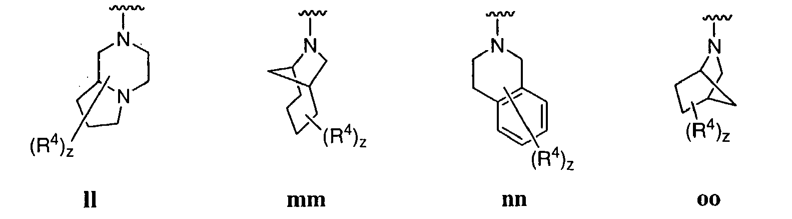 and Cy is optionally substituted at one or more substitutable carbon, nitrogen, or sulfur atoms with z independent occurrences of -R⁴;
   each z is independently 0-5;
   each R⁴ is independently halogen, CN, N0₂, -N(R')₂, -CH₂N(R')2_{,} -OR', -CH₂OR', -SR',
   -CH₂SR', -COOR', -NRCOR', -CON(R')₂, -OCON(R')₂, COR', -NHCOOR', -
   S0₂R', -S0₂N(R')2_{,} or an optionally substituted group selected from Cₗ₋C₆aliphatic, aryl, heteroaryl, cycloaliphatic, heterocycloaliphatic, arylC_{I}-C₆alkyl, heteroarylcₗ-C₆alkyl, cy_{d}oaliphaticcᵢ-C₆alkyl, or heterocycloaliphaticC¡-C₆alkyl;
   x is 0-4;
   each R³ is independently halogen, CN, N0₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO2R', -SO_{z}N(R')₂, or an optionally substituted group selected from C¡.C₆aliphatic, aryl, heteroaryl, cycloaliphatic, heterocycloaliphatic, arylC₁-C₆alkyl, heteroarylcₗ-C₆alkyl, cycloaliphaticCₗ-C₆alkyl, or heterocycloaliphaticC₁-C₆alkyl;
   y is 0-5;
   each R⁵ is independently halogen, CN, NO₂, -N(R')2_{,} -CH₂N(R')₂, -OR', -CH_{z}OR', -SR', -CH₂SR', -NRCOR', -CON(R')₂, -S(O)₂N(R')₂, -OCOR', -COR', -CO₂R', - OCON(R')2_{,} -NR'S02_{R}', -OP(O)(OR')2_{,} -P(O)(OR')₂, -OP(O)₂0R' , -P(O)₂0R' , - PO(R')2_{,} -OPO(R')2_{,} or an optionally substituted group selected from Cₗ₋C₆aliphatic, aryl, heteroaryl, cycloaliphatic, heterocycloaliphatic, arylC_{I}-C₆alkyl, heteroarylcₗ-C₆alkyl, cycloaliphaticC,-C₆alkyl, or heterocycloaliphaticC₁-C₆alkyl; and
   each occurrence of R' is independently hydrogen or an optionally substituted C₁-6
   aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound
   to form an optionally substituted 3-12 membered saturated, partially unsaturated, or fully unsaturated monocyclic or bicyclic ring having 0-4 heteroatoms independently selected from nitrogen, oxygen, or sulfur;
   and
(d) removing the suitable hydroxyl protecting group to form a compound of formula Ia or a suitable salt thereof.

Suitable hydroxyl protecting groups are well known in the art and include those described in detail in "Protecting Groups in Organic Synthesis", T. W. Greene and P. G. M. Wuts, 3`d edition, John Wiley & Sons, 1999, the entirety of which is incorporated herein by reference. Examples of suitable hydroxyl protecting group PG' of compounds of formulae IIa, IIb, and IIc further include, but are not limited to, esters, allyl ethers, ethers, silyl ethers, alkyl ethers, arylalkyl ethers, and alkoxyalkyl ethers. Examples of such esters include formates, acetates, carbonates, and sulfonates. Specific examples include formate, benzoyl formate, chloroacetate, trifluoroacetate, methoxyacetate, triphenylmethoxyacetate, p-chlorophenoxyacetate, 3-phenylpropionate, 4-oxopentanoate, 4,4-(ethylenedithio)pentanoate, pivaloate (trimethylacetyl), crotonate, 4-methoxy-crotonate, benzoate, p-benylbenzoate, 2,4,6-trimethylbenzoate, carbonates such as methyl, 9-fluorenylmethyl, ethyl, 2,2,2-trichloroethyl, 2-(trimethylsilyl)ethyl, 2-(phenylsulfonyl)ethyl, vinyl, allyl, and p-nitrobenzyl. Examples of such silyl ethers include trimethylsilyl, triethylsilyl, t-butyldimethylsilyl, t-butyldiphenylsilyl, triisopropylsilyl, and other trialkylsilyl ethers. Alkyl ethers include methyl, benzyl, p-methoxybenzyl, 3,4-dimethoxybenzyl, trityl, t-butyl, allyl, and allyloxycarbonyl ethers or derivatives. Alkoxyalkyl ethers include acetals such as methoxymethyl, methylthiomethyl, (2-methoxyethoxy)methyl, benzyloxymethyl, beta-(trimethylsilyl)ethoxymethyl, and tetrahydropyranyl ethers. Examples of arylalkyl ethers include benzyl, p-methoxybenzyl (MPM), 3,4-dimethoxybenzyl, O-nitrobenzyl, p-nitrobenzyl, p-halobenzyl, 2,6-dichlorobenzyl, p-cyanobenzyl, 2- and 4-picolyl. In certain embodiments, the suitable hydroxyl protecting group PG¹ of compounds of formulae IIa, IIb, and **IIc** is an ester group. In other embodiments, the suitable hydroxyl protecting group PG¹ of compounds of formulae IIa, lib, and IIc is a pivaloate (trimethylacetyl) group. In certain embodiments, the suitable hydroxyl protecting group PG' of compounds of formulae IIa, lib, and IIc is an ether group. In other embodiments, the suitable hydroxyl protecting group PG' of compounds of formulae IIa, lib, and IIc is a methyl ether group.

Methods of adding and removing such hydroxyl protecting groups are well-known in the art and available, for example, in P. J. Kocienski, Protecting Groups, Thieme, 1994, (which is hereby incorpoarted in its entirety by reference) and in T. W. Greene and P. G. M. Wuts, Protective Groups in Organic Synthesis, 3`d edition, John Wiley & Sons, 1999. One of ordinary skill in the art would recognize that the method appropriate to achieve removal of the protecting group of a compound of formula lie, at step (d), depends upon the actual protecting groups used and includes those described by Greene. For example, when said hydroxyl protecting group of a compound of formula IIc is an ester group, such removal may be achieved by saponification.

As used herein, a suitable leaving group is a chemical moiety that is readily displaced by a desired incoming chemical moiety. Suitable leaving groups are well known in the art, e.g., see, "Advanced Organic Chemistry," Jerry March, 4th Ed., pp. 351-357, John Wiley and Sons, N.Y. (1992) and "Comprehensive Organic Transformations," Larock, Richard C., 2"d Ed., John Wiley & Sons, 1999. Examples of suitable leaving group L^{l} of formula lib include, but are not limited to, halogen, alkoxy, sulphonyloxy, optionally substituted alkylsulphonyl, optionally substituted alkenylsulfonyl, optionally substituted arylsulfonyl, and diazonium moieties. Examples of suitable leaving group L¹ of formula IIb include chloro, iodo, bromo, fluoro, methanesulfonyl (mesyl), tosyl, triflate, nitro-phenylsulfonyl (nosyl), and bromo-phenylsulfonyl (brosyl). In certain embodiments, the suitable leaving group L¹ of formula lib is a halogen group. In other embodiments, the suitable leaving group L of formula IIb is a chloro group.

According to an alternate embodiment, the suitable leaving group may be generated *in situ* within the reaction medium. For example, a leaving group may be generated *in situ* from a precursor of that compound wherein said precursor contains a group readily replaced by said leaving group *in situ.*

In other embodiments, the preparation of a compound of formula Ia from a compound of formula II further comprises the step of forming a salt of the compound of formula Ia. According to one aspect of the present invention, salt is the oxalic acid salt. According to another aspect of the present invention, the compound of formula Ia is treated with oxalic acid to form the oxalic acid salt thereof then that salt is freebased and treated with methanesulfonic acid to form the mesylate salt of a compound of formula Ia.

According to another embodiment, the present invention provides a method for preparing a compound of formula Ia: 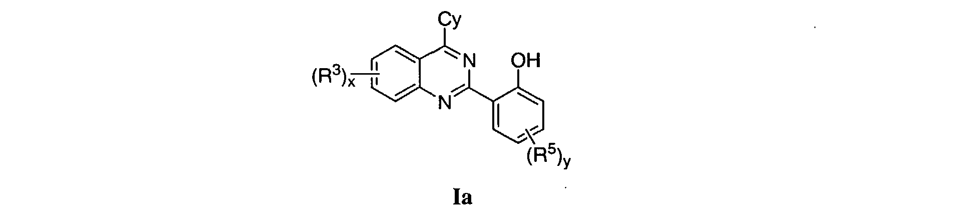 or a suitable salt thereof;
wherein:
Cy is a ring selected from: 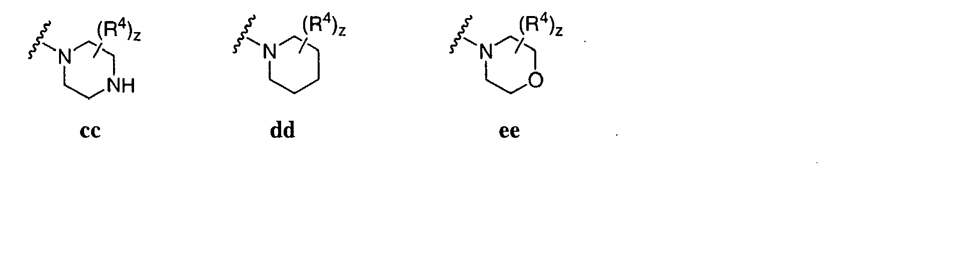 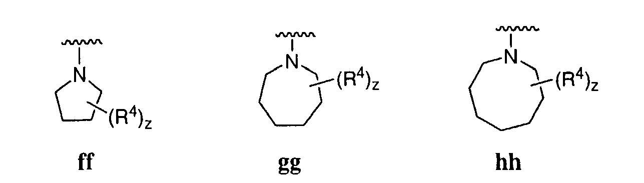 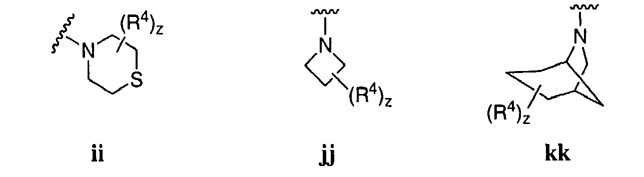 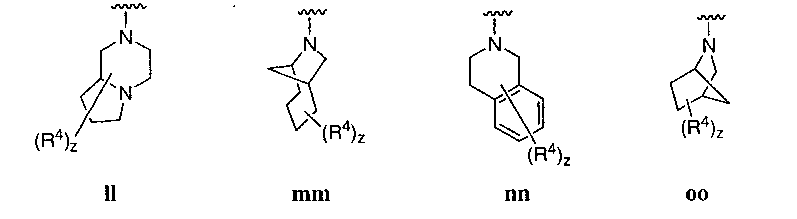 and Cy is optionally substituted at one or more substitutable carbon, nitrogen, or sulfur atoms with z independent occurrences of -R⁴;
each z is independently 0-5;
each R⁴ is independently halogen, CN, N0₂, -N(R')₂, -CH₂N(R')₂, -OR', -CH₂OR', -SR', -CH₂SR', -COOR', -NRCOR', -CON(R')₂, -OCON(R')2_{,} COR', -NHCOOR', - SO_{Z}R', -S0₂N(R')2_{,} or an optionally substituted group selected from C¡.C₆aliphatic, aryl, heteroaryl, cycloaliphatic, heterocycloaliphatic, arylCₜ-C₆alkyl, heteroarylcₗ-C₆alkyl, cycloaliphaticC,-C₆alkyl, or heterocycloaliphaticC,-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')2_{,} -OCON(R')_{2,} COR', -NHCOOR', - S0₂R', -S0₂N(R')2_{,} or an optionally substituted group selected from C¡_C₆aliphatic, aryl, heteroaryl, cycloaliphatic, heterocycloaliphatic, arylC₁-C₆alkyl, heteroarylcₗ-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(0)₂N(R')₂, -OCOR', -COR', -CO_{z}R', - OCON(R')₂, _{-NR}'S0₂R', -OP(O)(OR')₂, -P(O)(OR')₂, -OP(O)₂0R' , -P(O)₂0R' , - PO(R')₂, -OPO(R')_{2,} or an optionally substituted group selected from C₁-C₆aliphatic, aryl, heteroaryl, cycloaliphatic, heterocycloaliphatic, arylC₁-C₆alkyl, heteroarylC_{]}-C₆alkyl, cycloaliphaticC₁-C₆alkyl, or heterocycloaliphaticC,-C₆a]kyl; and
each occurrence of R' is independently hydrogen or an optionally substituted C¡-6 aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
comprising the steps of:
(a) providing a compound of formula III: 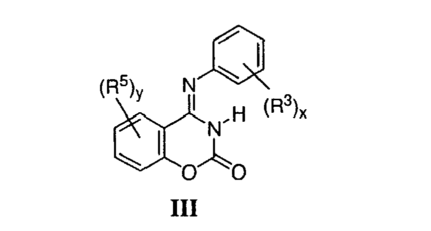 or a suitable salt thereof;
   wherein:
   x is 0-4;
   each R³ is independently halogen, CN, N0₂, -N(R')₂, -CH₂N(R')₂, -OR', -CH₂OR', -SR', -CH_{z}SR', -COOR', -NRCOR', -CON(R')₂, -OCON(R')₂, COR', -NHCOOR', - SO_{z}R', -SO₂N(R')_{2,} or an optionally substituted group selected from Cₗ-C₆aliphatic, aryl, heteroaryl, cycloaliphatic, heterocycloaliphatic, arylC₁-C₆alkyl, heteroarylcₗ-C₆alkyl, cycloaliphaticC₁-C₆alkyl, or heterocycloaliphaticC₁-C₆alkyl;
   y is 0-5;
   each R⁵ is independently halogen, CN, N0₂, -N(R')₂, -CH₂N(R')₂, -OR', -CH₂OR', -SR', -CH₂SR', -NRCOR', -CON(R')₂, -S(O)₂N(R')_{Z}, -OCOR', -COR', -CO₂R', - OCON(R')₂, -NR'S02_{R}', -OP(O)(OR')₂, -P(O)(OR')₂, -OP(0)₂0R', -P(O)₂0R', - PO(R')₂,- -OPO(R')₂, or an optionally substituted group selected from C,_C₆aliphatic, aryl, heteroaryl, cycloaliphatic, heterocycloaliphatic, arylC₁-C₆alkyl, heteroarylc₁-C₆alkyl, cycloaliphaticC₁-C₆alkyl, or heterocycloaliphaticC₁-C₆alkyl; and
   each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
   two occurrences of R' are taken together with the atom(s) to which they are bound
   to form an optionally substituted 3-12 membered saturated, partially unsaturated, or fully unsaturated monocyclic or bicyclic ring having 0-4 heteroatoms independently selected from nitrogen, oxygen, or sulfur;
(b) converting said compound of formula **III** to a compound of formula **II**: 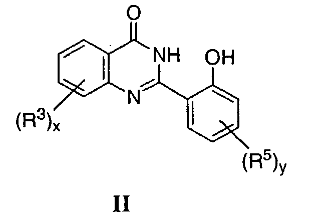 or a suitable salt thereof;
   wherein:
   x is 0-4;
   each R³ is independently halogen, CN, NO₂, -N(R')₂, -CH₂N(R')₂, -OR', -CH₂OR', -SR', -CH₂SR', -COOR', -NRCOR', -CON(R')2_{,} -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC,-C₆alkyl;
   y is 0-5;
   each R⁵ is independently halogen, CN, NO₂, -N(R')2_{,} -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂0R' , -P(O)₂0R', - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
   each occurrence of R' is independently hydrogen or an optionally substituted C₁-₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or: two occurrences of R' are taken together with the atom(s) to which they are bound
   to form an optionally substituted 3-12 membered saturated, partially unsaturated, or fully unsaturated monocyclic or bicyclic ring having 0-4 heteroatoms independently selected from nitrogen, oxygen, or sulfur;
   and
(c) converting said compound of formula **II** to a compound of formula **Ia**.

In certain embodiments, the conversion of a compound of formula **III** to a compound of formula **II**, at step (b), is affected by heating. In other embodiments, step (b) is performed at 150-275 °C. In still other embodiments, step (b) is performed at 200-250 °C in an aprotic solvent. According to another embodiment, step (b) is performed in phenylether.

According to another embodiment, the present invention provides a compound of formula **II**: 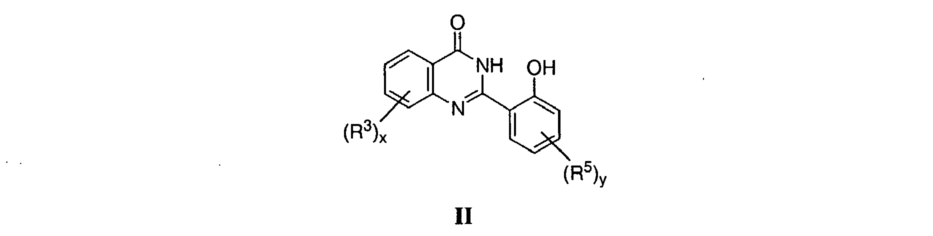 or a suitable salt thereof; wherein:
x is 0-4;
each R³ is independently halogen, CN, NO₂, -N(R')2_{,} -CH₂N(R')2_{,} -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆atkyt, cycloaliphaticC₁₋t-C₆alkyl, or heterocycloaliphaticC₁-C₆alkyl;
y is 0-5;
each R⁵ is independently halogen, CN, NO₂, -N(R')₂, -CH₂N(R')_{2,} -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')_{2,} -P(O)(OR')₂, -OP(O)₂OR', -P(O)₂OR' , -PO(R')_{2,} -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cyaoaliphaticc₁-C₆alkyl, or heterocycloaliphaticC₁-C₆alkyl; and
each occurrence of R' is independently hydrogen or an optionally substituted C₁-₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to
form an optionally substituted 3-12 membered saturated, partially unsaturated, or fully unsaturated monocyclic or bicyclic ring having 0-4 heteroatoms independently selected from nitrogen, oxygen, or sulfur.

In certain other embodiments, the present invention provides a compound of formula **II**: 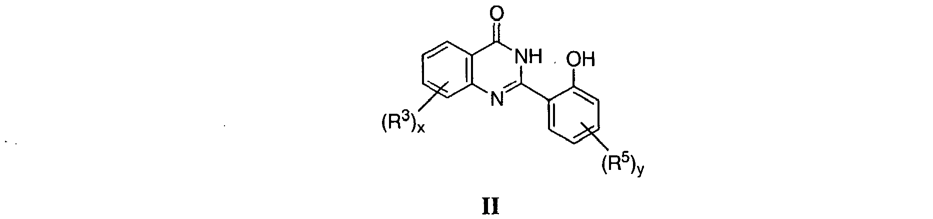 or a suitable salt thereof; wherein:
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C1-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')_{2,} -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁₋₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
provided that the following compounds are excluded:
Glycine, N-[2-[2-[6-[bis(carboxymethyl)amino]-2,3-difluorophenoxy]ethoxy]-4-(3,4-dihydro-4-oxo-2-quinazolinyl)-5-hydroxyphenyl]-N-(carboxymethyl)-, tetrapotassium salt;
Glycine, N-[2-[2-[2-[bis(carboxymethyl)amino]-5-(1,4-dihydro-4-oxo-2-quinazolinyl)-4-hydroxyphenoxy]ethoxy]-4-fluorophenyl]-N-(carboxymethyl)-, tetrapotassium salt;
Glycine, N-[2-[2-[2-[bis(2-methoxy-2-oxoethyl)amino]-5-(1,4-dihydro-4-oxo-2-quinazolinyl)-4-hydroxyphenoxy]ethoxy]-4-fluorophenyl]-N-(2-methoxy-2-oxoethyl)-, methyl ester;
Glycine, N-[2-[2-[6-[bis(2-methoxy-2-oxoethyl)amino]-2,3-difluorophenoxy]ethoxy]-4-(3,4-dihydro-4-oxo-2-quinazolinyl)-5-hydroxyphenyl]-N-(2-methoxy-2-oxoethyl)-, methyl ester;
Glycine, N-[2-[2-[2-[bis(carboxymethyl)amino]-5-(1,4-dihydro-4-oxo-2-quinazolinyl)-4-hydroxyphenoxy]ethoxy]-4-methylphenyl]-N-(carboxymethyl)-;
Glycine, N-[2-[2-[2-[bis(carboxymethyl)amino]-5-(1,4-dihydro-4-oxo-2-quinazolinyl)-4-hydroxyphenoxy]ethoxy]-4-fluorophenyl]-N-(carboxymethyl)-; 4(1H)-Quinazolinone, 6-amino-2-(2-hydroxyphenyl)-;
4(1H)-Quinazolinone, 2-(2-hydroxyphenyl)-6-nitro-;
4(1H)-Quinazolinone, 2-(2-hydroxyphenyl)-;
4(1H)-Quinazolinone, 6-chloro-2-(5-chloro-2-hydroxyphenyl)-:
4(1H)-Quinazolinone, 6-butyl-2-(5-butyl-2-hydroxyphenyl)-;
4(1H)-Quinazolinone, 6-bromo-2-(5-bromo-2-hydroxyphenyl)-;
4(1H)-Quinazolinone, 2-(2-hydroxy-5-pentylphenyl)-6-pentyl-;
4(1H)-Quinazolinone, 2-(2-hydroxyphenyl)-;
4(1H)-Quinazolinone, 6-chloro-2-(2-hydroxyphenyl)- ;
4(1H)-Quinazolinone, 2-(2-hydroxyphenyl)-6-methyl-;
4(1H)-Quinazolinone, 6-chloro-2-(5-chloro-2-hydroxyphenyl)-;
4(1H)-Quinazolinone, 2-(2-hydroxyphenyl)-6-iodo-;
4(1H)-Quinazolinone, 2-(5-chloro-2-hydroxyphenyl)-;
4(1H)-Quinazolinone, 2-(2-hydroxy-4-methoxyphenyl)-;
4(1H)-Quinazolinone, 6-chloro-2-(2-hydroxyphenyl)-;
4(1H)-Quinazolinone, 2-(2-hydroxyphenyl)-6-nitro-;
4(1H)-Quinazolinone, 6-chloro-2-(5-chloro-2-hydroxyphenyl)-;
4(1H)-Quinazolinone, 6-chloro-2-(3,5-dichloro-2-hydroxyphenyl)-;
4(1H)-Quinazolinone, 2-(2-hydroxy-5-methoxyphenyl)-;
4(1H)-Quinazolinone, 2-(2-hydroxy-5-nitrophenyl)-;
4(1H)-Quinazolinone, 6-chloro-2-(2-hydroxy-5-methoxyphenyl)-;
4(1H)-Quinazolinone, 2-(2-hydroxy-5-nitrophenyl)-6-nitro-;
4(1H)-Quinazolinone, 6-chloro-2-(2-hydroxy-5-nitrophenyl)-;
4(1H)-Quinazolinone, 2-(3-fluoro-2-hydroxyphenyl)-;
4(1H)-Quinazolinone, 6-chloro-2-(3-fluoro-2-hydroxyphenyl)-;
4(1H)-Quinazolinone, 2-[5-(1,1-dimethylethyl)-2-hydroxyphenyl]-;
4(1H)-Quinazolinone, 2-(4-hydroxy[1,1'-biphenyl]-3-yl)-;
4(1H)-Quinazolinone, 2-(4-chloro-2-hydroxyphenyl)-;
4(1H)-Quinazolinone, 2-(2-hydroxy-3-methylphenyl)-;
4(1H)-Quinazolinone, 2-(3,5-dibromo-2-hydroxyphenyl)-;
4(1H)-Quinazolinone, 8-bromo-2-(3,5-dibromo-2-hydroxyphenyl)-;
4(1H)-Quinazolinone, 6,8-dibromo-2-(3,5-dibromo-2-hydroxyphenyl)-;
4(1H)-Quinazolinone, 2-(3,5-dichloro-2-hydroxyphenyl)-;
4(3H)-Quinazolinone, 2-(4-ethoxy-2-hydroxyphenyl)-;
4(3H)-Quinazolinone, 6-chloro-2-(2-hydroxy-m-tolyl)-;
4(3H)-Quinazolinone, 2-(2-hydroxy-m-tolyl)-6-nitro-;
4(3H)-Quinazolinone, 2-(3,5-dichloro-2-hydroxyphenyl)-6-nitro-;
4(3H)-Quinazolinone, 2-(3,5-dichloro-2-hydroxyphenyl)-6-nitro-;
4(1H)-Quinazolinone, 2-(5-chloro-2-hydroxyphenyl)-6-nitro-;
4(3H)-Quinazolinone, 2-(2-hydroxy-5-iodophenyl)-;
4(3H)-Quinazolinone, 6-chloro-2-(2-hydroxy-5-iodophenyl)-;
4(3H)-Quinazolinone, 2-(2-hydroxy-5-iodophenyl)-6-nitro-;
4(3H)-Quinazolinone, 2-(5-bromo-2-hydroxyphenyl)-6-chloro-;
4(3H)-Quinazolinone, 2-(5-bromo-2-hydroxyphenyl)-6-nitro-;
4(3H)-Quinazolinone, 6-chloro-2-(4-ethoxy-2-hydroxyphenyl)-;
4(3H)-Quinazolinone, 2-(4-ethoxy-2-hydroxyphenyl)-6-nitro-;
4(3H)-Quinazolinone, 6-chloro-2-(2,4-dihydroxyphenyl)-;
4(3H)-Quinazolinone, 2-(2,4-dihydroxyphenyl)-6-nitro-;
4(3H)-Quinazolinone, 6-chloro-2-(3,5-dibromo-2-hydroxyphenyl)-;
4(3H)-Quinazolinone, 2-(3,5-dibromo-2-hydroxyphenyl)-6-nitro-;
4(3H)-Quinazolinone, 2-(2-hydroxy-3-biphenylyl)-;
4(3H)-Quinazolinone, 2-(2,5-dihydroxyphenyl)-;
4(3H)-Quinazolinone, 6-chloro-2-(2,5-dihydroxyphenyl)-;
4(3H)-Quinazolinone, 2-(2,5-dihydroxyphenyl)-6-nitro-;
[2-{2-[2-(Carboxymethyl-amino)-5-methyl-phenoxy]-ethoxy}-5-hydroxy-4-(4-hydroxy-quinazolin-2-yl)-phenylamino]-acetic acid; and
[2-{(2-[6-(Carboxymethyl-amino)-2,3-difluoro-phenoxy]-ethoxy)}-5-hydroxy-4-(4-hydroxy-quinazolin-2-yl)-phenylamino]-acetic acid.

In certain embodiments, the present invention provides a compound of formula II: 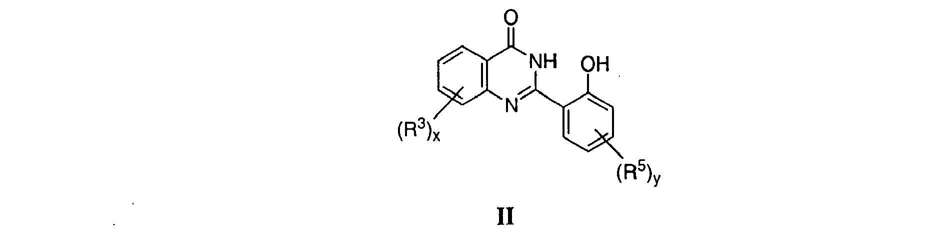 or a suitable salt thereof;
wherein:
x is 1 or 2;
each R³ is independently Cl, Br, F, CF₃, -OCF₃, Me, Et, CN, -COOH, -NH₂, -N(CH₃)₂, -
N(Et)₂, -N(iPr)₂, -O(CH₂)₂OCH₃, -CONH₂, -COOCH₃, -OH, -OCH₃, -OCH₂CH₃, - CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy;
y is 0-4; and
each R⁵ is independently Cl, Br, F, CF₃, Me, Et, CN, -COOH, -NH₂, -N(CH₃)₂, -N(Et)₂, - N(iPr)₂, -O(CH₂)₂OCH₃, -CONH₂, -COOCH₃, -OH, -OCH₃, -OCH₂CH₃, -CH₂OH, - NHCOCH₃, -SO₂NH₂, -SO₂NHC(CH₃)₂, -OCOC(CH₃)₃, -OCOCH₂C(CH₃)₃, - O(CH₂)₂N(CH₃)₂, 4-CH₃-piperazin-l-yl, OCOCH(CH₃)₂, OCO(cyclopentyl), -COCH₃, optionally substituted phenoxy, or optionally substituted benzyloxy.

In other embodiments, the present invention provides a compound of formula II: 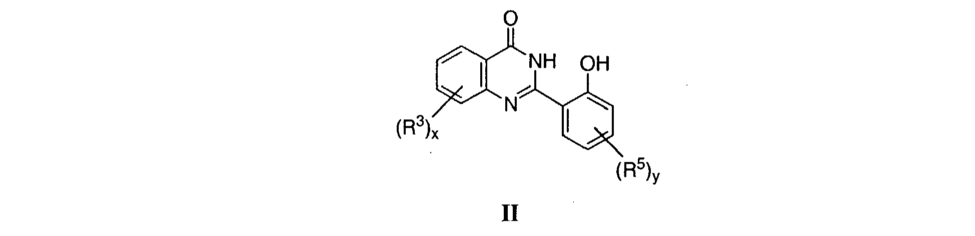 or a suitable salt thereof; wherein:
x is 1;
each R³ is independently Cl, Br, F, CF₃, -OCF₃, Me, Et, CN, -COOH, -OH, or -OCH₃; yis 0 or 1; and
each R⁵ is independently Cl, Br, F, CF₃, Me, -OH, -OCH₃, -OCH₂CH₃, -CH₂OH, - NHCOCH₃, -SO₂NH₂, -SO₂NHC(CH₃)₂.

According to another embodiment, the present invention provides compound II-1 or a suitable salt thereof:

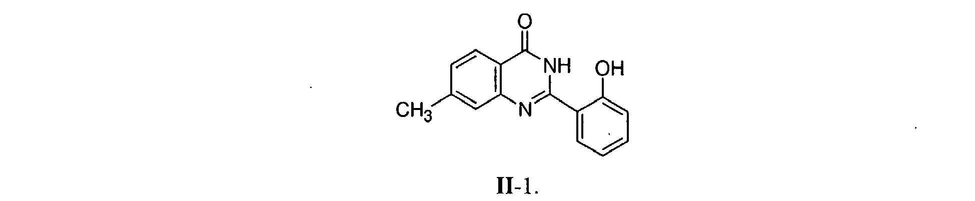

According to another embodiment, the present invention provides a compound of formula IIa: 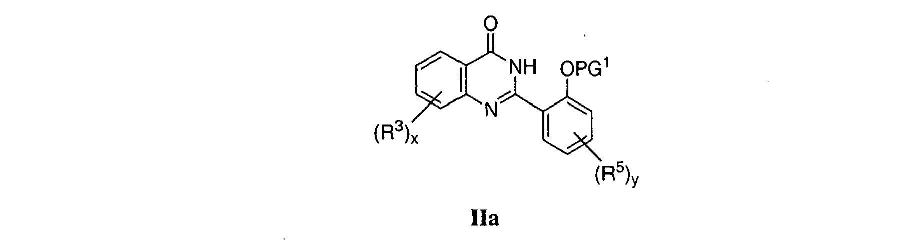 or a suitable salt thereof; wherein:
PG¹ is a suitable hydroxyl protecting group;
x is 0-4;
each R³ is independently halogen, CN, NO₂, -N(R')₂, -CH₂N(R')₂, -OR', -CH₂OR', -SR', -CH₂SR', -COOR', -NRCOR'., -CON(R')₂, -OCON(R')₂, COR', -NHCOOR', -SO₂R', -SO₂N(R')_{2,} or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC,-C₆alkyl; and
each occurrence of R' is independently hydrogen or an optionally substituted C₁-₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to
form an optionally substituted 3-12 membered saturated, partially unsaturated, or fully unsaturated monocyclic or bicyclic ring having 0-4 heteroatoms independently selected from nitrogen, oxygen, or sulfur.

In certain other embodiments, the present invention provides a compound of formula IIa: 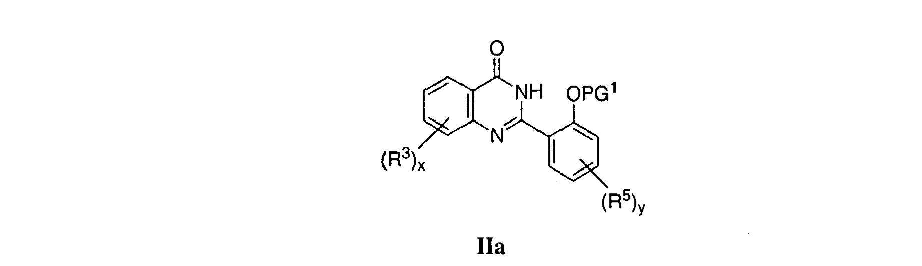 or a suitable salt thereof;
wherein:
PG¹ is a suitable hydroxyl protecting group;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂0R', -P(O)₂OR', -PO(R')₂, -OPO(R')₂, or an optionally substituted group selected from C1-C₆aliphatic, aryl, heteroaryl, cycloaliphatic, heterocycloaliphatic, arylC₁-C₆alkyl, heteroarylC₁-C₆alkyl, cycloaliphaticC₁,-C₆alkyl, or heterocycloaliphaticC₁-C₆alkyl; and
each occurrence of R' is independently hydrogen or an optionally substituted C₁₋₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur;
or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
provided that the following compounds are excluded:
4(1H)-Quinazolinone, 6-chloro-2-[5-chloro-2-(2,2-dimethoxyethoxy)phenyl]-;
4(1H)-Quinazolinone, 2-(2-methoxyphenyl)-;
4(1H)-Quinazolinone, 2-(2,4-dimethoxyphenyl)-;
4(1H)-Quinazolinone, 2-(2,3-dimethoxyphenyl)-;
4(1H)-Quinazolinone, 2-(2,5-dimethoxyphenyl)-;
Benzoic acid, 4-[(aminoiminomethyl)amino]-, 2-(1,4-dihydro-4-oxo-2-quinazolinyl)phenyl ester, monohydrochloride;
4(1H)-Quinazolinone, 2-[2-(acetyloxy)phenyl]-;
4(1H)-Quinazolinone, 2-(2-methoxyphenyl)-;
4(1H)-Quinazolinone, 2-(2-methoxyphenyl)-7-(trifluoromethyl)-;
4(1H)-Quinazolinone, 2-(2-methoxyphenyl)-7-methyl-;
4(1H)-Quinazolinone, 2-[2-(acetyloxy)-5-chlorophenyl]-6-chloro-;
6-Quinazolinecarboxylic acid, 2-(2,3-dimethoxyphenyl)-1,4-dihydro-4-oxo-;
6-Quinazolinecarboxylic acid, 2-(5-ethoxy-2-methoxyphenyl)-1,4-dihydro-4-;
6-Quinazolinecarboxylic acid, 1,4-dihydro-2-[2-methoxy-5-(2-propenyloxy)phenyl]-4-oxo-;
6-Quinazolinecarboxylic acid, 1,4-dihydro-2-(2-methoxy-3-methylphenyl)-4-oxo-;
6-Quinazolinecarboxylic acid, 1,4-dihydro-2-[2-methoxy-5-(1-methylethoxy)phenyl]-4-oxo-;
6-Quinazolinecarboxylic acid, 1,4-dihydro-2-(2-methoxy-5-propoxyphenyl)-4-oxo-;
6-Quinazolinecarboxylic acid, 2-[5-(2-ethoxyethoxy)-2-methoxyphenyl]-1,4-dihydro-4-oxo-;
6-Quinazolinecarboxylic acid, 2-(3-ethoxy-2-methoxyphenyl)-1,4-dihydro-4-oxo-;
6-Quinazolinecarboxylic acid, 1,4-dihydro-2-(2-methoxyphenyl)-4-oxo-;
6-Quinazolinecarboxylic acid, 1,4-dihydro-4-oxo-2-[2-(2-propenyloxy)phenyl]-;
6-Quinazolinecarboxylic acid, 2-[2-(2-ethoxyethoxy)phenyl]-1,4-dihydro-4-oxo-;
6-Quinazolinecarboxylic acid, 2-(2,3-dimethoxyphenyl)-1,4-dihydro-4-oxo-;
6-Quinazolinecarboxylic acid, 1,4-dihydro-2-(2-methoxy-3-methylphenyl)-4-oxo-;
6-Quinazolinecarboxylic acid, 1,4-dihydro-2-(2-methoxy-5-methylphenyl)-4-oxo-;
6-Quinazolinecarboxylic acid, 2-[2-(2-ethoxyethoxy)-3-methoxyphenyl]-1,4-dihydro-4-oxo-;
6-Quinazolinecarboxylic acid, 1,4-dihydro-2-(2-methoxyphenyl)-4-oxo-, methyl ester;
4(1H)-Quinazolinone, 6,7,8-trimethoxy-2-(2,3,4-trimethoxyphenyl)-;
Carbonic acid, ethyl ester, ester with 2-(o-hydroxyphenyl)-4(3H)-Quinazolinone;
4(3H)-Quinazolinone, 6-butyl-2-(o-methoxyphenyl)-;
4(1H)-Quinazolinone, 2-(3,5-dibromo-2-methoxyphenyl)-; and
2-(2'-acetoxyphenyl)-4(3H)-quinazolinone.

In certain embodiments, the x moiety of formula **IIa** is 1 or 2, and each R³ is independently Cl, Br, F, CF₃, -OCF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy.

In other embodiments, the x moiety of formula **IIa** is 1, and R³ is Cl, Br, F, CF₃, -OCF₃, Me, Et, CN, -COOH, -OH, or -OCH₃.

In still other embodiments, the y moiety of formula **IIa** is 0-4, and each R⁵ is independently Cl, Br, F, CF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -SO₂NH₂, -sO₂NHC(CH₃)₂, -OCOC(CH₃)₃, -OCOCH₂C(CH₃)₃, -O(CH₂)₂N(CH₃)₂,4-CH₃-piperazin-1-yl, OCOCH(CH₃)₂, OCO(cyclopentyl), -COCH₃, optionally substituted phenoxy, or optionally substituted benzyloxy.

According to another embodiment, the y moiety of formula **IIa** is 0 or 1, and R⁵ is Cl, Br, F, CF₃, Me, -OH, -OCH₃, -OCH₂CH₃, -CH₂OH, -NHCOCH₃, -SO₂NH₂, - SO₂NHC(CH₃)₂.

In other embodiments, the PG¹ group of formula **IIa** is an ester group.

According to another embodiment, the present invention provides compound IIa-1 or a suitable salt thereof:

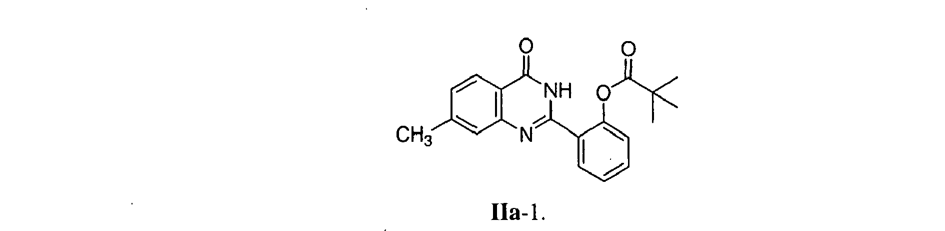

In certain embodiments, the present invention provides a compound of formula IIb: 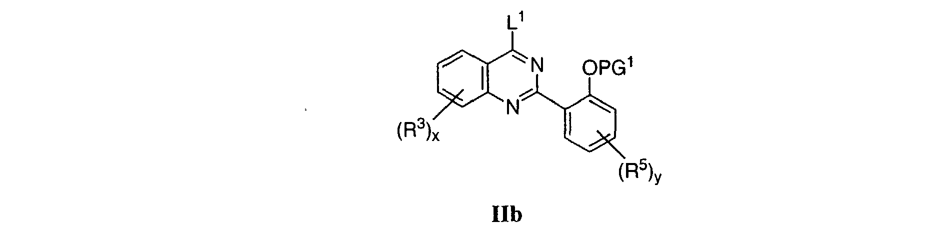 or a suitable salt thereof; wherein:
PG¹ is a suitable hydroxyl protecting group;
L¹ is a suitable leaving group;
x is 0-4;
each R³ is independently halogen, CN, NO₂, -N(R')₂, -CH₂N(R')₂, -OR', -CH_{Z}OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁₋C₆aliphatic, aryl, heteroaryl, cycloaliphatic, heterocycloaliphatic, arylC₁-C₆alkyl, heteroarylc₁-C₆alkyl, cyaoaliphaticC₁-C₆alkyl, or heterocycloaliphaticC₁-C₆alkyl;
y is 0-5;
each R⁵ is independently halogen, CN, NO₂, -N(R')2_{,} -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO₂R', -OP(O)(OR')₂, -P(O)(OR')₂, -OP(O)₂OR' , -P(O)₂OR' , - PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁-₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur.

In certain embodiments, the x moiety of formula **IIb** is 1 or 2, and each R³ is independently Cl, Br, F, CF₃, -OCF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy.

In other embodiments, the x moiety of formula **IIb** is 1, and R³ is CI, Br, F, CF₃, -OCF₃, Me, Et, CN, -COOH, -OH, or -OCH₃.

In still other embodiments, the y moiety of formula **IIb** is 0-4, and each R⁵ is independently Cl, Br, F, CF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -SO₂NH₂, -SO₂NHC(CH₃)₂, -OCOC(CH₃)₃, -OCOCH₂C(CH₃)₃, -O(CH₂)₂N(CH₃)₂,4-CH₃-piperazin-1-yl, OCOCH(CH₃)₂, OCO(cyclopentyl), -COCH₃, optionally substituted phenoxy, or optionally substituted benzyloxy.

According to another embodiment, the y moiety of formula IIb is 0 or 1, and R⁵ is Cl, Br, F, CF₃, Me, -OH, -OCH₃, -OCH₂CH₃, -CH₂OH, -NHCOCH₃, -SO₂NH₂, SO₂NHC(CH₃)₂

In other embodiments, the PG¹ group of formula **IIb** is an ester group.

According to another embodiment, the present invention provides compound IIb-1 or a suitable salt thereof:

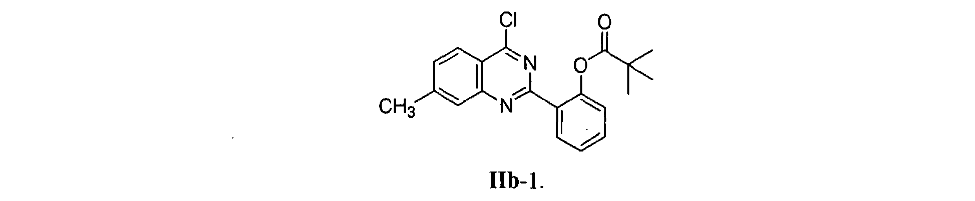

According to another aspect, the present invention provides a compound of formula IIc: 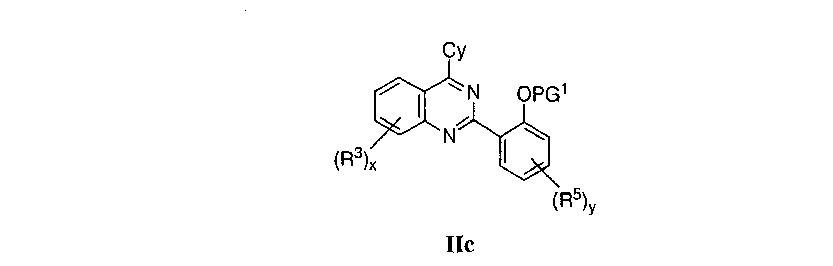 or a suitable salt thereof; wherein:
PG¹ is a suitable hydroxyl protecting group;
Cy is a ring selected from: 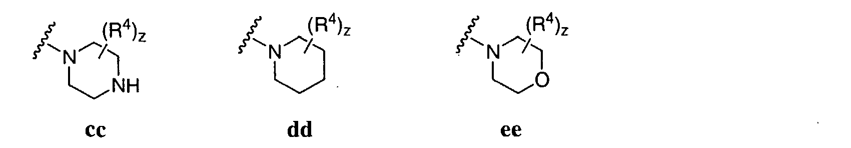 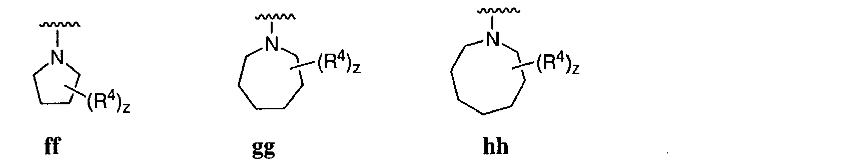 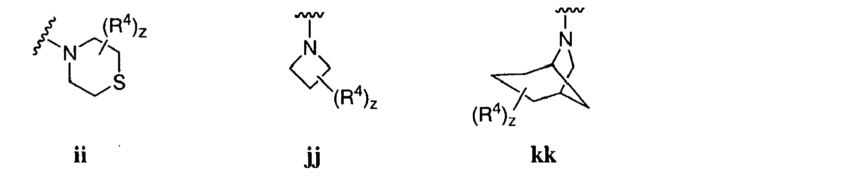 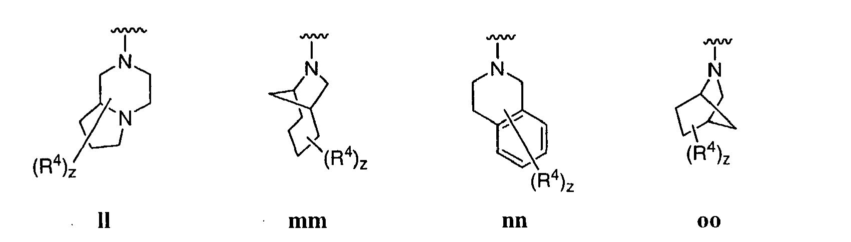 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆a]kyl, or heterocycloaliphaticC₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')2_{,} -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(0)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC1-C₆alkyl; and
each occurrence of R' is independently hydrogen or an optionally substituted C₁-₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to
form an optionally substituted 3-12 membered saturated, partially unsaturated, or fully unsaturated monocyclic or bicyclic ring having 0-4 heteroatoms independently selected from nitrogen, oxygen, or sulfur.

In certain other embodiments, the present invention provides a compound of formula IIc: 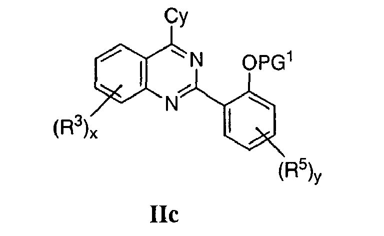 or a suitable salt thereof;
wherein:
PG¹ is a suitable hydroxyl protecting group;
Cy is a ring selected from: 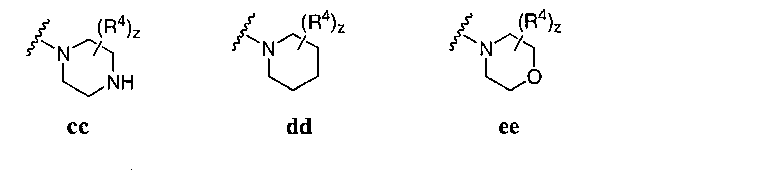 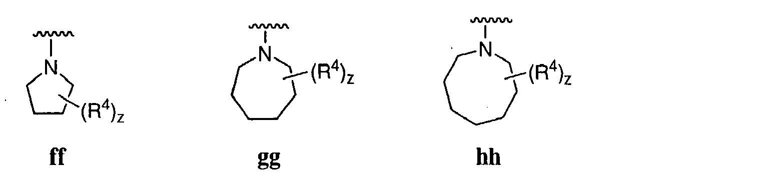 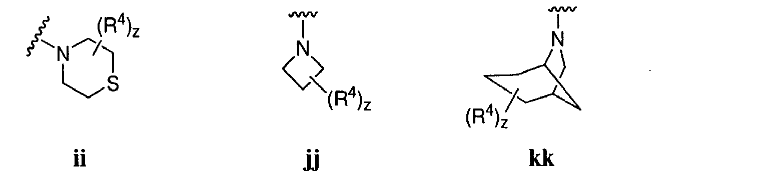 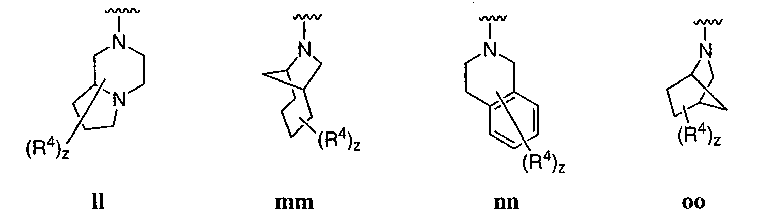 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁₋C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')2_{,} -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted C₁-₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an
optionally substituted 3-12 membered saturated, partially unsaturated, or fully unsaturated monocyclic or bicyclic ring having 0-4 heteroatoms independently selected from nitrogen, oxygen, or sulfur;
provided that the following compounds are excluded:
Quinazoline, 2-(2-methoxyphenyl)-4-(1-pyrrolidinyl)-;
Quinazoline, 6-bromo-2-(2-methoxyphenyl)-4-(4-morpholinyl)-;
Quinazoline, 6,8-dichloro-2-(2-methoxyphenyl)-4-(4-morpholinyl)-;
Quinazoline, 6-bromo-2-(2-methoxyphenyl)-4-(1-pyrrolidinyl)-;
Quinazoline, 6,8-dichloro-2-(2-methoxyphenyl)-4-(1-pyrrolidinyl)-;
Quinazoline, 2-(2-fluoro-6-methoxyphenyl)-6-methoxy-4-(4-morpholinyl)-;
Quinazoline, 2-(2-fluoro-6-methoxyphenyl)-4-(4-methyl-1-piperidinyl)-7-(trifluoromethyl)-;
Cyclopropanecarboxylic acid, 3-fluoro-2-[7-methyl-4-(4-methyl-1-piperidinyl)-2-quinazolinyl]phenyl ester;
Propanoic acid, 2-methyl-, 3-fluoro-2-[7-methyl-4-(4-methyl-1-piperidinyl)-2-quinazolinyl]phenyl ester;
Butanoic acid, 3-methyl-, 3-fluoro-2-[7-methyl-4-(4-methyl-1-piperidinyl)-2-quinazolinyl]phenyl ester;
Cyclopentanecarboxylic acid, 3-fluoro-2-[7-methyl-4-(4-methyl-1-piperidinyl)-2-quinazolinyl]phenyl ester;
Propanoic acid, 2,2-dimethyl-, 3-fluoro-2-[7-methyl-4-(4-methyl-1-piperidinyl)-2-quinazolinyl]phenyl ester;
Butanoic acid, 3,3-dimethyl-, 3-fluoro-2-[7-methyl-4-(4-methyl-l-piperidinyl)-2-quinazolinyl]phenyl ester;
Quinazoline, 7-chloro-2-(2-methoxyphenyl)-4-[3-(trifluoromethyl)-1-pyrrolidinyl];
Piperazine, 1-(butylsulfonyl)-4-[2-(2,4-dimethoxyphenyl)-7-methyl-4-quinazolinyl]-;
Phenol, 3-fluoro-2-[7-methyl-4-(4-methyl-1-piperidinyl)-2-quinazolinyl]-, acetate (ester);
Piperazine, 1-(butylsulfonyl)-4-[2-(2-fluoro-6-methoxyphenyl)-7-(trifluoromethyl)-4-quinazolinyl]-;
1-Piperazinecarboxylic acid, 4-[6-bromo-2-(2-methoxyphenyl)-4-quinazolinyl]-, 1,1-dimethylethyl ester;
Carbamic acid, (2-methylpropyl)-, 1-[2-(2-methoxyphenyl)-7-methyl-4-quinazolinyl]-4-piperidinyl ester;
6-Quinazolinecarboxylic acid, 4-[4-[(1,1-dimethylethoxy)carbonyll-l-piperazinyl]-2-(2-methoxyphenyl)-; and
Benzenesulfonamide, 2-methoxy-5-[2-[4-[2-(2-methoxyphenyl)-4-quinazolinyl]-1-piperazinyllethyl]-, (2Z)-2-butenedioate (2:3).

In certain embodiments, the present invention provides a compound of formula IIc: 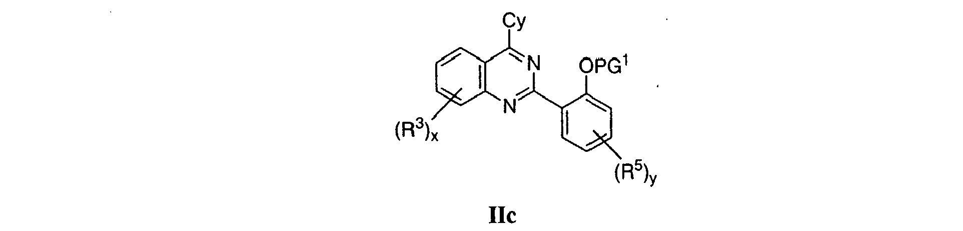 or a suitable salt thereof; wherein:
PG¹ is a suitable hydroxyl protecting group;
Cy is azetidin-1-yl (jj), pyrrolidin-1-yl (ff), piperidinl-yl (dd), or piperazin-1-yl (cc)
optionally substituted with 0-4 occurrences of R⁴;
each R⁴ is independently Cl, Br, F, CF₃, CH₃, -CH₂CH₃, CN, -COOH, -N(CH₃)₂, -N(Et)₂, -N(iPr)₂, -O(CH₂)_{Z}OCH₃, -CONH₂, -COOCH₃, -OH, -CH₂OH, -NHCOCH₃, -SO₂NH₂, -SO₂(CH₂)₃CH₃, -SO₂CH(CH₃)₂, -SO₂N(CH₃)₂, -SO₂CH₂CH₃, -C(O)OCH₂CH(CH₃)₂, -C(O)NHCH₂CH(CH₃)₂, -C(O)CH(OH)CH₂CH(CH₃)₂, -C(O)CH(OH)CH₂C(CH₃)₃, -NHCOOCH₃, -C(O)C(CH₃)₃, -COO(CH₂)₂CH₃, -C(O)NHCH(CH₃)₂, -C(O)CH₂CH₃, or an optionally substituted group selected from -piperidinyl, piperizinyl, morpholino, C₁-₄alkoxy, phenyl, phenyloxy, benzyl, benzyloxy, -CH₂cyclohexyl, pyridyl, -CH₂pyridyl, or -CH₂thiazolyl;
x is 1 or 2;
each R³ is independently Cl, Br, F, CF₃, -OCF₃, Me, Et, CN, -COOH, -NH₂, -N(CH₃)₂, -
N(Et)₂, -N(iPr)₂, -O(CH₂)₂OCH₃, -CONH₂, -COOCH₃, -OH, -OCH₃, -OCH₂CH₃, - CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy;
y is 0-4; and
each R⁵ is independently Cl, Br, F, CF₃, Me, Et, CN, -COOH, -NH₂, -N(CH₃)₂, -N(Et)₂, - N(iPr)₂, -O(CH₂)₂OCH₃, -CONH₂, -COOCH₃, -OH, -OCH₃, -OCH₂CH₃, -CH₂OH, - NHCOCH₃, -SO₂NH₂, -SO₂NHC(CH₃)₂, -OCOC(CH₃)₃, -OCOCH₂C(CH₃)₃, - O(CH₂)₂N(CH₃)₂, 4-CH₃-piperazin-1-yl, OCOCH(CH₃)₂, OCO(cyclopentyl), -COCH₃, optionally substituted phenoxy, or optionally substituted benzyloxy.

In other embodiments, the x moiety of formula **IIc** is 1 and R³ is at the 7-position of the quinazoline ring and is -Cl, -CH₃, -CH₂CH₃, -F, -CF₃, -OCF₃, -CONHCH₃, - CONHCH₂CH₃, -CONH(cyclopropyl), -OCH₃, -NH₂, -OCH₂CH₃, or -CN. In yet other embodiments, the x moiety of formula **IIc** is 1 and R³ is at the 7-position of the quinazoline ring and is -Cl, -CH₃, -CH₂CH₃, -F, -CF₃, -OCF₃, -OCH₃, or -OCH₂CH₃- In certain other embodiments, the x moiety of formula **IIc** is 1 and R³ is at the 7-position of the quinazoline ring and is methyl.

According to another embodiment, the present invention provides compound IIc-1 or a suitable salt thereof:

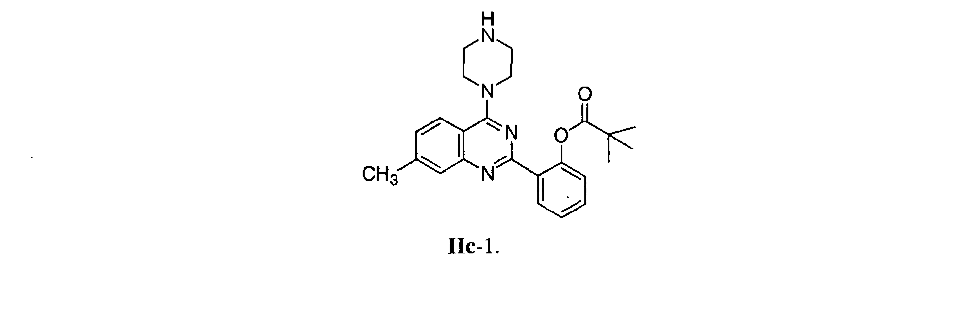

According to yet another embodiment, the present invention provides a compound of formula III: 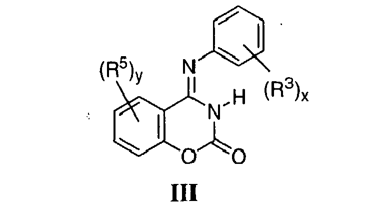 or a suitable salt thereof; wherein:
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cyc1oaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁C₆alkyl, heteroarylC₁₋C₆alkyl, cycloaliphaticC,-C₆alkyl, or heterocycloaliphaticC₁-C₆alkyl; and
each occurrence of R' is independently hydrogen or an optionally substituted C₁-₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to
form an optionally substituted 3-12 membered saturated, partially unsaturated, or fully unsaturated monocyclic or bicyclic ring having 0-4 heteroatoms independently selected from nitrogen, oxygen, or sulfur;
provided that:
(i) x and y are not simultaneously zero; and
(ii) when y is zero, and x is one, then R³ is not:
   chloro in the para-position; or
   methyl in the para-position.

In certain embodiments, the x moiety of formula III is 1 or 2, and each R³ is independently Cl, Br, F, CF₃, -OCF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -NHCOCH(CH₃)₂, -S0O₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy.

In other embodiments, the x moiety of formula III is 1, and R³ is Cl, Br, F, CF₃, -OCF₃, Me, Et, CN, -COOH, -OH, or -OCH₃.

In still other embodiments, the y moiety of formula III is 0-4, and each R⁵ is independently Cl, Br, F, CF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -SO₂NH₂, -SO₂NHC(CH₃)₂, -OCOC(CH₃)₃, -OCOCH₂C(CH₃)₃, -O(CH₂)₂N(CH₃)₂, 4-CH₃-piperazin-1-yl, OCOCH(CH₃)₂, OCO(cyclopentyl), -COCH₃, optionally substituted phenoxy, or optionally substituted benzyloxy.

According to another embodiment, the y moiety of formula III is 0 or 1, and R⁵ is Cl, Br, F, CF₃, Me, -OH, -OCH₃, -OCH₂CH₃, -CH₂OH, -NHCOCH₃, -SO₂NH₂, - SO₂NHC(CH₃)₂.

According to another embodiment, the present invention provides compound III-1 or a suitable salt thereof:

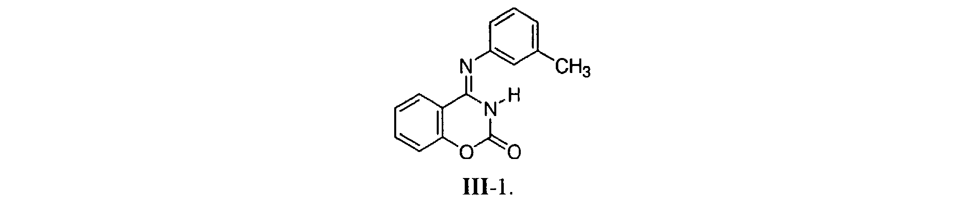

Yet another embodiment of the present invention provides a compound of formula IV: 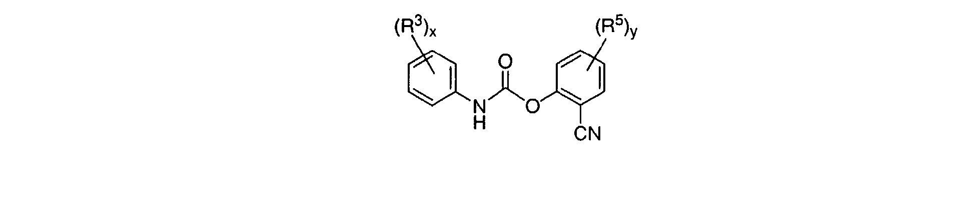 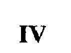 or a suitable salt thereof;
wherein:
x is 0-4;
each R³ is independently halogen, CN, NO₂, -N(R')₂, -CH₂N(R')2_{,} -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')_{2,} -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cyloaliphaticC₁-C₆alkyl, or heterocyloaliphaticC₁-C₆alkyl; and
each occurrence of R' is independently hydrogen or an optionally substituted C₁-₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to
form an optionally substituted 3-12 membered saturated, partially unsaturated, or fully unsaturated monocyclic or bicyclic ring having 0-4 heteroatoms independently selected from nitrogen, oxygen, or sulfur; provided that when x is one and R³ is methyl in the 3-position, then when y is one, R⁵ is not -S-CN in the 4-position.

In certain embodiments, the x moiety of formula IV is 1 or 2, and each R³ is independently Cl, Br, F, CF₃, -OCF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy.

In other embodiments, the x moiety of formula **IV** is 1, and R³ is Cl, Br, F, CF₃, -OCF₃, Me, Et, CN, -COOH, -OH, or -OCH₃.

In still other embodiments, the y moiety of formula **IV** is 0-4, and each R⁵ is independently Cl, Br, F, CF₃, Me, Et, CN, -COOH, -NH₂, -N(CH₃)₂, -N(Et)₂, -N(iPr)₂, -O(CH₂)₂OCH₃, -CONH₂, -COOCH₃, -OH, -OCH₃, -OCH₂CH₃, -CH₂OH, -NHCOCH₃, -SO₂NH₂, -SO₂NHC(CH₃)₂, -OCOC(CH₃)₃, -OCOCH₂C(CH₃)₃, -O(CH₂)₂N(CH₃)₂, 4-CH₃-piperazin-1-yl, OCOCH(CH₃)₂, OCO(cyclopentyl), -COCH₃, optionally substituted phenoxy, or optionally substituted benzyloxy.

According to another embodiment, the y moiety of formula **IV** is 0 or 1, and R⁵ is Cl, Br, F, CF₃, Me, -OH, -OCH₃, -OCH₂CH₃, -CH₂OH, -NHCOCH₃, -SO₂NH₂, - SO₂NHC(CH₃)₂.

According to another embodiment, the present invention provides compound **IV**-1 or a suitable salt thereof:

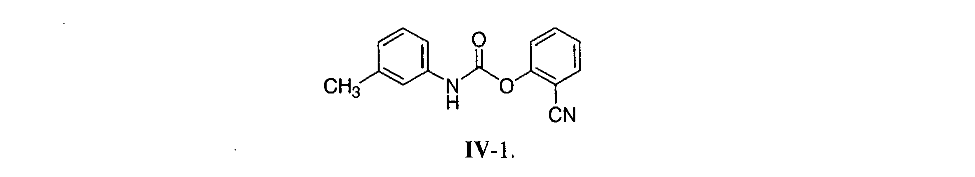

In order that the invention described herein may be more fully understood, the following examples are set forth. It should be understood that these examples are for illustrative purposes only and are not to be construed as limiting this invention in any manner.

### EXAMPLES

### Example 1

**4-m-Tolylimino-3,4-dihydro-benzo[e][1,3]oxazin-2-one (III-1):** 2-Cyanophenol (2.4 g) and m-tolylisocyanate (2.6 g) were combined with toluene (10 mL) and triethylamine (3 drops) and the resulting mixture heated at reflux. After 18 hours, the resulting solid was collected to afford 4.0 g of the title compound.

### Example 2

**2-(2-Hydroxy-phenyl)-7-methyl-3H-quinazolin-4-one (II-1):** 4-m-Tolylimino-3,4-dihydro-benzo[e][1,3]oxazin-2-one (1.014 g) was combined with diphenyl ether. The resulting mixture was heated at 250°C for 1 hour. The reaction was allowed to cool and the resulting solid was collected to afford 0.91 g of the title compound as a tan solid. ¹H-NMR (CDCl₃): 8.25 (1H, d), 8.0 (1H, d), 7.55 (1H, s), 7.45 (1H, t), 7.4 (1H, d), 6.85 (1H, d), 6.8 (1H, t).

### Example 3

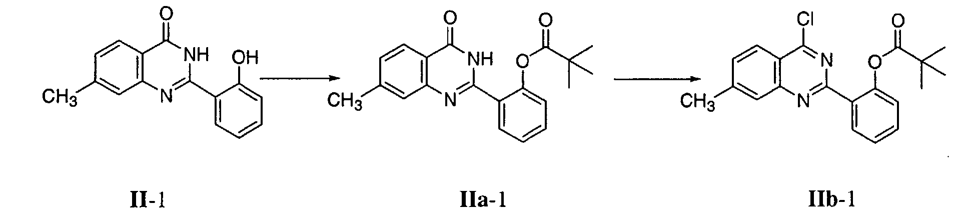

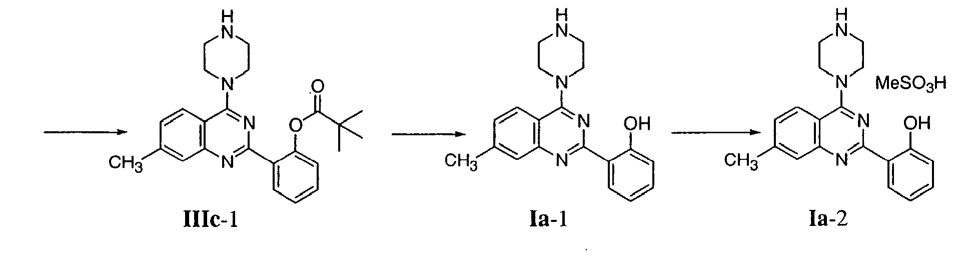

**2,2-Dimethyl-propionic acid 2-(7-methyl-4-oxo-3,4-dihydro-quinazolin-2-yl)-phenyl ester (IIa-1):** 2-(2-Hydroxy-phenyl)-7-methyl-3H-quinazolin-4-one (1.0 g) was suspended in DMF (10 mL). Trimethylacetic anhydride (1.0 mL) and pyridine (0.63 mL) were added and the resulting mixture heated at 50°C for 1 hour. The reaction was poured into water (100 mL) and extracted with methylene chloride (3 x 50 mL). The organic extracts were combined and concentrated *in vacuo* to afford the title compound which was used directly in the next step.

**2,2-Dimethyl-propionic acid 2-(4-chloro-7-methyl-quinazolin-2-yl)-phenyl ester (IIb-1):** The 2,2-dimethyl-propionic acid 2-(7-methyl-4-oxo-3,4-dihydro-quinazolin-2-yl)-phenyl ester prepared above was dissolved in toluene (10 mL) and treated with phosphoryl oxychloride (0.37 mL) and pyridine (0.63 mL). The resulting solution was stirred at 80°C. The reaction was then poured into ice water and extracted with methylene chloride (3 x 50 mL). The combined organic extracts were concentrated *in vacuo* to approximately 20 mL and this concentrate was used directly in the next step.

**2,2-Dimethyl-propionic acid 2-(7-methyl-4-piperazin-1-yl-quinazolin-2-yl)-phenyl ester (IIIc-1):** To the concentrate from above was added piperazine (1.36 g) and triethylamine (3.36 mL). The resulting mixture was allowed to stir. The reaction mixture was washed with water then concentrated *in vacuo* and the resulting solid used directly in the next step.

**2-(7-Methyl-4-piperazin-1-yl-quinazolin-2-yl)-phenol (Ia-2) and mesylate salt (Ia-3):** The solid formed above was dissolved in ethanol (28 mL) and treated with KOH (3.0 g). Upon complete reaction, the mixture was poured into water (100 mL), neutralized with HCl and extracted with methylene chloride (3 x 50 mL). To the resulting solution was added oxalic acid. The resulting solids were collected then freebased and treated with methanesulfonic acid. The resulting solids were recrystallized from ethanol (10 mL) to afford 0.71 g of the mesylate salt as a yellow solid.

### Example 4

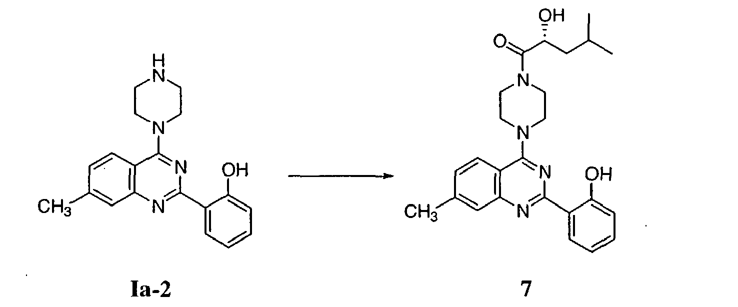

**(R)-2-hydroxy-1-(4-(2-(2-hydroxyphenyl)-7-methylquinazolin-4-yl)piperazin-1-yl)-4-methylpentan-1-one (7):** A mechanically stirred suspension of 2-(7-methyl-4-piperazin-1-yl-quinazolin-2-yl)-phenol (**Ia-2**) in DMF (3.6 liters) and N-methylmorpholine (214 ml, 1.2 equivalents) was treated with 1-(3-dimethylaminopropyl)-3-ethylcarbodiimide HCl (EDC, 200g, 1.2 equivalents) and 1-hydroxybenzotriazole hydrate (HOBt, 160g, 1.2 equivalents). A solution of (R)-isocaproic acid (140g, 1.2 equivalents, prepared by methods know in the art from D-leucine) in dimethylformamide (400 ml) was added to the mixture over a period of 6 hours. The mixture was cooled to 0-5°C and water (8 liters) was added slowly to the mixture. The resulting solid was removed from the reaction mixture both manually and via filtration and the solid was then dissolved in THF (2.0 liters), filtered, and treated with methane sulfonic acid (1.2 equivalents). The resulting solid salt was filtered, then dissolved with heating in anhydrous ethanol (3A grade, 4 liters), cooled to 45°C, held at that temperature for 1 hour and then allowed to cool to room temperature. The resulting mesylate was collected by filtration. The salt was then suspended in methylene chloride (2 liters) and treated with potassium carbonate (145.12g, 2 equivalents) previously dissolved in water (2 liters). The organic layer was collected and evaporated to afford a yellow solid, which after drying under vacuum (125 Torr, room temperature) gave 261.2g of desired product 5 (63% yield) as a yellow solid and as the free base with consistent analytical data. The present invention further comprises the aspects defined in the following clauses (which form part of the present description but are not considered as claims in accordance with decision J 15/88 of Legal Board of Appeal of the European Patent Office):

## Claims

1. **A method for preparing a compound of formula Ia:** 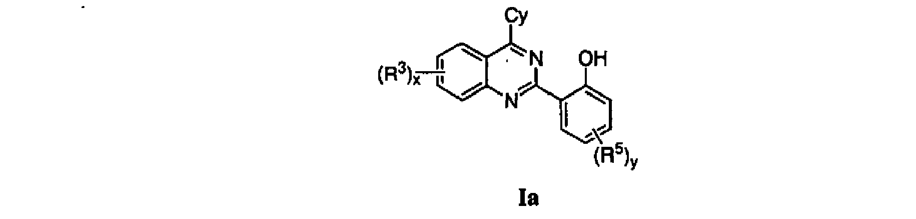 or a suitable salt thereof; wherein:
Cy is a ring selected from: 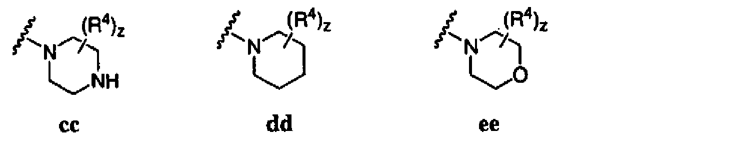 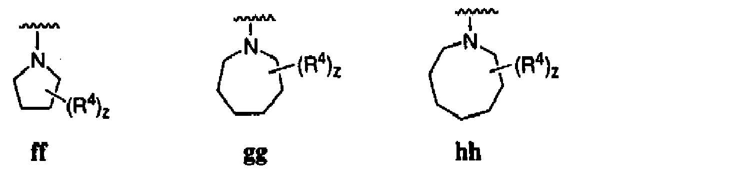 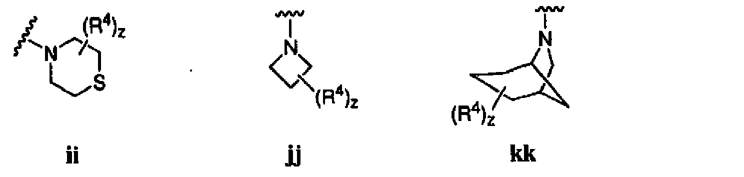 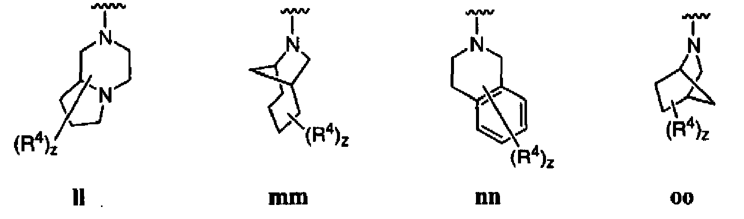 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')2_{,} or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl,
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')_{2,} -OCON(R')₂, COR', -NHCOOR', - SO₂R', -SO₂N(R')2_{,} or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', - SR', -CH₂SR', -NRCOR', -CON(R')₂, -S(O)₂N(R')₂, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁C₆alkyl, cycloaliphaticC1-4alkyl, or heterocycloaliphaticC₁-C₆alkyl; and
each occurrence of R' is independently hydrogen or an optionally substituted C₁-₆
aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
comprising the steps of:
(a) providing a compound of formula II: 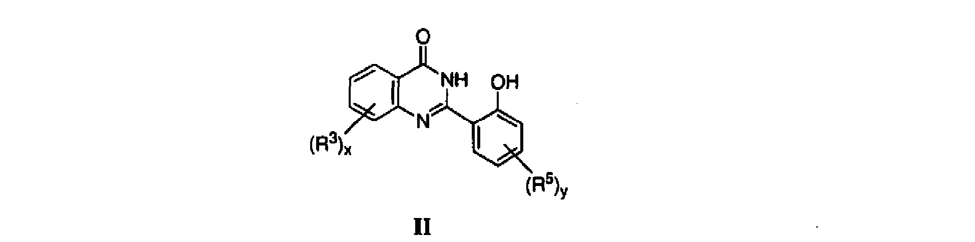 or a suitable salt thereof;
wherein:
x is 0-4;
each R³ is independently halogen, CN, N0₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylCₜ-C₆alkyl, heteroarylCₗ-C₆alkyl, cycloaliphaticC.-C₆alkyl, or beterocycloaliphaticCₜ-C₆alkyl;
y is 0-5;
each R⁵ is independently halogen, CN, N0₂, -N(R')₂, -CH₂N(R')2_{,} -OR', -CH₂OR', -SR', -CH₂SR', -NRCOR', -CON(R')2_{,} -S(O)₂N(R')₂, -OCOR', -COR', -CO₂R', - OCON(R')2_{,} -NR'SO₂R', -OP(O)(OR')₂, -P(O)(OR')₂, -OP(O)₂OR', -P(O)₂0R', - PO(R')₂, -OPO(R')2_{,} or an optionally substituted group selected from C₁-C₆aliphatic, aryl, heteroaryl, cycloaliphatic, heterocycloaliphatic, ary]C₁-C₆a]kyl, heteroarylC₁-C₆alkyl, cyaoaliphaticcᵢ-C₆alkyl, or heterocycloaliphaticC,-C₆alkyl; and
each occurrence of R' is independently hydrogen or an optionally substituted C₁₋₆
aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or: two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
and
(b) converting said compound of formula II or a suitable salt thereof, to a compound of formula Is or a suitable salt thereof.

2. The method according to clause 1, further comprising the steps of:
(a) protecting the hydroxyl group of compound II with a suitable hydroxyl protecting group to form a compound of formula **IIa:** 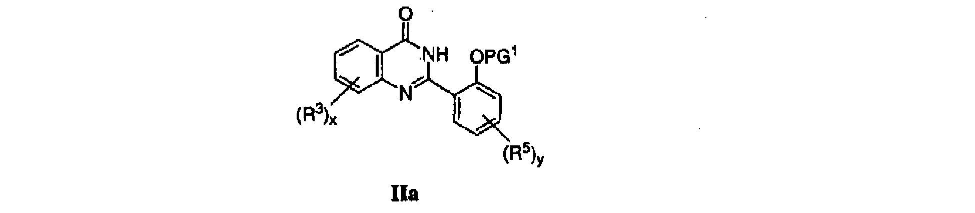 or a suitable salt thereof;
wherein:
PG' is a suitable hydroxyl protecting group;
x is 0-4;
each R³ is independently halogen, CN, N0₂, -N(R')₂, -CH₂N(R')₂, -OR', -CH₂OR', -SR', -CH₂SR', -COOR', -NRCOR', -CON(R')2_{,} -OCON(R')₂, COR', -NHCOOR', -SO₂R', -S0₂N(R')_{z}, or an optionally substituted group selected from C_{I-}Qatiphatic, . aryl, heteroaryl, cycloaliphatic, heterocycloaliphatic, arylC₁-4alkyl, heteroarylcₗ-C₆alkyl, cycloaliphaticC_{I}-4alkyl, or heterocycloaliphaticC.-4alkyl;
y is 0-5;
each R⁵ is independently halogen, CN, N0₂, -N(R')2_{,} -CH₂N(R')₂. -OR', -CH₂OR', -SR', -CH₂SR', -NRCOR', -CON(R')₂, -S(O)₂N(R')₂, -OCOR', -COR', -CO₂R', - OCON(R')₂, -NR'S02_{R}', -OP(O)(OR')₂, -P(O)(OR')₂, -OP(O)2OR', -P(O)₂0R', - PO(R')2, -OPO(R')₂, or an optionally substituted group selected from C₁.C₆aliphatic, aryl, heteroaryl, cycloaliphatic, heterocycloaliphatic, arylC1 -C₆alkyl, heteroarylC₁ C₆alkyl, cycloaliphaticC₁-C₆alkyl, or heterocycloaliphaticCₜ-4alkyl; and
each occurrence of R' is independently hydrogen or an optionally substituted C₁₋₆
aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially
unsaturated, or fully unsaturated monocyclic or bicyclic ring having 0-4
heteroatoms independently selected from nitrogen, oxygen, or sulfur;
(b) converting the ketone moiety of the compound of formula **IIa** or a suitable salt thereof, to a suitable leaving group to form a compound of formula **IIb:** 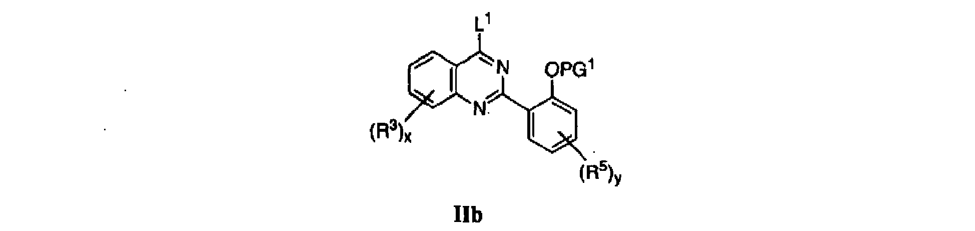 or a suitable salt thereof;
wherein:
PG' is a suitable hydroxyl protecting group;
L^{l} is a suitable leaving group;
x is 0-4;
each R³ is independently halogen, CN, NO₂, -N(R')₂, -CH₂N(R')₂, **-**OR.' **,** -CH₂OR', -SR', -CH₂SR', -COOR', -NRCOR', -CON(R')2_{,} -OCON(R')2_{,} COR', -NHCOOR', -SO₂R', -SO₂N(R')₂, or an optionally substituted group selected from Cₗ₋C₆aliphatic, aryl, heteroaryl, cycloaliphatic, heterocycloaliphatic, arylC₁-C₆alkyl, heteroaryIC₁-C₆alkyl, cycloaliphaticcᵢ-C₆alkyl, or heterocycloaliphaticC₁-C₆alkyl;
y is 0-5;
each R⁵ is independently halogen, CN, N0₂, -N(R.')₂, -CH₂N(R')₂, -OR', -CH₂OR', -SR', -CH₂SR', -NRCOR', -CON(R')₂, -S(OhN(R')₂, -OCOR', -COR', -CO₂R', - OCON(R')₂, -NR'SO₂R', -OP(O)(OR')₂, -P(O)(OR')2_{,} -OP(O)₂0R', -P(O)₂OR', - PO(R')₂, -OPO(R')2, or an optionally substituted group selected from C₁.C₆aliphatic, aryl, heteroaryl, cycloaliphatic, heterocycloaliphatic, atylC₁-C₆alkyl, heteroarylc₁-C₆alkyl, cycloaliphaticC₁-C₆allcyl, or heterocycloaliphaticC₁-C₆a]kyl; and
each occurrence of R' is independently hydrogen or an optionally substituted C₁-₆
aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
(c) displacing said suitable leaving group with a suitable Cy moiety to form a compound of formula **IIc:** 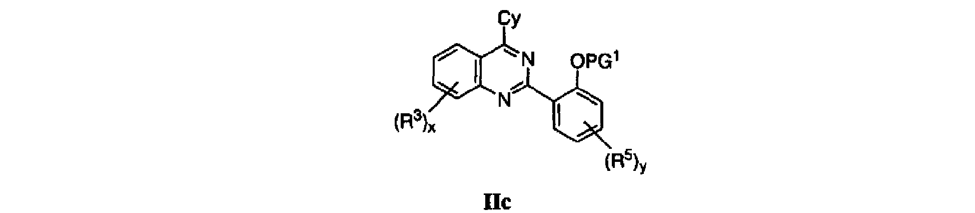 or a suitable salt thereof;
wherein:
PG' is a suitable hydroxyl protecting group;
Cy is a ring selected from: 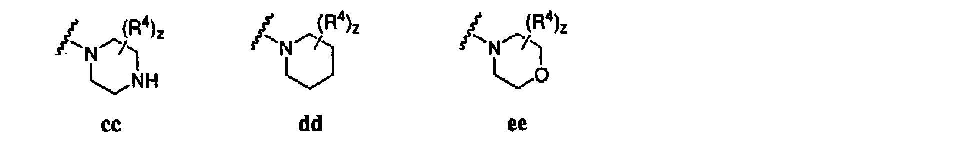 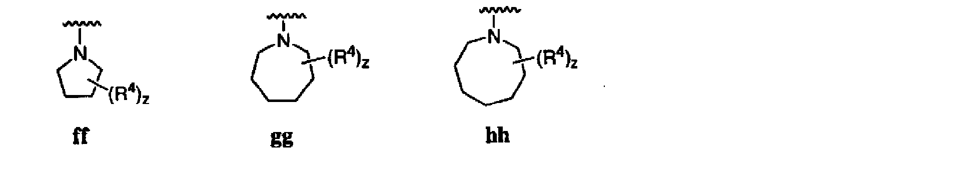 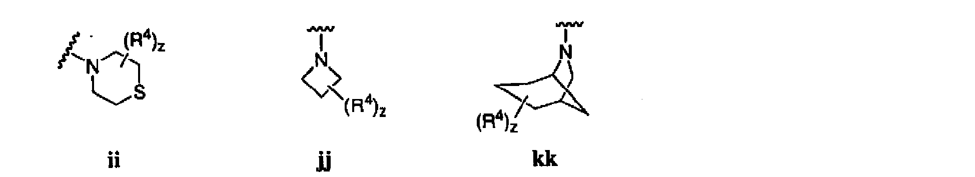 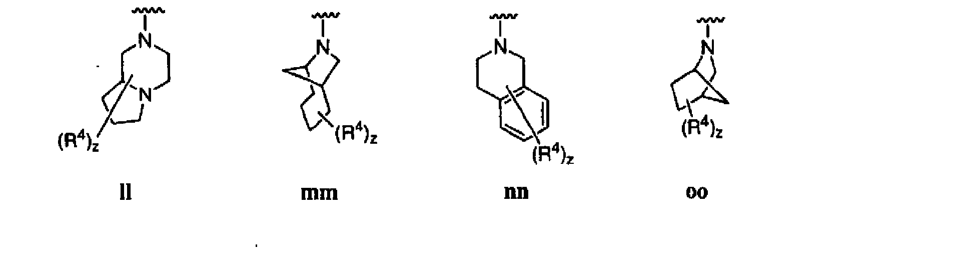 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')2_{,} -OCON(R')₂, COR', -NHCOOR', - SO2R', -SO₂N(R'h, or an optionally substituted group selected from C₁-C₆aliphatic, aryl, heteroaryl, cycloaliphatic, heterocycloaliphatic, arylC₁-C₆₈1kyl, heteromylC₁-C₆alkyl, cycloaliphaticC₁-C₆S,lkyl, or heterocyclaaliphatic₁-C₆alkyl;
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')2_{,} -OCON(R')2_{,} COR', -NHCOOR', -SO₂R', -SO₂N(R')2, or an optionally substituted group selected from C₁C₆aliphatic, aryl, heteroaryl, cycloaliphatic, heterocycloaliphatic, arylC₁-C₆alkyl, heteroarylC₁-C₆alkyl, cycloaliphaticCl-C6alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', - OCON(R')₂, -NR'SO2_{R}', -OP(O)(OR')2_{,} -P(O)(OR')_{2,} -OP(O)₂OR', -P(O)₂OR', - PO(R')₂, -OPO(R')₂, or an optionally substituted group selected from C₁.C₆aliphatic, aryl, heteroaryl, cycloaliphatic, heterocycloaliphatic, arylC1-C₆alkyl, heteroarylC₁-C₆alkyl, cycloaliphaticC₁₋C₆alkyl, or heterocycloaliphaticc₁-c₆alkyl; and
each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
and
(d) removing the suitable hydroxyl protecting group to form a compound of formula **Ia.**

3. The method according to clause 2, wherein said method further comprises the step of forming a salt of the compound of formula **Ia.**

4. A method for preparing a compound of formula Ia: 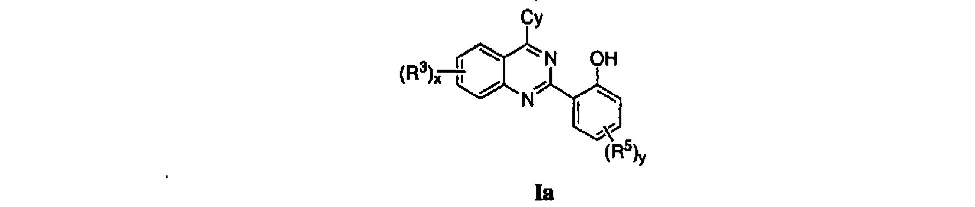 or a suitable salt thereof; wherein:
Cy is a ring selected from: 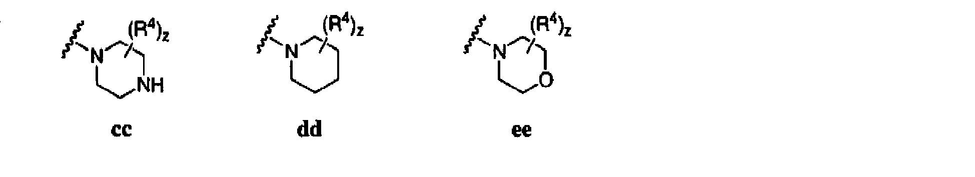 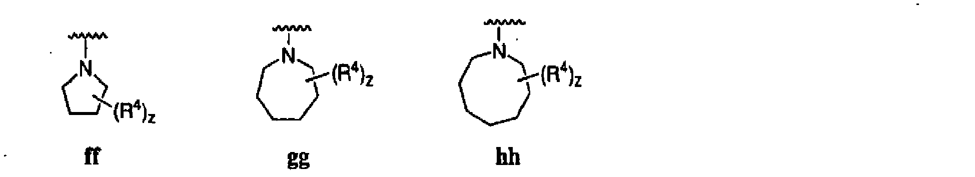 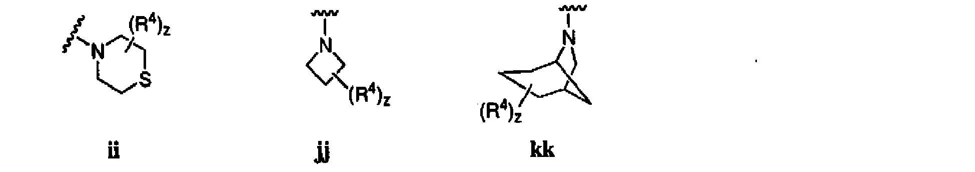 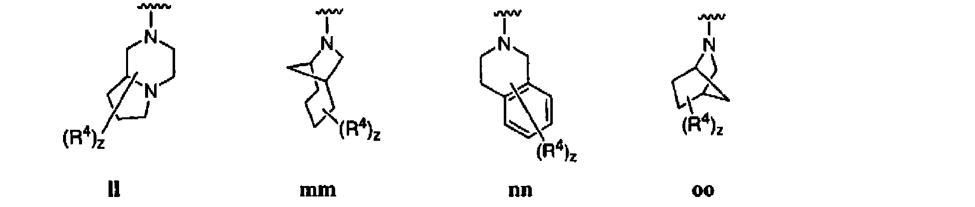 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')-2, COR', -NHCOOR', - SO₂R', -SO₂N(R')2_{,} or an optionally substituted group selected from C₁-C₆aliphatic, aryl, heteroaryl, cyctoaliphatic, heterocycloaliphatic, arylC₁-C₆alkyl, beteroarylc₁-C₆alkyl, cycloaliphaticC₁-C₆alkyl, or heterocycloaliphaticC₁-C₆alky I;
x is 0-4;
each R³ is independently halogen, CN, N0₂, -N(R')₂, -CH₂N(R')₂, -OR', -CH₂OR', -SR', -CH₂SR', -COOR', -NRCOR', -CON(R')₂, -OCON(R')₂, COR', -NHCOOR', - S0₂R', -S0₂N(R')₂, or an optionally substituted group selected from C₁₋C₆aliphatic, aryl, heteroaryl, cycloaliphatic, heterocycloaliphatic, arylC₁₋C₆a]kyl, heteroarylC₁-C₆alkyl, cyaoaliphaticc₁-C₆alkyl, or heterocycloaliphaticC₁-C₆alkyl;
y is 0-5;
each R⁵ is independently halogen, CN, N0₂, -N(R')2_{,} -CH₂N(R')₂, -OR', -CH₂OR', -SR', -CH₂SR', -NRCOR', -CON(R')₂, -S(0)₂N(R')₂, -OCOR', -COR', -CO₂R', - OCON(R')₂, NR'SO₂R', -OP(O)(OR')₂, -P(0)(OR')₂, -OP(0)₂0R', -P(O)₂OR', - PO(R')₂, -OPO(R')2_{,} or an optionally substituted group selected from C1.C₆aliphatic, aryl, heteroaryl, cycloaliphatic, heterocycloaliphatic, arylC₁-C₆alkyl, heteroarylc₁-C₆alkyl, cycloaliphaticC₁-C₆alkyl, or heterocycloaliphaticC₁-C₆alkyl; and
each occurrence of R' is independently hydrogen or an optionally substituted Cₗ-₆
aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or: two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
comprising the steps of:
(a) providing a compound of formula **III:** 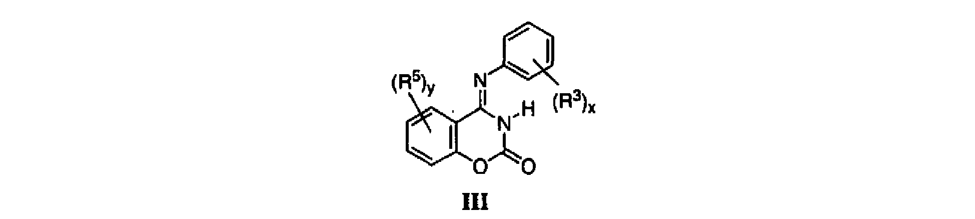 or a suitable salt thereof;
wherein:
x is 0-4;
each R³ is independently halogen, CN, NO₂, -N(R')₂, .CH₂N(R')₂, -OR', -CH₂OR', -SR', -CH₂SR', -COOR', -NRCOR', -CON(R')₂, -OCON(R')₂, COR', -NHCOOR', - SO₂R', -SO₂N(R')₂, or an optionally substituted group selected from C₁₋C₆aliphatic, aryl, heteroaryl, cycloaliphatic, heterocycloaliphatic, arylC₁-C₆alkyl, heteroarylCₗ-C₆alkyl, cycloaliphaticC₁-C₆alkyl, or hetervcycloaliphaticC₁-Cₛalkyl;
y is 0-5;
each R⁵ is independently halogen, CN, NO₂, -N(R')₂, -CH2N(R')z, -OR', -CH₂OR', -SR', .-CH₂SR', -NRCOR', -CON(R')₂, -S(O)₂N(R')₂, -OCOR', -COR', -CO₂R', - OCON(R')₂, -NR'SO₂R', -OP(O)(OR')2_{,} -P(O)(OR')₂, -OP(O)_{z}OR', -P(O)₂OR', - PO(R')₂, -OPO(R')₂, or an optionally substituted group selected from C₁-C,6a]iphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C6alkyl; and
each occurrence of R' is independently hydrogen or an optionally substituted C₁₋₆
aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or Fully unsaturated bicyclic ring system having 0-5 heteroatoms independently selected from nitrogen, oxygen, or sulfur; or:
two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
(b) converting said compound of formula **III** or a suitable salt thereof, to a compound of formula **II:** 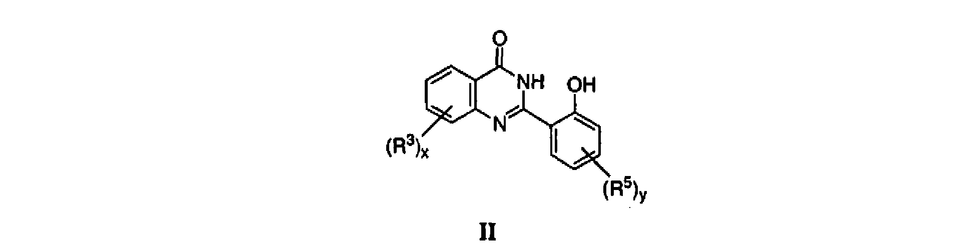 or a suitable salt thereof;
wherein:
x is 0-4;
each R³ is independently halogen, CN, NO₂, -N(R')₂, -CH₂N(R')₂, -OR', -CH₂OR', -SR', -CH₂SR', -COOR', -NRCOR', -CON(R')2, -OCON(R')₂. COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C6alkyl, cycloaliphatícC¡-C₆alkyl, or heterocyaoaliphaticC₁-C₆alkyl;
y is 0-5;
each R⁵ is independently halogen, CN, NO₂, -N(R')2_{,} -CH₂N(R')2_{,} -OR', -CH₂OR', -SR', -CH₂SR', -NRCOR', -CON(R')2_{,} -S(O)₃N(R')₂, -OCOR', -COR', -CO₂R', - OCON(R')₂, -NR'SO₂R', -OP(O)(OR')₂, -P(O)(OR')2_{,} -OP(O)₂OR', -P(O)₂OR', - PO(R')₂, -OPO(R')₂, or an optionally substituted group selected from C₁.C₆anphatic, aryl, heteroaryl, cycloaliphatic, heterocycloaliphatic, arylC₁-C₆alkyl, heteroarylc₁-C₆alkyl, cycloaliphaticC₁-C₆alkyl, or heterocycloaliphaticC₁-C₆alkyl; and
each occurrence of R' is independently hydrogen or an optionally substituted C₁₋₆
aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or: two occurrences of R' are taken together with the atom(s) to which they are bound
to form an optionally substituted 3-12 membered saturated, partially unsaturated, or fully unsaturated monocyclic or bicyclic ring having 0-4 heteroatoms independently selected from nitrogen, oxygen, or sulfur;
and
(c) converting said compound of formula **II** or a suitable salt thereof, to a compound of formula **Ia** or a suitable salt thereof.

5. The method according to clause 4, wherein the conversion of a compound of formula III to a compound of formula **II**, at step (b), is affected by heating.

6. The method according to clause 4 to prepare a compound of formula V from a compound of formula **Iaa-1:** 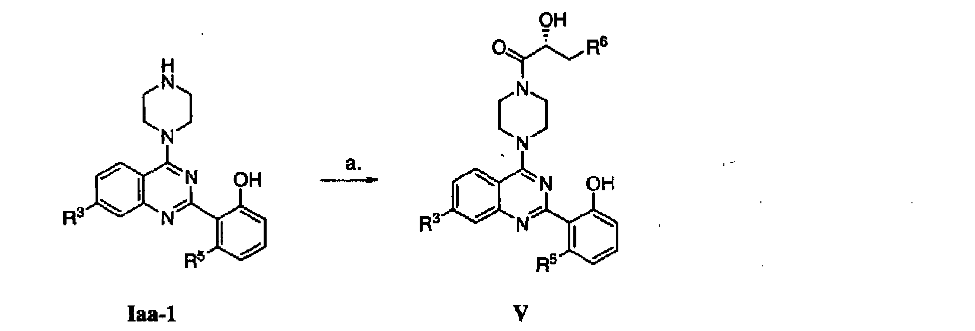 comprising the additional step of:
(a) reacting compound of formula laa-1 with (R) isocaproic acid under suitable
amide coupling conditions;
wherein R⁶ is isopropyl or t-butyl, R³ is methyl or hydrogen, and R⁵ is fluorine or hydrogen.

7. The method according to clause 6, wherein said method further comprises the step of forming a salt of the compound of formula V.

8. The method according to clause 6 or 7, wherein R⁶ is isopropyl, R³ is methyl,
and R⁵ is hydrogen.

9. The method according to clause 6 or 7, wherein R⁶ is t-butyl, R³ is methyl, and R⁵ is hydrogen.

10. The method according to clause 6 or 7, wherein R⁶ is isopropyl, R³ is hydrogen, and R⁵ is hydrogen.

11. The method according to clause 6 or 7, wherein R⁶ is t-butyl, R³ is methyl, and R⁵ is fluorine.

12. A compound of formula II: 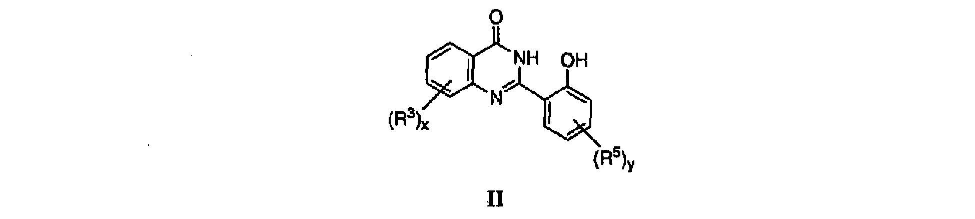 or a suitable salt thereof;
wherein:
x is 0-4;
each R³ is independently halogen, CN, NO₂, .N(R' )₂, -CH2N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')2, -S(0)₂N(R')₂, -OCOR', -COR', -CO₂R', -OCON(R')2_{,} -NR'SO₂R', -OP(O)(OR')₂, -P(O)(OR')₂, -OP(0)₂0R', -P(0)₂0R'-, -PO(R')₂, -OPO(R')₂, or an optionally substituted group selected from C₁_C₆aliphatic, aryl, heteroaryl, cycloaliphatic, heterocycloaliphatic, arylC_{I}-C₆alkyl, heteroary1C₁-C₆alkyl, cycloaliphaticC₁-C₆alkyl, or heterocycloaliphaticC₁-C₆1kyl; and
each occurrence of R' is independently hydrogen or an optionally substituted C₁₋₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
provided that the following compounds are excluded;
Glycine, N-[2-[2-[6-[bis(carboxymethyl)amino]-2,3-difluorophenoxylethoxy]-4-(3,4-dihydro-4-oxo-2-quinazolinyl)-5-hydroxyphenyl]-N-(carboxymethyl)-, tetrapotassium salt;
Glycine, N-[2-[2-[2-[bis(carhoxymethyi}amino]-5-(1,4-dihydro-4-oxo-2-quinazolinyl)-4-hydroxyphenoxy]ethoxy]-4-fluorophenyl]-N-(carboxymethyl)-, tetrapotassium salt;
Glycine, N-[2-[2-[2-[bis(2-methoxy-2-oxoethyl)amino]-5-(1,4-dihydro-4-oxo-2-quinazolinyl)-4-hydroxyphenoxy]ethoxy]-4-fluorophenyl]-N-(2-methoxy-2-oxoethyl)-, methyl ester;
Glycine, N-[2-[2-[6-[bis(2-methoxy-2-oxoethyl)amino]-2,3-difluorophenoxy]ethoxy]-4-(3,4-dihydro-4-oxo-2-quinazolinyl)-5-hydroxyphenyl]-N-(2-methoxy-2-oxoethyl)-, methyl ester;
Glycine, N-[2-[2-[2-[bis(carboxymethyl)amino]-5-(1,4-dihydro-4-oxo-2-quinazolinylp4-hydroxyphenoxy]ethoxy]-4-methylphenyl]-N-(carboxymethyl)-;
Glycine, N-[2-[2-[2-[bis(carboxymethyl)amino]-5-(1,4-dihydro-4-oxo-2-quinazolinyl)-4-hydroxyphenoxy]ethoxy]-4-fluorophenyl]-N-(carboxymethyl)-; 4(1H)-Quinazolinone, 6-amino-2-(2-hydroxyphenyl)-;
4(1H)-Quinazolinone, 2-(2-hydroxyphenyl)-6-nitro-;
4(1H)-Quinazolinone, 2-(2-hydroxyphenyl)-;
4(1H)-Quinazolinone, 6-chloro-2-(5-chloro-2-hydroxyphenyl)
4(1H)-Quinazolinone, 6-butyl-2-(5-butyl-2-hydroxyphenyl)-;
4(IH)-Quinazolinone, 6-bromo-2-(5-bromo-2-hydroxyphenyl}-;
4(1H)-Quinazolinone, 2-(2-hydroxy-5-pentylphenyl)-6-pentyt-;
4(1H)-Quinazolinone, 2-(2-hydroxyphenyl)-;
4(1H)-Quinazofinane, 6-chloro-2-(2-hydroxyphenyl)
4(l H)-Quinazolinone, 2-(2-hydroxyphenyl)-6-methyl-;
4(1 H)-Quinazolinone, 6-chloro-2-(5-chloro-2-hydroxyphenyl)-,
4(1 H)-Quinazolinone. 2-(2-hydroxyphenyl)-6-iodo-;
4(1H)-Quinazolinone, 2-(5-chloro-2-hydroxyphenyl)-;
4(1 H)-QuinazoJinone, 2-(2-hydroxy-4-methoxyphenyl)-;
4(1 H)-Quinazolinone, 6-chloro-2-(2-hydroxyphenyl)-;
4(1 H)-Quinazohnone, 2-(2-hydroxyphenyl)-6-nitro-;
4(1 H)-Quinazolinone, 6-chloro-2-(5-chloro-2-hydroxypheny 1)-;
4(1H)-Quinazolinone, 6-chloro-2-{3,5-dichloro-2-hydraxyphenyl)-;
4(1H)-Quinazolinane, 2-(2-hyd_{TOX}y-5-methoxyphenyl)-;
4(IH)-Quinazolinone, 2-(2-hydrox y-5-nitrophenyl)-;
4(1H)-Quinazolinone, 6-chloro-2-(2-hydroxy-5-methoxyphenyl)-;
4(IH)-Quinazolinone, 2-(2-hydroxy-5-nitrophenyl)-6-nitro-;
4(1H)-Quinazolinone, 6-chloro-2-(2-hydroxy-5-nitrophenyl)-;
4(1H}-Quinazolinone, 2-(3-fluoro-2-hydroxyphenyl)-;
4(1H)-Quinazolinone, 6-chloro-2-(3-fluoro-2-hydroxyphenyl)-;
4(1H)-Quinazolinone, 2-[5-( 1 , 1-dimethylethyl)-2-hydroxyphenyl]-;
4(1H)-Quinazolinone, 2-(4-hydroxy[1,1'-biphenyll-3-yi)-;
4(1H)-Quinazolinone, 2-(4-chloro-2-hydroxyphenyl}-;
4(1H)-Quinazolinone, 2-(2-hydroxy-3-methylphenyI)-;
4(1H)-Quinazolinone, 2-(3,5-dibromo-2-hydroxyphenyt)-;
4(1H)-Quinazolinone, 8-bromo-2-(3,5-dibromo-2-hydroxyphenyl)-;
4(1H)-Quinazolinone, b,8-dibroimo-2-(3,5-dibromo-2-hydroxyphenyl)-;
4(1H)-Quinazolinone, 2-(3,S-dichloro-2-hydroxyphenyl)-;
4(3H)-Quinazolinone, 2-(4-ethoxy-2-hydroxyphenyl)-;
4(3H)-Quinazolinone, 6-chloro-2-(2-hydrox y-m-tolyl)-;
4(3H)-Quinazolinone, 2-(2-hydroxy-m-tolyl)-6-nitro-;
4(3H)-Quinazolinone, 2-(3,5-dichloro-2-hydroxyphenyl)-6-nitro-;
4(3H)-Quinazolinone, 2-(3,5-dichloro-2-hydroxyphenyl)-6-nitro-;
4(1 H)-Quinazolinone, 2-(5-chloro-2-hydroxyphenyl)-6-nitro-;
4(3H)-Quinazolinone, 2-(2-hydroxy-5-iodophenyl)-;
4(3H)-Quinazolinone, 6-chloro-2-(2-hy&oxy-5-iodophenyl)-;
4(3H)-Quinazolinone, 2-(2-hydroxy-5-iodophenyl)-6-nitro-;
4(3H)-Quinazolinone, 2-(5-bromo-2-hydroxyphenyl)-6-chloro-;
4(3H)-Quinazolinone, 2-(5-bromo-2-hydroxyphenyl)-6-nitro-;
4(3H)-Quinazolinone, 6-chloro.2-( 4-ethoxy- 2-hydroxyphenyl)-;
4(3H)-Quinazolinone, 2-(4-ethoxy-2-hydroxyphenyl)-6-nitro-;
4(3H)-Quinazolinone, 6-chloro-2-(2,4-dihydroxyphenyl)-;
4(3H)-Quinazolinone, 2-(2,4-dihydroxyphenyl)-6-nitro-;
4(3H)-Quinazolinone, 6-chloro-2-(3,5-dibromo-2-hydroxyphenyl)-;
4(3H)-Quinazolinone, 2-(3,5-dibromo-2-hydroxyphenyl}-b-nitro-;
4(3H)-Quinazoli none, 2-(2-hydroxy-3-biphenylyl)-;
4(3H)-Quinazoli none, 2-(2,5-dihydroxyphenyl)--,
4(3H)-Quinazolinone, 6-chloro-2-(2,5-dihydroxyphenyl)-;
4(3H)-Quinazolinone, 2-(2,5-dihydroxyphenyl)-6-nitro-;
[2-(2-12-(Carboxymethyl-amino)-5-methyl-phenoxy]-ethoxyl-5-hydroxy-4-(4-hydroxy-quinazolin-2-yl)-phenylamino]-acetic acid; and
12-(2-[6-(Ca_{T}boxyrnethyl-amino)-2,3-difluoro-phenoxyl-ethoxy)-5-hydroxy-4-(4-hydroxy-quinazolin-2-yI)-phenylamino]-acetic acid.

13. The compound according to clause 12, wherein:
x is 1 or 2;
each R³ is independently Cl, Br, F, CF₃, -OCF₃, Me, Et, CN, -COOH, -NH₂, -N(CH₃h, - N(Etl₂, -N(iPr)₂, -O(CH₂)₂0CH₃, -CONH₂, -COOCH₃, -OH, -OCH₃, -OCH₂CH₃, - CH₂OH, -NHCOCH₃, -NHCOCH(CH₃)₂, -SO₂NH₂, -CONH(_{C}yclopropyl), -CONHCH₃, -CONHCH₂CH₃, or an optionally substituted group selected from -piperidinyl, piperizinyl, morpholino, phenyl, phenyloxy, benzyl, or benzyloxy;
y is 0-4; and
each R⁵ is independently Cl, Br, F, CF₃, Me, Et, CN, -COOH, -NH₂, -N(CH₃)₂, -N(Et)_{z}, - N(iPrh. -O(CH₂)₂0CH₃, -CONH₂, -COOCH₃, -OH, -OCH₃, -OCH₂CH₃, -CH₂OH, _ NHCOCH₃, -SO₂NH₂, -S0₂NHC(CH₃)₂, -OCOC(CH₃)₃, -OCOCH₂C(CH₃ₕ, - Q(CH_{z})_{z}N(CH₃ₕ. 4-CH₃-_{P}iperazin-1-yl, OCOCH(CH),2. OCO(cyclopentyl), -COCH₃. optionally substituted phenoxy, or optionally substituted benzyloxy.

14. The compound according to clause 13 wherein:
x is 1;
each R³ is independently Cl, Br, F, CF₃, -OCF₃, Me, Et, CN, -COOH, -OH, or -OCH₃;
y is 0 or 1; and
each R^{s} is independently Cl, Br, F, CF₃, Me, -OH, -OCH₃, -OCH₂CH₃, -CH₂OH, - NHCOCH₃, -SO₂NH₂, -S0₂NHC(CH₃)₂. clause

15. The compound according to clause 14, wherein said compound is compound **II-1** or a suitable salt thereof:

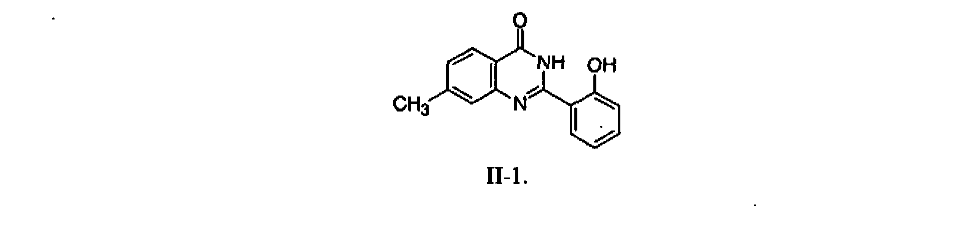

16. A compound of formula IIa: 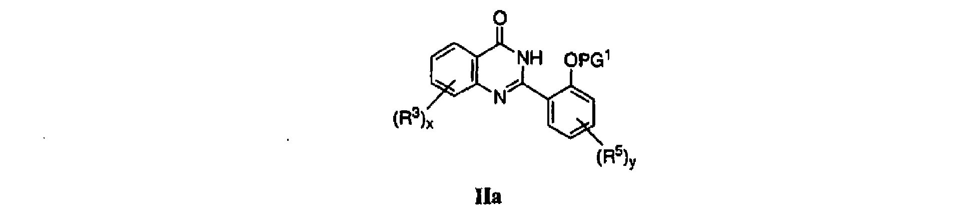 or a suitable salt thereof;
wherein:
PG^{t} is a suitable hydroxyl protecting group;
x is 0-4;
each R³ is independently halogen, CN, NO₂, -N(R')x, -CH2N(R')₂, -OR', -CH₂0R', -SR', -CH₂SR', -COOR', -NRCOR', -CON(R')_{z}, -OCON(R')2_{,} COR', -NHCOOR', .S0₂R' , -SO₂N(R'}₂, or an optionally substituted group selected from Cₗ_C₆aliphatic, aryl, heteroaryl, cycloaliphatic, heterocycloaliphatic, arylC₁-C₆alkyl, heteroaryIC¡-C₆alky I, cycloaliphaticCₜ-4alkyl, or heterocycloaliphaticCₗ-C₆alkyl;
y is 0-5;
each R⁵ is independently halogen, CN, NO₂, -N(R')₂, -CH₂N(R')_{z}, -OR', -CH_{z}OR', -SR', -CH_{Z}SR', -NRCOR', -CON(R')₂, -S(0)₂N(R')₂, -OCOR', -COR', -CO₂R', -OCON(R')₂, -NR'S02_{R}', -OP(O)(OR'h, -P(O)(OR')₂, -OP(O)_{z}OR', -P(O)2OR', -PO(R')2_{,} -OPO(R')2_{,} or an optionally substituted group selected from C_{I}.C₆aliphatic, aryl, heteroaryl, cycloaliphatic, heterocycloaliphatic, arylCᵢ-C₆alkyl, heteroarylC,-C₆alkyl, cycloaliphaticC,-C₆a]kyl, or heterocycloaliphaticC₁-C₆alkyl; and
each occurrence of R' is independently hydrogen or an optionally substituted Cₗ.₆ aliphatic
group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
provided that the following compounds are excluded:
4(1H)-Quinazolinone, 6-chloro-2-[5-chloro-2-(2,2-dimethoxyethoxy)phenyl]-
4(1H)-Quinazolinone, 2-(2-methoxyphenyl)-;
4(1H)-Quinazolinone, 2-(2,4-dimethoxyphenyl)-;
4(1H)-Quinazolinone, 2-(2,3-dimethoxyphenyl)-;
4(1H)-Quinazolinone, 2-(2,5-dimethoxyphenyl)-;
Benzoic acid, 4-[(aminoiminomethyl)amino]-, 2-(1,4-dihydro-4-oxo-2-quinazolinyl)phenyl ester, monohydrochloride;
4(1H)-Quinazolinone, 2- [2-(acetytoxy)phenyl]-;
4(1H)-Quinazolinone, 2-(2-methoxyphenyl)-;
4(1H)-Quinazoli none, 2-(2-methoxyphenyl)-7-{trifluaramethyl}-;
4(1H)-Quinazolinone, 2-(2-methoxyphenyl)-7-methyl-;
4(1H)-Quinazolinone, 2-[2-(acetyloxy)-5-chlorophenyl]-6-chloro-;
6-Quinazolinecarboxylic acid, 2-(2,3-dimethoxyphenyl)-1,4-dihydro-4-oxo-;
6-Quinazolinecarboxylic acid, 2-(5-edioxy-2-methoxyphenyl)-1,4-dihydro-4-;
6-Quinazolinecarboxylic acid, 1,4-dihydro-2-[2-methoxy-5-(2-propenyloxy)phenyl]-4-oxo-;
6-Quinazolinecarboxylic acid, 1 ,4-dihydro-2-(2.methoxy-3-methylphenyl)-4-oxo-;
6-Quinazolinecarboxylic acid, 1,4-dihydro-2-[2-methoxy-5-(1-methylethoxy)phenyl]-4-oxo-;
6-Quinazolinecarboxylic acid, 1,4-dihydro-2-(2-methoxy-5-propoxyphenyl)-4-oxo-;
6-Quinazolinecarboxylic acid, 2-[5-(2-ethoxyethoxy)-2-methoxyphenyl]-1,4-dihydro-4-oxo-;
6-Quinazolinecarboxylic acid, 2-(3-ethoxy-2-methoxyphenyl)-1,4-dihydro-4-oxo-;
6-Quinazolinecarboxylic acid, 1,4-dihydra-2-(2-methoxyphenyl)-4-oxo-; 6-Quinazolinecarboxylic acid, 1,4-dihytlro-4-oxa-2-[2-(2-propenyloxy)phenyl]-;
6-Quinazolinecarboxylic acid, 2-[2-(2-ethoxyethoxy)phenyl]-1,4-dihydro-4-oxo-;
6-Quinazolinecarboxylic acid, 2-(2,3-dimethoxyphenyl)-I,4-dihydro-4-oxo-;
6-Quinazolinecarboxylic acid, 1,4-dihydra-2-(2-methoxy-3-methylphenyl)-4-oxo-;
6-Quinazolinecarboxylic acid, 1,4-dihydro-2-(2-methoxy-5-methylphenyl)-4-oxo-;
6-Quinazolinecarboxylic acid, 2-[2-(2-ethoxyethoxy)-3-methoxyphenyt]-1,4-dihydro-4-oxo-;
6-Quinazolinecarboxylic acid, 1,4-dihydro-2-(2-methoxyphenyl)-4-oxo-, methyl ester;
4(1H)-Quinazolinone, 6,7 ,8-trimethoxy-2-(2,3,4-trimethoxyphenyl)-; Carbonic acid, ethyl ester, ester with 2-(o-hydroxypbenyl)-4(3H)-Quinazolinone;
4(3H)-Quinazolinone, 6-butyl-2-(o-methoxyphenyl)-;
4(1H)-Quinazolinone, 2-(3,5-dibromo-2-methoxyphenyl)-; and
2-(2'-acetoxyphenyl)-4(3H)-quinazolinone.

17. The compound according to clause 16, wherein:
x is 1 or 2; and
each R³ is independently Cl, Br, F, CF₃, -OCF₃, Me, Et, CN, -COOH, -NH₂, -N(CH₃)₂, -
N(Et)₂, .N(iPr)₂, -O(CH₂)₂0CH₃, -CONH₂, -COOCH₃, -OH, -OCH₃, -OCH₂CH₃, - CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy.

18. The compound according to clause 17, wherein:
x is 1; and
R³ i5 Cl, Br, F, CF₃, -OCF₃, Me, Et, CN, -COOH, -OH, or -OCH₃.

19. The compound according to clause 18, wherein:
y is 0-4; and
each R⁵ is independently Cl, Br, F, CF₃, Me, Et, CN, -COOH, -NH₂, -N(CH₃)₂, -N(Et)₂, - N(iPr)₂, -O(CH₂)₂OCH₃, -CONH₂, -COOCH₃, -OH, -OCH₃, -OCH₂CH₃, -CH₂OH, - NHCOCH₃, -SO₂NH₂, -SO_{z}NHC(CH₃)₂, -OCOC(CH₃)₃, -OCOCH₂C(CH₃)₃, - O(CH₂)2N(CH₃)2,4-CH₃-piperazin-1-yl, OCOCH(CH₃)₂, OCO(cyclopentyl), -COCH₃, optionally substituted phenoxy, or optionally substituted benzyloxy.

20. The compound according to clause 19, wherein:
y is 0 or 1; and
R⁵ is Cl, Br, F, CF₃, Me, -OH, -OCH₃, -OCH₂CH₃, -CH₂OH, -NHCOCH₃, -S0₂NH₂, -SO₂NHC(CH₃)₂.

21. The compound according to clause 16, wherein said compound is compound IIa-l or a suitable salt thereof:

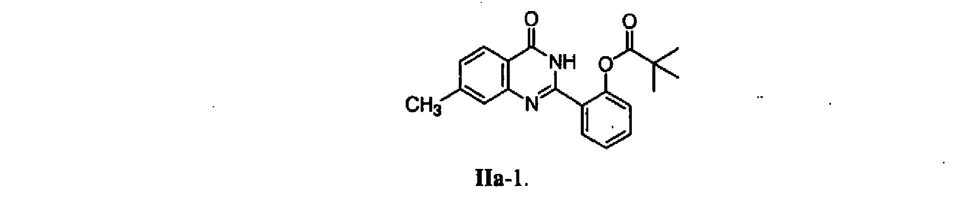

22. A compound of formula IIc: 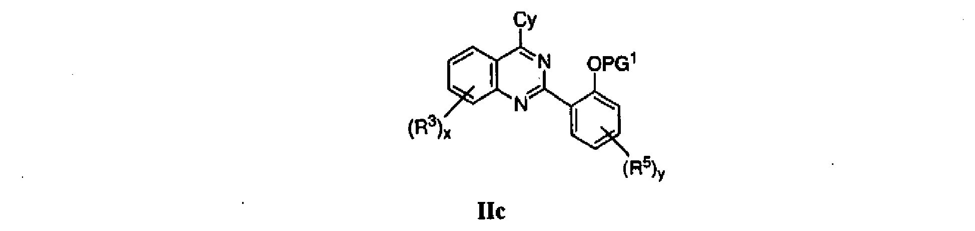 or a suitable salt thereof;
wherein:
PG' is a suitable hydroxyl protecting group;
Cy is a ring selected from: 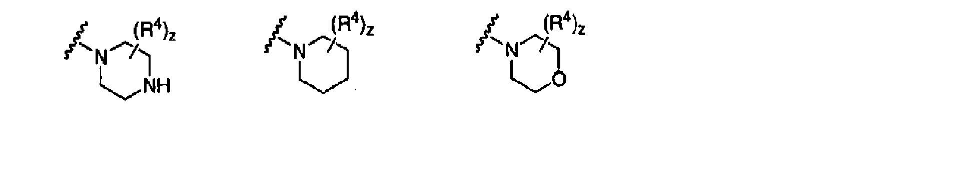 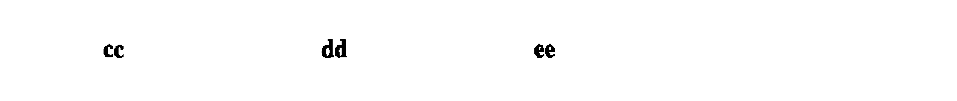 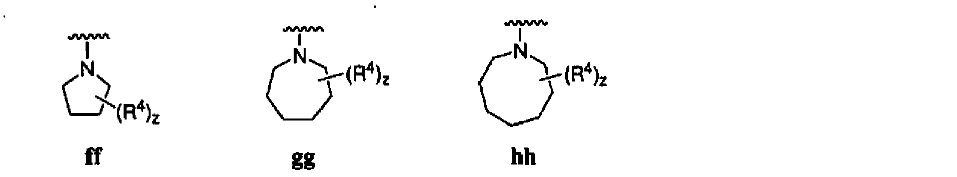 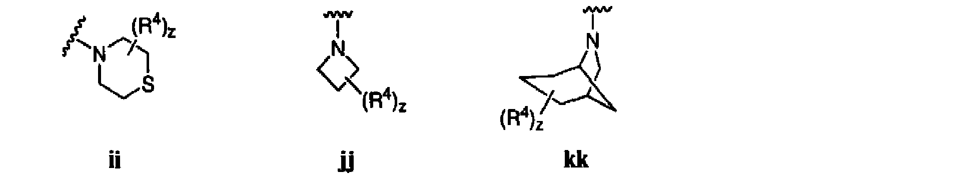 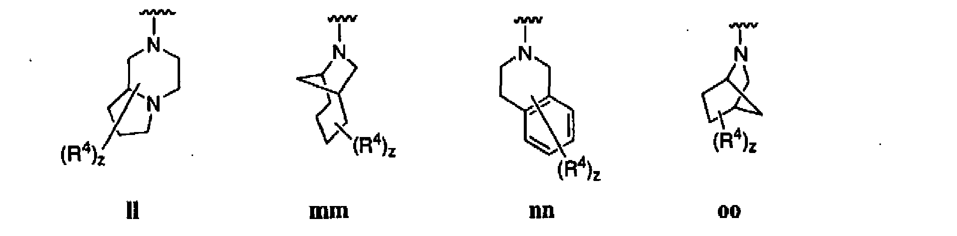 and Cy is optionally substituted at one or more substitutable carbon, nitrogen, or sulfur atoms with z independent occurrences of -R⁴;
each z is independently 0-5;
each R⁴ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')_{2,} or an optionally substituted group selected from C₁-C₆aliphatic, aryl, heteroaryl, cycloaliphatic, hete;ocycloaiipharic, arylC₁-C₆alkyl, heteroarylC_{I}-C₆alkyl, cycloaliphaticC₁-C₆alkyl, or heterocycloaliphaticC₁-C₆alkyl;
x is 0-4;
each R³ is independently halogen, CN, NO₂, -N(R')2_{,} -CH₂N(R')₂, -OR', -CH₂OR', -SR', -CH₂SR', -COOR', -NRCOR', -CON(R')₂, -OCON(R')₂, COR', -NHCOOR', -SO2R', -S02N(R')₂, or an optionally substituted group selected from C₁-C₆aliphatic, aryl, heteroaryl, cycloaliphatic, heterocycloaliphatic, arylC₁-C₆alkyl, heteroarylC_{I}-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)2N(R')2, -OCOR', -COR', -CO₂R', -OCON(R')₂, -NR'SO₂R', -OP(O)(OR')₂, -P(O)(OR')₂, -OP(O)₂OR', -P(O)₂OR', -PO(R')₂, -OPO(R')₂,
or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl; and
each occurrence of R' is independently hydrogen or an optionally substituted Cₗ-₆ aliphatic group, a 3-8-membered saturated, partially unsaturated, or fully unsaturated monocyclic ring having 0-3 heteroatoms independently selected from nitrogen, oxygen, or sulfur, or an $-12 membered saturated, partially unsaturated, or fully unsaturated bicyclic ring system having 0-5 heteroatoms independently selected from nitrogen, oxygen, or sulfur;
or:
two occurrences of R' are taken together with the atom(s) to which they are bound to form an
optionally substituted 3-12 membered saturated, partially unsaturated, or fully unsaturated monocyclic or bicyclic ring having 0-4 heteroatoms independently selected from nitrogen, oxygen, or sulfur;
provided that the following compounds are excluded:
Quinazoline, 2-(2-methoxyphenyl)-4-(1-pyrrolidinyl)-;
Quinazoline, 6-bromo-2-(2-methoxyphenyl)-4-(4-morpholinyl)-;
Quinazoline, 6,8-dichloro-2-(2-methoxyphenyl)-4-(4-morpholinyl)-;
Quinazoline, 6-bromo-2-(2-methoxyphenyl)-4-(1-pyrrolidinyl)-;
Quinazoline, 6,8-dichloro-2-(2-methoxyphenyl)-4-(1-pyrrolidinyl)-;
Quinazoline, 2-(2-fluoro-6-methoxyphenyl)-6-methoxy-4-(4-morpholinyl)-;
Quinazoline, 2-(2-fluoro-6-methoxyphenyl)-4-(4-methyl- I -piperidinyl)-7-(trifluoromethyl)-;
Cyclopropanecarboxylic acid, 3-fluoro-2-[7-methyl-4-(4-methyl-1-piperidinyl)-2-quinazolinyl]phenyl ester;
Propanoic acid, 2-methyl-, 3-fluoro-2-[7-methyl-4-(4-methyl-1-piperidinyl)-2-quinazolinyl]phenyl ester;
Butanoic acid, 3-methyl-, 3-fluoro-2-[7-methyl-4-(4-methyl-1-piperidinyl)-2-quinazolinyl]phenyl ester;
Cyclopentanecarboxylic acid, 3-fluoro-2-[7-methyl-4-(4-methyl-1-piperidinyl)-2-quinazolinyl]phenyl ester;
Propanoic acid, 2,2-dimethyl-, 3-fluoro-2-[7-methyl-4-(4-methyl-1-piperidinyl)-2-quinazolinyl]phenyl ester;
Butanoic acid, 3,3-dimethyl-, 3-fluoro-2-[7-methyl-4-(4-methyl-l-piperidinyl)-2-quinazolinyl]phenyl ester;
Quinazoline, 7-chloro-2-(2-methoxyphenyl)-4-[3-(trifluoromethyl)-1-pyrrolidinyl];
Piperazine, 1-(butylsulfonyl)-4-[2-(2,4-dimethoxyphenyl)-7-methyl-4-quinazolinyl]-;
Phenol, 3-fluoro-2-[7-methyl-4-(4-methyl-1-piperidinyl)-2-quinazolinyl]-, acetate (ester);
Piperazine, 1-(butylsulfonyl)-4-[2-(2-fluoro-6-methoxyphenyl)-7-(trifluoromethyl)-4-quinazolinyl]-;
1-Piperazinecarboxylic acid, 4-[6-bromo-2-(2-methoxyphenyl)-4-quinazolinyl]-, 1,1-dimethylethyl ester;
Carbamic acid, (2-methylpropyl)-, 1-[2-(2-methoxyghenyl)-7-methyl-4-quinazolinyl]-4-piperidinyl ester;
6-Quinazolinecarboxylic acid, 4-[4-[(1,1-dimethytethoxy)carbonyl]-1-piperazinyl]-2-(2-methoxyphenyl)-; and
Benzenesulfonaxnide, 2-methoxy-5-[2-(4-[2-(2-methoxyphenyl)-4-quinazolinyl]-1-piperazinyl]ethyl]-, (2Z)-Z-butenedioate (2:3).

23. The compound according to clause 22, wherein:
PG¹ is a suitable hydroxyl protecting group;
Cy is azetidin-1-yl (jj), pyrrolidin-1-yl (ff), piperidinl-yl (dd), or piperazin-1-yl (cc)
optionally substituted with 0-4 occurrences of R⁴;
each R⁴ is independently Cl, Br, F, CF₃, CH₃, -CH₂CH₃, CN, -COOH, -N(CH₃)₂, -N(Et)₂, -N(iPr)₂, -O(CH₂)₂OCH₃, -CONH₂, -COOCH₃, -OH, -CH₂OH, -NHCOCH₃, -S0₂NH₂, -SO₂(CH₂)₃CH₃, -SO₂CH(CH₃)₂, -SO₂N(CH₃)₂, -SO₂CH₂CH₃, -C(O)OCH₂CH(CH₃)₂, -C(0)NHCH₂CH(CH₃)₂, -C(O)CH(OH)CH₂CH(CH₃)₂, -C(O)CH(OH)CH₂C(CH₃)₃. -NHCOOCH₃, -C(O)C(CH₃)₃, -COO(CH₂)₂CH₃, -C(O)NHCH(CH₃)₂, ₋C(O)CH₂CH₃, or an optionally substituted group selected from -piperidinyl, piperizinyl, morpholino, C₁-₄alkoxy, phenyl, phenyloxy, benzyl, benzyloxy, -CH₂cyclohexyl, pyridyl, -CH₂pyridyl, or
-CH₂thiazolyl;
x is I or2;
each R³ is independently Cl, Br, F, CF₃, -OCF₃, Me, Et, CN, -COOH, -NH₂, -N(CH₃)₂, -
N(Et)₂. -N(iPr)₂, -O(CH₂)₂OCH₃, -CONH₂, -COOCH₃, -OH, -OCH₃, -OCH₂CH₃, - CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy;
y is 0-4; and
each R⁵ is independently Cl, Br, F, CF₃, Me, Et, CN, -COOH, -NH₂, -N(CH₃)₂, -N(Et)₂,-N(iPr)₂, -O(CH₂)₂0CH₃, -CONH₂, -COOCH₃, -OH, -OCH₃, -OCH₂CH₃, -CH₂OH, - NHCOCH₃, -SO₂NH₂, -SO₂NHC(CH₃)₂, -OCOC(CH₃)₃, -OCOCH₂C(CH₃)₃, - 0(CH₂)₂N(CH₃)₂, 4-CH₃-piperazin-1-yl, OCOCH(CH₃)₂, OCO(cyclopentyl),-COCH₃, optionally substituted phenoxy, or optionally substituted benzyloxy.

24. The compound of clause 16, wherein:
x is 1; and
R³ is at the 7-position of the quinazoline ring and is -Cl, -CH₃, -CH₂CH₃, -F, -CF₃, -OCF₃ₜ -CONHCH₃, -CONHCH₂CH₃. -CONH(cyclopropyl), -OCH₃, -NH₂, -OCH₂CH₃, or -CN.

25. The compound according to clause 24, wherein:
x is 1; and
R³ is at the 7-position of the quinazoline ring and is -Cl, -CH₃, -CH₂CH₃, -F, -CF₃, -OCF₃, -OCH₃, or -OCH₂CH₃

26. The compound according to clause 25, wherein said compound is compound IIc-1 or a suitable salt thereof:

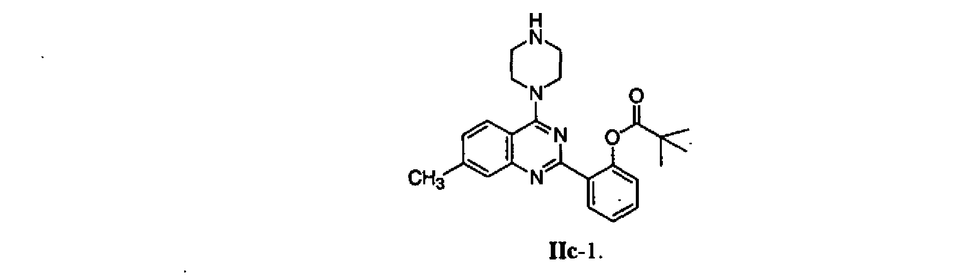

27. A compound of formula III: 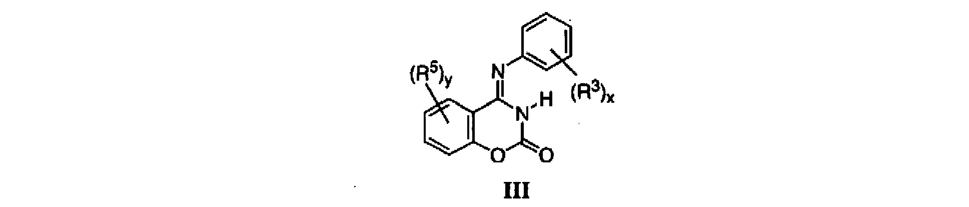 or a suitable salt thereof; wherein:
x is 0-4;
each R³ is independently halogen; CN, N0₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, N0₂, -N(R')2_{,} -CH₂N(R')₂, -OR', -CH₂OR', -SR', -CH₂SR', -NRCOR', -CON(R')_{2,} -S(O)₂N(R')2_{,} -OCOR', -COR', -CO₂R', -OCON(R')₂, -NR'S0₂R', -OP(O)(OR')₂, -P(O)(OR')₂, -OP(O)₂OR', -P(O)₂OR', -PO(R')₂, -OPO(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl_{;} and
each occurrence of R' is independently hydrogen or an optionally substituted C₁-₆ aliphatic group, a 3-8-membered saturated, partially unsaturated, or fully unsaturated monocyclic ring having 0-3 heteroatoms independently selected from nitrogen, oxygen, or sulfur, or an $-12 membered saturated, partially unsaturated, or fully unsaturated bicyclic ring system having 0-5 heteroatoms independently selected from nitrogen, oxygen, or sulfur;
or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
provided that:
(i) x and y are not simultaneously zero; and
(ii) when y is zero, and x is one, then R³ is not:
chloro in the para-position; or
methyl in the para-position.

28. The compound according to clause 27, wherein:
x is 1 or 2; and
each R³ is independently C1, Br, F, CF₃, -OCF₃, Me, Et, CN, -COOH, -NH₂, -N(CH₃)₂, - N(Et)₂, -N(iPr)₂, -O(CH₂)₂OCH₃, -CONH₂, -COOCH₃, -OH, -OCH₃, -OCH₂CH₃, - CH₂OH, -NHCOCH₃, -NHCOCH(CH₃)₂, -S0₂NH₂, -CONH(cyclopropyl), -CONHCH₃,
-CONHCH_{z}CH₃, or an optionally substituted group selected from -piperidinyl, piperizinyl, morpholino, phenyl, phenyloxy, benzyl, or benzyloxy.

29. The compound according to clause 28, wherein:
x is 1; and
R³ is Cl, Br, F, CF₃, -OCF₃, Me, Et, CN, -COO, -OH, or -OCH₃. clause

30. The compound according to clause 29, wherein:
y is 0-4; and
each R⁵ is independently Cl, Br, F, CF₃, Me, Et, CN, -COOH, -NH₂, -N(CH₃)₂, -N(Et)_{Z}, - N(iPr)₂, -O(CH₂ₕOCH), -CONH₂, -COOCH₃, -OH, -OCH₃, -OCH₂CH₃, -CH₂OH, - NHCOCH₃, -SO₂NH₂, -S0₂NHC(CH₃)₂, -OCOC(CH₃)₃, -OCOCH₂C(CH₃)₃, - 0(CH₂)₂N(CH₃)₂, 4-CH₃-piperazin-1-yl, OCOCH(CH₃)₂, OCO( cyc1opentyl), -COCH_{,} optionally substituted phenoxy, or optionally substituted benzyloxy.

31. The compound according to clause 30, wherein:
y is 0 or 1; and
R⁵ is Cl. Br, F, CF₃, Me, -OH, -OCH₃, -OCH_{Z}CH₃, -CH₂OH, -NHCOCH₃, -S0₂NH₂, - S0₂NHC(CH₃)₂.

32. The compound according to clause 31, wherein said compound is compound **III.1** or a suitable salt thereof:

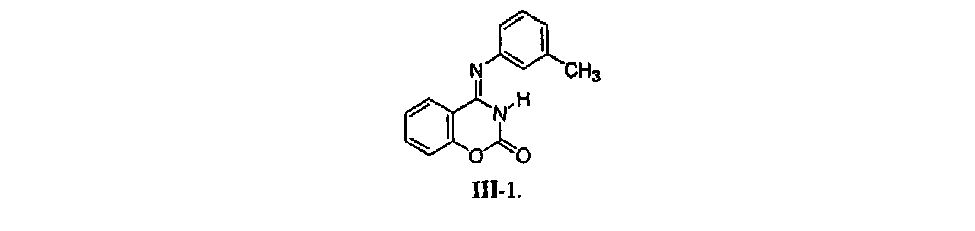

33. A compound of formula IV. 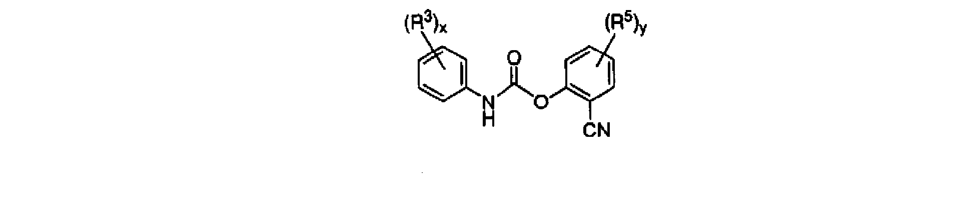 IV
or a suitable salt thereof;
wherein;
x is 0-4.;
each R³ is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -SO₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, NO₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO₂R', -OP(O)(OR')2_{,} -P(O)(OR')₂, -OP(O)₂OR', -P(O)20R', -PO(R')2_{,} -OPO(R')2_{,} or an optionally substituted group selected from C₁-C₆alipbatic, aryl, heteroaryl, cycloaliphatic, heterocycloaliphatic, arylC₁-C₆alkyl, heteroarylC₁-C₆alkyl, cyc1oaliphaticCₜ-Ctialkyt, or heterocycloaliphaticC₁-C₆alkyl; and
each occurrence of R' is independently hydrogen or an optionally substituted C₁-₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur;
or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
provided that when x is one and R³ is methyl in the 3-position, then when y is one, R⁵ is not -S-CN in the 4-position.

34. The compound according to clause 33, wherein:
x is 1 or 2; and
each R³ is independently Cl, Br, F, CF₃, -OCF₃, Me, Et, CN, -COOH, -NH₂, -N(CH₃)₂, - N(Et)₂, -N(iPr)₂, -O(CH₂)₂OCH₃, -CONH₂, -COOCH₃, -OH, -OCH₃, -OCH₂CH₃, - CH₂OH, -NHCOCH₃, -NHCOCH(CH₃)₂, -SO₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy.

35. The compound according to clause 34, wherein:
x is 1; and
R³ is Cl, Br, F, CF₃, -OCF₃, Me, Et, CN, -COOH, -OH, or .-OCH₃. clause

36. The compound according to clause 35, wherein:
y is 0-4; and
each R⁵ is independently Cl, Br, F, CF₃, Me, Et, CN, -COOH, -NH_{z}, -N(CH₃)₂, -N(Et)_{z}, - N(iPr)₂, -O(CH₂)₂OCH₃, -CONH₂, -COOCH₃, -OH, -OCH₃, -OCH₂CH₃, -CH₂OH, - NHCOCH₃, -SO₂NH₂, -SO₂NHC(CH₃), -OCOC(CH₃)₃, -OCOCH₂C(CH₃)₃, - O(CH₂)₂N(CH₃)2,4-CH₃-piperazin-1-yl, OCOCH(CH₃)₂, OCO(cyclopentyl), -COCH₃, optionally substituted phenoxy, or optionally substituted benzyloxy.

37. The compound according to clause 36, wherein:
y is 0 or 1; and
R⁵ is Cl, Br, F, CF₃, Me, -OH, -OCH₃, -OCH₂CH₃, -CH₂OH, -NHCOCH₃, -SO₂NH₂, -SO₂NHC(CH₃)₂-

38. The compound according to clause 37, wherein said compound is compound IV-1 or a suitable salt thereof:

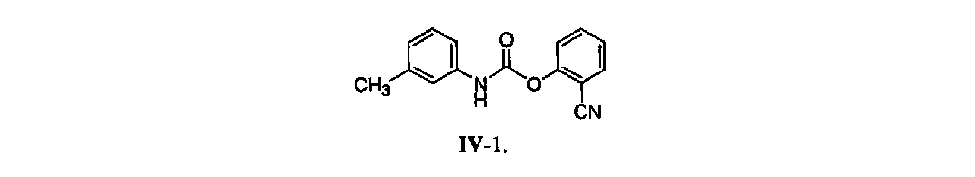

## Amended claims

### Amended claims in accordance with Rule 137(2) EPC.

**1.** A compound of formula IIc: 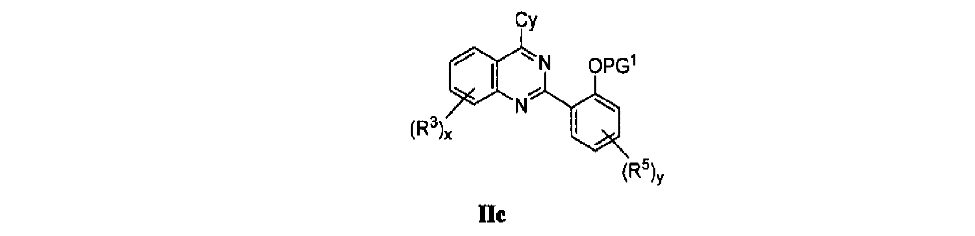 or a suitable salt thereof;
wherein:
PG¹ is a suitable hydroxyl protecting group;
Cy is a ring selected from: 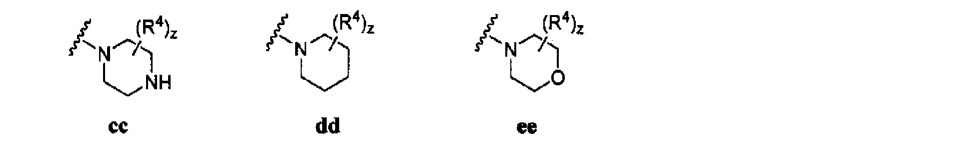 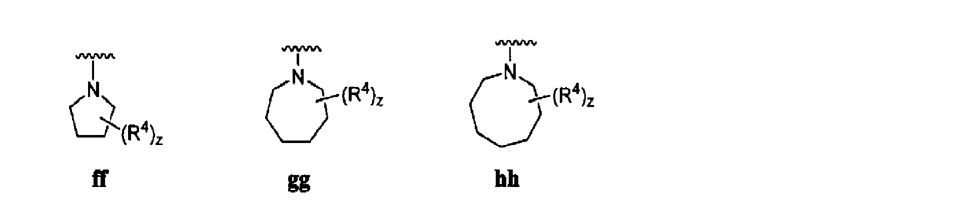 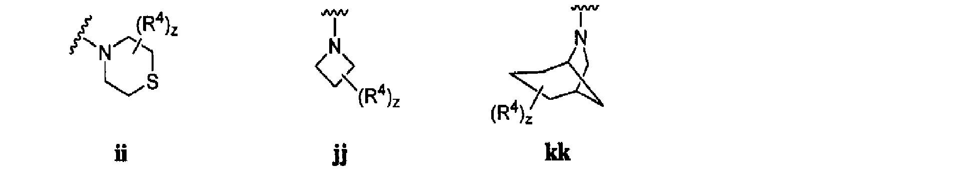 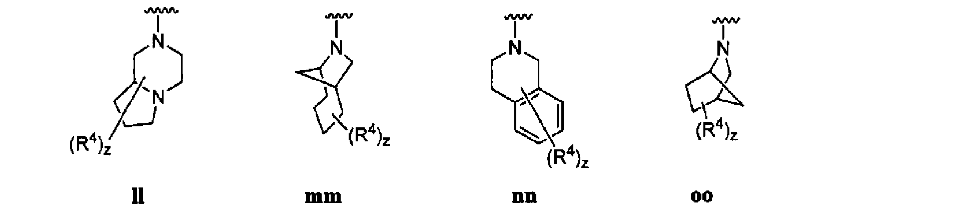 and Cy is optionally substituted at one or more substitutable carbon, nitrogen, or sulfur atoms
with z independent occurrences of -R⁴;
each z is independently 0-5;
each R4 is independently halogen, CN, NO₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -S0₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
x is 0-4;
each R³ is independently halogen, CN, N0₂, -N(R')₂, -CH₂N(R')₂, -OR', -CH₂OR', -SR', -CH₂SR', -COOR', -NRCOR', -CON(R')₂, -OCON(R')₂, COR', -NHCOOR', -SO₂R', -S0₂N(R')₂, or an optionally substituted group selected from C₁-C₆aliphatic, aryl, heteroaryl, cycloaliphatic, heterocycloaliphatic, arylC₁-C₆alkyl, heteroarylC₁-C₆alkyl, cycloaliphaticC₁-C₆alkyl, or heterocycloaliphaticC₁-C₆alkyl;
y is 0-5;
each R⁵ is independently halogen, CN, N0₂, -N(R')₂, -CH₂N(R')₂, -OR', -CH₂OR', -SR', -CH₂SR', -NRCOR', -CON(R')₂, -S(O)₂N(R')₂, -OCOR', -COR', -CO₂R', -OCON(R')₂, -NR'SO2R', -OP(O)(OR')₂, -P(O)(OR')₂, -OP(O)₂OR', -P(O)₂OR', -PO(R')₂, -OPO(R')₂, or an optionally substituted group selected from C₁₋C₆aliphatic, aryl, heteroaryl, cycloaliphatic, heterocycloaliphatic, arylC₁-C₆alkyl, heteroarylc₁-C₆alkyl, cycloaliphaticC₁-C₆alkyl, or heterocycloaliphaticC₁-C₆alkyl; and
each occurrence of R' is independently hydrogen or an optionally substituted C₁-₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur;
or:
two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
provided that the following compounds are excluded:
Quinazoline, 2-(2-methoxyphenyl)-4-(1-pyrrolidinyl)-;
Quinazoline, 6-bromo-2-(2-methoxyphenyl)-4-(4-morpholinyl)-;
Quinazoline, 6,8-dichloro-2-(2-methoxyphenyl)-4-(4-morpholinyl)-;
Quinazoline, 6-bromo-2-(2-methoxyphenyl)-4-(1-pyrrolidinyl)-;
Quinazoline, 6,8-dichloro-2-(2-metlloxyphenyl)-4-(1 -pyrrolidinyl)-;
Quinazoline, 2-(2-fluoro-6-methoxyphenyl)-6-methoxy-4-(4-morpholinyl)-;
Quinazoline, 2-(2-fluoro-6-methoxyphenyl)-4-(4-methyl-1-piperidinyl)-7-(trifluoromethyl)-;
Cyclopropanecarboxylic acid, 3-fluoro-2-[7-methyl-4-(4-methyl-1-piperidinyl)-2-quinazolinyl]phenyl ester;
Propanoic acid, 2-methyl-, 3-fluoro-2-[7-methyl-4-(4-methyl-1-piperidinyl)-2-quinazolinyl]phenyl ester;
Butanoic acid, 3-methyl-, 3-fluoro-2-[7-methyl-4-(4-methyl-1-piperidinyl)-2-quinazolinyl]phenyl ester;
Cyclopentanecarboxylic acid, 3-fluoro-2-[7-methyl-4-(4-methyl-l-pipendinyl)-2-quinazolinyl]phenyl ester;
Propanoic acid, 2,2-dimethyl-, 3-fluoro-2-[7-methyl-4-(4-methyl-1-piperidinyl)-2-quinazolinyl]phenyl ester;
Butanoic acid, 3,3-dimethyl-, 3-fluoro-2-[7-methyl-4-(4-methyl-1-piperidinyl)-2-quinazolinyl]phenyl ester;
Quinazoline, 7 -chloro-2-(2-methoxyphenyl)-4-[3-(trifluoromethyl)-I-pyrrolidinyl];
Piperazine, 1-(butylsulfonyl)-4-[2-(2,4-dimethoxyphenyl)-7-methyl-4-quinazolinyl]-;
Phenol, 3-fluoro-2-[7-methyl-4-(4-methyl-1-piperidinyl)-2-quinazolinyl]-, acetate (ester);
Piperazine, 1-(butylsulfonyl)-4-[2-(2-fluoro-6-methoxyphenyl)-7-(trifluoromethyl)-4-quinazolinyl]-;
1-Piperazinecarboxylic acid, 4-[6-bromo-2-(2-methoxyphenyl)-4-quinazolinyl]-, 1,1-dimethylethyl ester;
Carbamic acid, (2-methylpropyl)-, l-[2-(2-methoxyphenyl)-7-methyl-4-quinazolinyl]-4-piperidinyl ester;
6-Quinazolinecarboxylic acid, 4-[4-[(1,1-dimethylethoxy)carbonyl]-1-piperazinyl] -2-(2-methoxyphenyl)-; and
Benzenesulfonamide, 2-methoxy-5-[2-[4-[2-(2-methoxyphenyl)-4-quinazolinyl]-1-piperazinyl]ethyl]-, (2Z)-2-butenedioate (2:3).

**2.** The compound according to claim 1, wherein:
PG¹ is a suitable hydroxyl protecting group;
Cy is azetidin-1-yl (jj), pyrrolidin-1-yl (ft), piperidinl-yl (dd), or piperazin-1-yl (cc) optionally substituted with 0-4 occurrences of R⁴;
each R⁴ is independently Cl, Br, F, CF₃, CH₃, -CH₂CH₃, CN, -COOH, -N(CH₃)₂, -N(Et)₂, -N(iPr)₂, -O(CH₂)₂0CH₃, -CONH₂, -COOCH₃, -OH, -CH₂OH, -NHCOCH₃, -S0₂NH₂, -S0₂(CH₂)₃CH₃, -SO₂CH(CH₃)₂, -SO₂N(CH₃)₂, -SO₂CH₂CH₃, -C(O)OCH₂CH(CH₃)₂, -C(O)NHCH₂CH(CH₃)₂, -C(O)CH(OH)CH₂CH(CH₃)₂, -C(O)CH(OH)CH₂C(CH₃)₃, -NHCOOCH₃, -C(O)C(CH₃)₃, -COO(CH₂)₂CH₃, .C(O)NHCH(CH₃)₂, -C(O)CH₂CH₃, or an optionally substituted group selected from -piperidinyl, piperizinyl, morpholino, C₁-₄alkoxy, phenyl, phenyloxy, benzyl, benzyloxy, -CH₂cyclohexyl, pyridyl, -CH_{2P}yridyl, or -C.'H₂thla_{Z}^{p}lyl;
x is 1 or 2;
each R³ is independently Cl, Br, F, CF₃, -OCF₃, Me, Et, CN, -COOH, -NH₂, -N(CH₃)₂, - N(Et)₂, -N(iPr)₂, -O(CH₂)₂0CH₃, -CONH₂, -COOCH₃, -OH, -OCH₃, -OCH₂CH₃, - CH₂OH, -NHCOCH₃, -NHCOCH(CH₃)₂, -S0₂NH₂, -CONH(cyclopropyl), -CONHCH₃, -CONHCH₂CH₃, or an optionally substituted group selected from -piperidinyl, piperizinyl, morpholino, phenyl, phenyloxy, benzyl, or benzyloxy;
y is 0-4; and
each R⁵ is independently Cl, Br, F, CF₃, Me, Et, CN, -COOH, -NH₂, N(CH₃)₂, -N(Et)₂, - N(iPr)₂, -O(CH₂)₂OCH₃, -CONH₂, -COOCH₃, -OH, -OCH₃, -OCH₂CH₃, -CH₂OH, - NHCOCH₃, -S0₂NH₂, -S0₂NHC(CH₃)₂, -OCOC(CH₃)₃, -OCOCH₂C(CH₃)₃, - O(CH₂)_{Z}N(CH₃)₂, 4-CH₃-piperazin-1-yl, OCOCH(CH₃)₂, OCO(cyclopentyl), -COCH₃, optionally substituted phenoxy, or optionally substituted benzyloxy.

**3.** The compound of claim 2, wherein:
x is 1; and
R³ is at the 7-position of the quinazoline ring and is -Cl, ..CH₃, -CH₂CH₃, -F, -CF₃, -OCF₃, -CONHCH₃, -CONHCH₂CH₃, -CONH(cyclopropyl), -OCH₃, -NH₂, -OCHzCH3, or -CN.

**4.** The compound according to claim 3, wherein:
xis 1; and
R³ is at the 7-position of the quinazoline ring and is -Cl, -CH₃, -CH₂CH₃, -F, -CF₃, -OCF₃, -OCH₃, or -OCH₂CH₃

**5.** The compound according to claim 4, wherein said compound is compound He-1 or a suitable salt thereof
